(19) Europäisches Patentamt
European Patent Office
Office européen des brevets

(11) EP 3 283 886 B1

(12) **EUROPEAN PATENT SPECIFICATION**

(45) Date of publication and mention of the grant of the patent:
**15.01.2020 Bulletin 2020/03**

(21) Application number: **16718820.0**

(22) Date of filing: **14.04.2016**

(51) Int Cl.:
***G01N 33/574*** *(2006.01)* ***A61K 39/395*** *(2006.01)*
***G01N 33/82*** *(2006.01)*

(86) International application number:
**PCT/US2016/027497**

(87) International publication number:
**WO 2016/168440 (20.10.2016 Gazette 2016/42)**

(54) **METHODS FOR TREATING LUNG CANCER**

VERFAHREN ZUR BEHANDLUNG VON LUNGENKREBS

PROCÉDÉS DE TRAITEMENT DU CANCER DU POUMON

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.04.2015 US 201562149184 P**

(43) Date of publication of application:
**21.02.2018 Bulletin 2018/08**

(73) Proprietor: **Eisai Inc.**
**Woodcliff Lake, NJ 07677 (US)**

(72) Inventor: **O'SHANNESSY, Daniel, John**
**Schwenksville, PA 19473 (US)**

(74) Representative: **Carpmaels & Ransford LLP**
**One Southampton Row**
**London WC1B 5HA (GB)**

(56) References cited:

- **ANISH THOMAS ET AL: "Farletuzumab in lung cancer", LUNG CANCER., vol. 80, no. 1, 1 April 2013 (2013-04-01), pages 15-18, XP055283145, NL ISSN: 0169-5002, DOI: 10.1016/j.lungcan.2012.12.021**
- **DANIEL J O 'SHANNESSY ET AL: "Folate Receptor Alpha Expression in Lung Cancer: Diagnostic and Prognostic Significance", ONCOTARGET, 29 April 2012 (2012-04-29), XP055283147,**
- **DANIEL J O'SHANNESSY ET AL: "Characterization of the Human Folate Receptor Alpha Via Novel Antibody-Based Probes", ONCOTARGET, vol. 2, no. 12, 27 December 2011 (2011-12-27), pages 1227-1243, XP055022611,**
- **R Bremer ET AL: "Folate Receptor Alpha Expression in Lung and Ovarian Cancers by IHC Using a High Affinity Folate Receptor Alpha Antibody [26B3.F2]", As Presented at CAP, Poster Session #500, Poster Assignment #4, 20 September 2012 (2012-09-20), XP055283157, Retrieved from the Internet: URL:https://biocare.net/wp-content/uploads /AB-FRa100.pdf [retrieved on 2016-06-23]**
- **JULIA D MALTZMAN ET AL: "Phase 2 double-blind, placebo-controlled study of three-weekly farletuzumab with a platinum containing doublet in subjects with previously untreated folate receptor alpha (FRA) expressing non-small-cell lung cancer (NSCLC).", 15TH WORLD CONFERENCE ON LUNG CANCER, 1 October 2013 (2013-10-01), XP055283207,**
- **DANIEL C CHRISTOPH ET AL: "Significance of Folate Receptor Alpha and Thymidylate Synthase Protein Expression in Patients with Non-Small-Cell Lung Cancer Treated with Pemetrexed", JOURNAL OF THORACIC ONCOLOGY, vol. 8, no. 1, 1 January 2013 (2013-01-01) , pages 19-30, XP055283219,**
- **MARIA INES NUNEZ ET AL: "High Expression of Folate Receptor Alpha in Lung Cancer Correlates with Adenocarcinoma Histology and Mutation", JOURNAL OF THORACIC ONCOLOGY, vol. 7, no. 5, 13 April 2012 (2012-04-13), pages 833-840, XP055283276,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> MORPHOTEK, INC.

<120> METHODS FOR TREATING LUNG CANCER

<130> 104018.000950

<140>
<141>

<150> 62/149,184
<151> 2015-04-17

<160> 46

<170> PatentIn version 3.5

<210> 1
<211> 10
<212> PRT
<213> Artificial Sequence

<220>
<221> source
<223> /note="Description of Artificial Sequence: Synthetic peptide"

<400> 1

```
Gly Phe Thr Phe Ser Gly Tyr Gly Leu Ser
1               5                   10
```

<210> 2
<211> 17
<212> PRT
<213> Artificial Sequence

<220>
<221> source
<223> /note="Description of Artificial Sequence: Synthetic peptide"

<400> 2

```
Met Ile Ser Ser Gly Gly Ser Tyr Thr Tyr Tyr Ala Asp Ser Val Lys
1               5                   10                  15


Gly
```

<210> 3
<211> 10
<212> PRT
<213> Artificial Sequence

<220>
<221> source
<223> /note="Description of Artificial Sequence: Synthetic peptide"

<400> 3

His Gly Asp Asp Pro Ala Trp Phe Ala Tyr
1 5 10

<210> 4
<211> 12
<212> PRT
<213> Artificial Sequence

<220>
<221> source
<223> /note="Description of Artificial Sequence: Synthetic peptide"

<400> 4

Ser Val Ser Ser Ser Ile Ser Ser Asn Asn Leu His
1 5 10

<210> 5
<211> 7
<212> PRT
<213> Artificial Sequence

<220>
<221> source
<223> /note="Description of Artificial Sequence: Synthetic peptide"

<400> 5

Gly Thr Ser Asn Leu Ala Ser
1 5

<210> 6
<211> 11
<212> PRT
<213> Artificial Sequence

<220>
<221> source
<223> /note="Description of Artificial Sequence: Synthetic peptide"

<400> 6

Gln Gln Trp Ser Ser Tyr Pro Tyr Met Tyr Thr
1 5 10

<210> 7
<211> 217

<212> PRT
<213> Artificial Sequence

<220>
<221> source
<223> /note="Description of Artificial Sequence: Synthetic polypeptide"

<400> 7

```
Asp Ile Gln Leu Thr Gln Ser Pro Ser Ser Leu Ser Ala Ser Val Gly
1               5                   10                  15
```

```
Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Pro Trp
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Tyr Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys Arg Thr
            100                 105                 110

Val Ala Ala Pro Ser Val Phe Ile Phe Pro Pro Ser Asp Glu Gln Leu
        115                 120                 125

Lys Ser Gly Thr Ala Ser Val Val Cys Leu Leu Asn Asn Phe Tyr Pro
    130                 135                 140

Arg Glu Ala Lys Val Gln Trp Lys Val Asp Asn Ala Leu Gln Ser Gly
145                 150                 155                 160

Asn Ser Gln Glu Ser Val Thr Glu Gln Asp Ser Lys Asp Ser Thr Tyr
                165                 170                 175

Ser Leu Ser Ser Thr Leu Thr Leu Ser Lys Ala Asp Tyr Glu Lys His
            180                 185                 190

Lys Val Tyr Ala Cys Glu Val Thr His Gln Gly Leu Ser Ser Pro Val
        195                 200                 205

Thr Lys Ser Phe Asn Arg Gly Glu Cys
    210                 215
```

<210> 8
<211> 449
<212> PRT
<213> Artificial Sequence

<220>
<221> source
<223> /note="Description of Artificial Sequence: Synthetic polypeptide"

<400> 8

```
Glu Val Gln Leu Val Glu Ser Gly Gly Gly Val Val Gln Pro Gly Arg
1               5                   10                  15

Ser Leu Arg Leu Ser Cys Ser Ala Ser Gly Phe Thr Phe Ser Gly Tyr
            20                  25                  30

Gly Leu Ser Trp Val Arg Gln Ala Pro Gly Lys Gly Leu Glu Trp Val
        35                  40                  45

Ala Met Ile Ser Ser Gly Gly Ser Tyr Thr Tyr Tyr Ala Asp Ser Val
    50                  55                  60

Lys Gly Arg Phe Ala Ile Ser Arg Asp Asn Ala Lys Asn Thr Leu Phe
65                  70                  75                  80

Leu Gln Met Asp Ser Leu Arg Pro Glu Asp Thr Gly Val Tyr Phe Cys
                85                  90                  95

Ala Arg His Gly Asp Asp Pro Ala Trp Phe Ala Tyr Trp Gly Gln Gly
            100                 105                 110

Thr Pro Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255
```

Arg Thr Pro Glu Val Thr Cys Val Val Val Asp Val Ser His Glu Asp
260 265 270

Pro Glu Val Lys Phe Asn Trp Tyr Val Asp Gly Val Glu Val His Asn
275 280 285

Ala Lys Thr Lys Pro Arg Glu Glu Gln Tyr Asn Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Ala Leu Pro Ala Pro Ile Glu Lys
325 330 335

Thr Ile Ser Lys Ala Lys Gly Gln Pro Arg Glu Pro Gln Val Tyr Thr
340 345 350

Leu Pro Pro Ser Arg Asp Glu Leu Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Val Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro Gly
435 440 445

Lys

<210> 9
<211> 962
<212> DNA
<213> Homo sapiens

<400> 9

tcaaggttaa acgacaagga cagacatggc tcagcggatg acaacacagc tgctgctcct 60

tctagtgtgg gtggctgtag taggggaggc tcagacaagg attgcatggg ccaggactga 120

```
gcttctcaat gtctgcatga acgccaagca ccacaaggaa aagccaggcc ccgaggacaa      180
gttgcatgag cagtgtcgac cctggaggaa gaatgcctgc tgttctacca acaccagcca      240
ggaagcccat aaggatgttt cctacctata tagattcaac tggaaccact gtggagagat      300
ggcacctgcc tgcaaacggc atttcatcca ggacacctgc ctctacgagt gctcccccaa      360
cttggggccc tggatccagc aggtggatca gagctggcgc aaagagcggg tactgaacgt      420
gcccctgtgc aaagaggact gtgagcaatg gtgggaagat tgtcgcacct cctacacctg      480
caagagcaac tggcacaagg gctggaactg gacttcaggg tttaacaagt gcgcagtggg      540
agctgcctgc caacctttcc atttctactt ccccacaccc actgttctgt gcaatgaaat      600
ctggactcac tcctacaagg tcagcaacta cagccgaggg agtggccgct gcatccagat      660
gtggttcgac ccagcccagg gcaaccccaa tgaggaggtg gcgaggttct atgctgcagc      720
catgagtggg gctgggccct gggcagcctg gcctttcctg cttagcctgg ccctaatgct      780
gctgtggctg ctcagctgac ctccttttac cttctgatac ctggaaatcc ctgccctgtt      840
cagccccaca gctcccaact atttggttcc tgctccatgg tcgggcctct gacagccact      900
ttgaataaac cagacaccgc acatgtgtct tgagaattat ttggaaaaaa aaaaaaaaaa      960
aa                                                                    962
```

<210> 10
<211> 257
<212> PRT
<213> Homo sapiens

<400> 10

```
Met Ala Gln Arg Met Thr Thr Gln Leu Leu Leu Leu Leu Val Trp Val
1               5                   10                  15

Ala Val Val Gly Glu Ala Gln Thr Arg Ile Ala Trp Ala Arg Thr Glu
            20                  25                  30

Leu Leu Asn Val Cys Met Asn Ala Lys His His Lys Glu Lys Pro Gly
        35                  40                  45

Pro Glu Asp Lys Leu His Glu Gln Cys Arg Pro Trp Arg Lys Asn Ala
    50                  55                  60

Cys Cys Ser Thr Asn Thr Ser Gln Glu Ala His Lys Asp Val Ser Tyr
65                  70                  75                  80

Leu Tyr Arg Phe Asn Trp Asn His Cys Gly Glu Met Ala Pro Ala Cys
                85                  90                  95

Lys Arg His Phe Ile Gln Asp Thr Cys Leu Tyr Glu Cys Ser Pro Asn
```

```
                100                 105                 110

Leu Gly Pro Trp Ile Gln Gln Val Asp Gln Ser Trp Arg Lys Glu Arg
        115                 120                 125

Val Leu Asn Val Pro Leu Cys Lys Glu Asp Cys Glu Gln Trp Trp Glu
    130                 135                 140

Asp Cys Arg Thr Ser Tyr Thr Cys Lys Ser Asn Trp His Lys Gly Trp
145                 150                 155                 160

Asn Trp Thr Ser Gly Phe Asn Lys Cys Ala Val Gly Ala Ala Cys Gln
                165                 170                 175

Pro Phe His Phe Tyr Phe Pro Thr Pro Thr Val Leu Cys Asn Glu Ile
            180                 185                 190

Trp Thr His Ser Tyr Lys Val Ser Asn Tyr Ser Arg Gly Ser Gly Arg
        195                 200                 205

Cys Ile Gln Met Trp Phe Asp Pro Ala Gln Gly Asn Pro Asn Glu Glu
    210                 215                 220

Val Ala Arg Phe Tyr Ala Ala Ala Met Ser Gly Ala Gly Pro Trp Ala
225                 230                 235                 240

Ala Trp Pro Phe Leu Leu Ser Leu Ala Leu Met Leu Leu Trp Leu Leu
                245                 250                 255

Ser
```

<210> 11
<211> 119
<212> PRT
<213> Artificial Sequence

<220>
<221> source
<223> /note="Description of Artificial Sequence: Synthetic polypeptide"

<220>
<221> MOD_RES
<222> (3)..(3)
<223> Any amino acid

<220>
<221> MOD_RES
<222> (6)..(6)
<223> Any amino acid

<400> 11

Gln Val Xaa Leu Gln Xaa Ser Gly Gly Asp Leu Val Lys Pro Gly Gly
1               5                   10                  15

Ser Leu Lys Leu Ser Cys Ala Ala Ser Gly Phe Thr Phe Ser Gly Tyr
            20                  25                  30

Gly Leu Ser Trp Val Arg Gln Thr Pro Asp Lys Arg Leu Glu Trp Val
        35                  40                  45

Ala Met Ile Ser Ser Gly Gly Ser Tyr Thr Tyr Tyr Ala Asp Ser Val
    50                  55                  60

Lys Gly Arg Phe Ala Ile Ser Arg Asp Asn Ala Lys Asn Ser Leu Phe
65                  70                  75                  80

Leu Gln Met Ser Ser Leu Lys Ser Asp Asp Thr Ala Ile Tyr Ile Cys
                85                  90                  95

Ala Arg His Gly Asp Asp Pro Ala Trp Phe Ala Tyr Trp Gly Gln Gly
            100                 105                 110

Thr Leu Val Thr Val Ser Ala
        115

<210> 12
<211> 110
<212> PRT
<213> Artificial Sequence

<220>
<221> source
<223> /note="Description of Artificial Sequence: Synthetic polypeptide"

<400> 12

```
Asp Ile Glu Leu Thr Gln Ser Pro Ala Leu Met Ala Ala Ser Pro Gly
1               5                   10                  15

Glu Lys Val Thr Ile Thr Cys Ser Val Ser Ser Ser Ile Ser Ser Asn
            20                  25                  30

Asn Leu His Trp Tyr Gln Gln Lys Ser Glu Thr Ser Pro Lys Pro Trp
        35                  40                  45

Ile Tyr Gly Thr Ser Asn Leu Ala Ser Gly Val Pro Leu Arg Phe Arg
    50                  55                  60

Gly Phe Gly Ser Gly Thr Ser Tyr Ser Leu Thr Ile Ser Ser Met Glu
65                  70                  75                  80

Ala Glu Asp Ala Ala Thr Tyr Tyr Cys Gln Gln Trp Ser Ser Tyr Pro
                85                  90                  95

Tyr Met Tyr Thr Phe Gly Gly Gly Thr Lys Leu Glu Ile Lys
            100                 105                 110
```

<210> 13

<400> 13
000

<210> 14
<211> 5
<212> PRT
<213> Artificial Sequence

<220>
<221> source
<223> /note="Description of Artificial Sequence: Synthetic peptide"

<400> 14

```
Gly Tyr Phe Met Asn
1               5
```

<210> 15
<211> 17
<212> PRT
<213> Artificial Sequence

<220>
<221> source
<223> /note="Description of Artificial Sequence: Synthetic peptide"

<400> 15

Arg Ile Phe Pro Tyr Asn Gly Asp Thr Phe Tyr Asn Gln Lys Phe Lys
1 5 10 15

Gly

<210> 16
<211> 7
<212> PRT
<213> Artificial Sequence

<220>
<221> source
<223> /note="Description of Artificial Sequence: Synthetic peptide"

<400> 16

Gly Thr His Tyr Phe Asp Tyr
1 5

<210> 17
<211> 11
<212> PRT
<213> Artificial Sequence

<220>
<221> source
<223> /note="Description of Artificial Sequence: Synthetic peptide"

<400> 17

Arg Thr Ser Glu Asn Ile Phe Ser Tyr Leu Ala
1 5 10

<210> 18
<211> 7
<212> PRT
<213> Artificial Sequence

<220>
<221> source
<223> /note="Description of Artificial Sequence: Synthetic peptide"

<400> 18

Asn Ala Lys Thr Leu Ala Glu
1 5

<210> 19
<211> 9
<212> PRT
<213> Artificial Sequence

<220>
<221> source
<223> /note="Description of Artificial Sequence: Synthetic peptide"

<400> 19

Gln His His Tyr Ala Phe Pro Trp Thr
1 5

<210> 20
<211> 5
<212> PRT
<213> Artificial Sequence

<220>
<221> source
<223> /note="Description of Artificial Sequence: Synthetic peptide"

<400> 20

His Pro Tyr Met His
1 5

<210> 21
<211> 17
<212> PRT
<213> Artificial Sequence

<220>
<221> source
<223> /note="Description of Artificial Sequence: Synthetic peptide"

<400> 21

Arg Ile Asp Pro Ala Asn Gly Asn Thr Lys Tyr Asp Pro Lys Phe Gln
1 5 10 15

Gly

<210> 22
<211> 10
<212> PRT
<213> Artificial Sequence

<220>
<221> source
<223> /note="Description of Artificial Sequence: Synthetic peptide"

<400> 22

Glu Glu Val Ala Asp Tyr Thr Met Asp Tyr
1 5 10

<210> 23
<211> 15
<212> PRT
<213> Artificial Sequence

<220>
<221> source
<223> /note="Description of Artificial Sequence: Synthetic peptide"

<400> 23

Arg Ala Ser Glu Ser Val Asp Thr Tyr Gly Asn Asn Phe Ile His
1 5 10 15

<210> 24
<211> 7
<212> PRT
<213> Artificial Sequence

<220>
<221> source
<223> /note="Description of Artificial Sequence: Synthetic peptide"

<400> 24

Leu Ala Ser Asn Leu Glu Ser
1 5

<210> 25
<211> 9
<212> PRT
<213> Artificial Sequence

<220>
<221> source
<223> /note="Description of Artificial Sequence: Synthetic peptide"

<400> 25

Gln Gln Asn Asn Gly Asp Pro Trp Thr
1 5

<210> 26
<211> 6
<212> PRT
<213> Artificial Sequence

<220>
<221> source
<223> /note="Description of Artificial Sequence: Synthetic peptide"

<400> 26

Ser Gly Tyr Tyr Trp Asn
1 5

<210> 27
<211> 16
<212> PRT
<213> Artificial Sequence

<220>
<221> source
<223> /note="Description of Artificial Sequence: Synthetic peptide"

<400> 27

Tyr Ile Lys Ser Asp Gly Ser Asn Asn Tyr Asn Pro Ser Leu Lys Asn
1 5 10 15

<210> 28
<211> 7
<212> PRT
<213> Artificial Sequence

<220>
<221> source
<223> /note="Description of Artificial Sequence: Synthetic peptide"

<400> 28

```
Glu Trp Lys Ala Met Asp Tyr
1               5
```

<210> 29
<211> 12
<212> PRT
<213> Artificial Sequence

<220>
<221> source
<223> /note="Description of Artificial Sequence: Synthetic peptide"

<400> 29

```
Arg Ala Ser Ser Thr Val Ser Tyr Ser Tyr Leu His
1               5                   10
```

<210> 30
<211> 7
<212> PRT
<213> Artificial Sequence

<220>
<221> source
<223> /note="Description of Artificial Sequence: Synthetic peptide"

<400> 30

```
Gly Thr Ser Asn Leu Ala Ser
1               5
```

<210> 31
<211> 9
<212> PRT
<213> Artificial Sequence

<220>
<221> source
<223> /note="Description of Artificial Sequence: Synthetic peptide"

<400> 31

```
Gln Gln Tyr Ser Gly Tyr Pro Leu Thr
1               5
```

<210> 32
<211> 5

<212> PRT
<213> Artificial Sequence

<220>
<221> source
<223> /note="Description of Artificial Sequence: Synthetic peptide"

<400> 32

Ser Tyr Ala Met Ser
1 5

<210> 33
<211> 17
<212> PRT
<213> Artificial Sequence

<220>
<221> source
<223> /note="Description of Artificial Sequence: Synthetic peptide"

<400> 33

Glu Ile Gly Ser Gly Gly Ser Tyr Thr Tyr Tyr Pro Asp Thr Val Thr
1 5 10 15

Gly

<210> 34
<211> 9
<212> PRT
<213> Artificial Sequence

<220>
<221> source
<223> /note="Description of Artificial Sequence: Synthetic peptide"

<400> 34

Glu Thr Thr Ala Gly Tyr Phe Asp Tyr
1 5

<210> 35
<211> 11
<212> PRT
<213> Artificial Sequence

<220>
<221> source
<223> /note="Description of Artificial Sequence: Synthetic peptide"

<400> 35

Ser Ala Ser Gln Gly Ile Asn Asn Phe Leu Asn
1 5 10

<210> 36

<211> 7
<212> PRT
<213> Artificial Sequence

<220>
<221> source
<223> /note="Description of Artificial Sequence: Synthetic peptide"

<400> 36

Tyr Thr Ser Ser Leu His Ser
1               5

<210> 37
<211> 9
<212> PRT
<213> Artificial Sequence

<220>
<221> source
<223> /note="Description of Artificial Sequence: Synthetic peptide"

<400> 37

Gln His Phe Ser Lys Leu Pro Trp Thr
1               5

<210> 38
<211> 110
<212> PRT
<213> Artificial Sequence

<220>
<221> source
<223> /note="Description of Artificial Sequence: Synthetic polypeptide"

<400> 38

```
Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Leu Leu
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Phe Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys
            100                 105                 110
```

<210> 39
<211> 110
<212> PRT
<213> Artificial Sequence

<220>
<221> source
<223> /note="Description of Artificial Sequence: Synthetic polypeptide"

<400> 39

```
Asp Ile Gln Leu Thr Gln Ser Pro Ser Ser Leu Ser Ala Ser Val Gly
1               5                   10                  15
```

```
Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Leu Leu
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Tyr Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys
            100                 105                 110
```

<210> 40
<211> 110
<212> PRT
<213> Artificial Sequence

<220>
<221> source
<223> /note="Description of Artificial Sequence: Synthetic polypeptide"

<400> 40

```
Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Pro Trp
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Phe Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys
            100                 105                 110
```

<210> 41
<211> 110
<212> PRT
<213> Artificial Sequence

<220>
<221> source
<223> /note="Description of Artificial Sequence: Synthetic polypeptide"

<400> 41

```
Asp Ile Gln Leu Thr Gln Ser Pro Ser Ser Leu Ser Ala Ser Val Gly
1               5                   10                  15

Asp Arg Val Thr Ile Thr Cys Ser Val Ser Ser Ser Ile Ser Ser Asn
            20                  25                  30

Asn Leu His Trp Tyr Gln Gln Lys Pro Gly Lys Ala Pro Lys Pro Trp
        35                  40                  45

Ile Tyr Gly Thr Ser Asn Leu Ala Ser Gly Val Pro Ser Arg Phe Ser
    50                  55                  60

Gly Ser Gly Ser Gly Thr Asp Tyr Thr Phe Thr Ile Ser Ser Leu Gln
65                  70                  75                  80

Pro Glu Asp Ile Ala Thr Tyr Tyr Cys Gln Gln Trp Ser Ser Tyr Pro
                85                  90                  95

Tyr Met Tyr Thr Phe Gly Gln Gly Thr Lys Val Glu Ile Lys
            100                 105                 110
```

<210> 42
<211> 119
<212> PRT
<213> Artificial Sequence

<220>
<221> source
<223> /note="Description of Artificial Sequence: Synthetic polypeptide"

<400> 42

```
Gln Val Gln Leu Gln Glu Ser Gly Pro Gly Leu Val Arg Pro Ser Gln
1               5                   10                  15

Thr Leu Ser Leu Thr Cys Thr Ala Ser Gly Phe Thr Phe Ser Gly Tyr
            20                  25                  30

Gly Leu Ser Trp Val Arg Gln Pro Pro Gly Arg Gly Leu Glu Trp Val
        35                  40                  45
```

Ala Met Ile Ser Ser Gly Gly Ser Tyr Thr Tyr Tyr Ala Asp Ser Val
50 55 60

Lys Gly Arg Val Thr Met Leu Arg Asp Thr Ser Lys Asn Gln Phe Ser
65 70 75 80

Leu Arg Leu Ser Ser Val Thr Ala Ala Asp Thr Ala Val Tyr Tyr Cys
85 90 95

Ala Arg His Gly Asp Asp Pro Ala Trp Phe Ala Tyr Trp Gly Gln Gly
100 105 110

Ser Leu Val Thr Val Ser Ser
115

<210> 43
<211> 119
<212> PRT
<213> Artificial Sequence

<220>
<221> source
<223> /note="Description of Artificial Sequence: Synthetic polypeptide"

<400> 43

Gln Val Gln Leu Gln Glu Ser Gly Pro Gly Leu Val Arg Pro Ser Gln
1 5 10 15

Thr Leu Ser Leu Thr Cys Thr Ala Ser Gly Phe Thr Phe Ser Gly Tyr
20 25 30

Gly Leu Ser Trp Val Arg Gln Pro Pro Gly Arg Gly Leu Glu Trp Val
35 40 45

Ala Met Ile Ser Ser Gly Gly Ser Tyr Thr Tyr Tyr Ala Asp Ser Val
50 55 60

Lys Gly Arg Phe Ala Ile Ser Arg Asp Asn Ser Lys Asn Gln Phe Ser
65 70 75 80

Leu Arg Leu Ser Ser Val Thr Ala Ala Asp Thr Ala Val Tyr Tyr Cys
85 90 95

Ala Arg His Gly Asp Asp Pro Ala Trp Phe Ala Tyr Trp Gly Gln Gly
100 105 110

Ser Leu Val Thr Val Ser Ser
115

<210> 44
<211> 119
<212> PRT
<213> Artificial Sequence

<220>
<221> source
<223> /note="Description of Artificial Sequence: Synthetic polypeptide"

<400> 44

```
Gln Val Gln Leu Gln Glu Ser Gly Pro Gly Leu Val Arg Pro Ser Gln
1               5                   10                  15

Thr Leu Ser Leu Thr Cys Thr Ala Ser Gly Phe Thr Phe Ser Gly Tyr
            20                  25                  30

Gly Leu Ser Trp Val Arg Gln Pro Pro Gly Arg Gly Leu Glu Trp Val
        35                  40                  45

Ala Met Ile Ser Ser Gly Gly Ser Tyr Thr Tyr Tyr Ala Asp Ser Val
    50                  55                  60

Lys Gly Arg Val Thr Met Leu Arg Asp Thr Ser Lys Asn Ser Leu Phe
65                  70                  75                  80

Leu Arg Leu Ser Ser Val Thr Ala Ala Asp Thr Ala Val Tyr Tyr Cys
                85                  90                  95

Ala Arg His Gly Asp Asp Pro Ala Trp Phe Ala Tyr Trp Gly Gln Gly
            100                 105                 110

Thr Thr Val Thr Val Ser Ser
        115
```

<210> 45
<211> 119
<212> PRT
<213> Artificial Sequence

<220>
<221> source
<223> /note="Description of Artificial Sequence: Synthetic polypeptide"

<400> 45

Gln Val Gln Leu Gln Glu Ser Gly Pro Gly Leu Val Arg Pro Ser Gln
1               5                   10                  15

Thr Leu Ser Leu Thr Cys Thr Ala Ser Gly Phe Thr Phe Ser Gly Tyr
            20                  25                  30

Gly Leu Ser Trp Val Arg Gln Pro Pro Gly Arg Gly Leu Glu Trp Val
        35                  40                  45

Ala Met Ile Ser Ser Gly Gly Ser Tyr Thr Tyr Tyr Ala Asp Ser Val
    50                  55                  60

Lys Gly Arg Val Thr Met Leu Arg Asp Thr Ser Lys Asn Gln Phe Ser
65                  70                  75                  80

Leu Arg Leu Ser Ser Val Thr Ala Ala Asp Thr Ala Ile Tyr Ile Cys
                85                  90                  95

Ala Arg His Gly Asp Asp Pro Ala Trp Phe Ala Tyr Trp Gly Gln Gly
            100                 105                 110

Ser Leu Val Thr Val Ser Ser
        115

<210> 46
<211> 119
<212> PRT
<213> Artificial Sequence

<220>
<221> source
<223> /note="Description of Artificial Sequence: Synthetic polypeptide"

<400> 46

```
Glu Val Gln Leu Val Glu Ser Gly Gly Gly Val Val Gln Pro Gly Arg
1               5                   10                  15

Ser Leu Arg Leu Ser Cys Ser Ala Ser Gly Phe Thr Phe Ser Gly Tyr
            20                  25                  30

Gly Leu Ser Trp Val Arg Gln Ala Pro Gly Lys Gly Leu Glu Trp Val
        35                  40                  45

Ala Met Ile Ser Ser Gly Gly Ser Tyr Thr Tyr Tyr Ala Asp Ser Val
    50                  55                  60

Lys Gly Arg Phe Ala Ile Ser Arg Asp Asn Ala Lys Asn Thr Leu Phe
65                  70                  75                  80

Leu Gln Met Asp Ser Leu Arg Pro Glu Asp Thr Gly Val Tyr Phe Cys
                85                  90                  95

Ala Arg His Gly Asp Asp Pro Ala Trp Phe Ala Tyr Trp Gly Gln Gly
            100                 105                 110

Thr Pro Val Thr Val Ser Ser

                              115
```

## Description

SEQUENCE LISTING

The instant application contains a Sequence Listing which has been submitted electronically in ASCII format. Said ASCII copy, created on April 13, 2016, is named 104018.000950_SL.txt and is 30,473 bytes in size.

TECHNICAL FIELD

The subject matter described herein relates to methods for predicting a likelihood of responsiveness to treatment with an antibody according to the present invention that immunospecifically binds folate receptor alpha (FRA) in a patient having a FRA-expressing lung cancer and to an antibody according to the present invention that immunospecifically binds folate receptor alpha (FRA) for use in methods of treating FRA-expressing lung cancer in a patient.

BACKGROUND

According to the American Cancer Society, an estimated 221,200 new cases of lung and bronchus cancer will be diagnosed in the United States in 2015, accounting for about 14% of all new cancer cases. In addition, an estimated 158,040 deaths from lung and bronchus cancer will occur in the United States in 2015, accounting for about 27% of all cancer deaths. Approximately 84% of all lung cancers are non-small cell lung cancer (NSCLC), and the five year survival rate is only 18%. American Cancer Society. Cancer Facts & Figures 2015. Atlanta: American Cancer Society. 2015.

Advanced NSCLC remains a difficult to treat cancer where durable long-term survival is exceedingly rare. Chemotherapy, especially platinum-based doublets, is the established treatment for patients with absent or unknown activating mutations for targeted therapy. Histology is an increasingly significant factor when selecting chemotherapy for patients with advanced NSCLC. (Li et al., J Clin Oncol 2013;31: 1039-1049.) A large Phase 3 study in advanced NSCLC found cisplatin plus pemetrexed provided statistically superior overall survival (OS) when compared with cisplatin plus gemcitabine in subjects with either adenocarcinoma or large-cell carcinoma. (Scagliotti, et al., J Clin Oncol 2008;26:3543-3551.) However, in the same study, those subjects with squamous cell histology had a better outcome when treated with gemcitabine plus cisplatin.

A number of driver mutations have been identified recently, allowing for targeted therapies with better outcomes in NSCLC. The Lung Cancer Mutation Consortium found such "actionable" mutations in 64% of the 1,007 patients with adenocarcinoma that they assessed, the most common being *KRAS, EGFR,* and *ALK.* The use of targeted therapies in this study resulted in median OS of 3.5 years in the subset of patients with oncogenic drivers who received targeted therapy versus OS of 2.4 years in those patients with identified mutations but no targeted therapy and 2.1 years in those who did not demonstrate oncogenic drivers ($P$ = 0.001). (Kris et al., JAMA 2014;311:1998-2006.)

Folate receptor alpha (FRA) is a cell surface GPI-anchored protein whose expression and biology is associated with a number of malignant cell types. Antibodies to FRA have been developed and tested in preclinical and clinical studies to evaluate their effect on suppressing cancer growth in patients whose tumors express this antigen. Recently trials in ovarian and lung cancer have been conducted to test the effect of anti-FRA antibodies on these diseases (Armstrong et al., Gynecol. Oncol. 2013 Jun;129(3):452-8; Thomas et al., Lung Cancer. 2013; 80(1):15-8.). Results from these trials demonstrate that the anti-FRA therapy has failed to provide a statistically significant clinical benefit in broad, non-enriched or biomarker-selected, heterogeneous intent-to-treat populations which consist in part of patients with cancers exhibiting varying levels of the FRA antigen as well as other factors that may affect the pharmacologic activity(s) of anti-FRA antibodies (Vergote et al., Cancer Metastasis Rev. 2015 Jan 7, DOI 10.1007/s10555-014-9539-8; Thomas et al., Lung Cancer. 2013; 80(1): 15-8.). While most anti-FRA antibody clinical studies have included all comers, none has selected patients based on FRA expression levels to determine if threshold levels of FRA expression in the tumor may correlate with therapeutic response to anti-FRA antibody therapy. Currently no data suggesting a correlation of the expression level of FRA to the therapeutic response to an anti-FRA antibody therapy exists. Indeed, findings of a correlative relationship between tumor antigen expression and response to antigen-targeted therapy have been difficult to ascertain for a number of tumor antigens. One of the best studied tumor antigens, HER2, is one of the many examples demonstrating lack of expression-clinical benefit correlation whereby patients treated with the anti-HER2 antibody Herceptin (trastuzumab) have been found to have clinical benefit regardless of high or low tumor antigen (Paik et.al; N Engl J Med 2008; 358:1409-1411).

A pressing need exists for methods for identifying NSCLC patients who would be responsive to treatment regimens that target FRA. The methods and kits described herein satisfy this need.

SUMMARY

Embodiments of the present invention are set out in the enclosed claims. In one embodiment, the present invention provides an antibody accoding to the present invention that immunospecifically binds folate receptor alpha (FRA) for use in

(a) a method for treating a FRA-expressing lung cancer in a patient, said method comprising:

determining the patient's FRA expression level in a biological sample of the patient;
comparing the patient's FRA expression level to a reference FRA expression level; and
administering a therapeutically effective amount of said antibody to said patient if said patient's FRA expression level equals or exceeds said reference FRA expression level; or

(b) treating a FRA-expressing lung cancer in a patient, wherein the patient's FRA expression level equals or exceeds a reference FRA expression level;

wherein said reference FRA expression level corresponds to the FRA expression level above which a patient population afflicted with said FRA-expressing lung cancer that is administered said antibody that immunospecifically binds FRA demonstrated a statistically significant improvement in at least one clinical outcome relative to a patient population afflicted with said FRA-expressing lung cancer that is administered placebo;
wherein said reference FRA expression level is

(i) 42% +1 or greater anti-FRA staining;
(ii) 21% +2 or greater anti-FRA staining;
(iii) 14% +3 or greater staining;
(iv) a FRAMSCOR of 7; or
(v) a HBSCOR of 0.25;

and
wherein the antibody according to the present invention that immunospecifically binds FRA is:
an antibody comprising SEQ ID NO:1 as CDRH1, SEQ ID NO:2 as CDRH2, SEQ ID NO:3 as CDRH3, SEQ ID NO:4 as CDRL1, SEQ ID NO:5 as CDRL2 and SEQ ID NO:6 as CDRL3;
an antibody comprising a mature light chain variable region comprising the amino acid sequence of SEQ ID NO:7 and/or a mature heavy chain variable region comprising the amino acid sequence of SEQ ID NO: 8;
farletuzumab;
an antibody that specifically binds to FRA comprising a mature light and heavy chain variable regions having at least 90% and preferably at least 95% or 99% sequence identity to SEQ ID NO: 7 and SEQ ID NO: 8, respectively; or
an antibody or derivative thereof can competitively inhibit binding of farletuzumab to FRA.

In another embodiment, the present invention provides a method for predicting a likelihood of responsiveness to treatment with an antibody according to the present invention that immunospecifically binds folate receptor alpha (FRA) in a patient having a FRA-expressing lung cancer, said method comprising:

determining the patient's FRA expression level in a biological sample of the patient; and
comparing said patient's FRA expression level to a reference FRA expression level,
wherein said reference FRA expression level corresponds to the FRA expression level above which a patient population afflicted with said FRA-expressing lung cancer that is administered said antibody that immunospecifically binds FRA demonstrated a statistically significant improvement in at least one clinical outcome relative to a patient population afflicted with said FRA-expressing lung cancer that is administered placebo;
wherein said reference FRA expression level is

(i) 42% +1 or greater anti-FRA staining;
(ii) 21% +2 or greater anti-FRA staining;
(iii) 14% +3 or greater staining;
(iv) a FRAMSCOR of 7; or
(v) a HBSCOR of 0.25;

wherein said patient is likely to respond to treatment with said antibody that immunospecifically binds FRA if said patient's FRA expression level equals or exceeds said reference FRA expression level, and

wherein the antibody according to the present invention that immunospecifically binds FRA is:

an antibody comprising SEQ ID NO:1 as CDRH1, SEQ ID NO:2 as CDRH2, SEQ ID NO:3 as CDRH3, SEQ ID NO:4 as CDRL1, SEQ ID NO:5 as CDRL2 and SEQ ID NO:6 as CDRL3;
an antibody comprising a mature light chain variable region comprising the amino acid sequence of SEQ ID NO:7 and/or a mature heavy chain variable region comprising the amino acid sequence of SEQ ID NO: 8;
farletuzumab;
an antibody that specifically binds to FRA comprising a mature light and heavy chain variable regions having at least 90% and preferably at least 95% or 99% sequence identity to SEQ ID NO: 7 and SEQ ID NO: 8, respectively; or
an antibody or derivative thereof can competitively inhibit binding of farletuzumab to FRA.

Provided herein are methods for predicting a likelihood of responsiveness to treatment with an antibody according to the present invention that immunospecifically binds FRA in a patient having folate receptor alpha (FRA)-expressing lung cancer. The methods for predicting such likelihood of responsiveness to treatment involve determining the patient's FRA expression level in a biological sample; and comparing the patient's FRA expression level to a reference FRA expression level. The patient is likely to respond to treatment with an antibody according to the present invention that immunospecifically binds FRA if the patient's FRA expression level equals or exceeds the reference FRA expression level.

Also provided herein is an antibody according to the present invention that immunospecifically binds FRA for use in methods for treating folate receptor alpha FRA-expressing lung cancer in a patient. Such methods involve determining the patient's FRA expression level in a biological sample; comparing the patient's FRA expression level to a reference FRA expression level; and administering the antibody according to the present invention that immunospecifically binds FRA to the patient if the patient's FRA expression level equals or exceeds the reference FRA expression level. In some embodiments, a chemotherapeutic agent (e.g., standard of care chemotherapy as described herein) is administered to the patient with or without the antibody according to the present invention that immunospecifically binds FRA.

In some embodiments of the methods for predicting a likelihood of responsiveness to treatment with an antibody according to the present invention that immunospecifically binds FRA and the antibody according to the present invention that immunospecifically binds FRA for use in methods for treating folate receptor alpha FRA-expressing lung cancer in a patient, the antibody that immunospecifically binds FRA comprises farletuzumab. In some embodiments, the antibody that immunospecifically binds FRA is conjugated to a toxin, such as, for example, a microtubule inhibitor, a DNA damaging agent (e.g., a radionuclide), a DNA repair inhibitor, or a signal transduction inhibitor. An exemplary antibody-drug conjugate that can be employed as a FRA-targeting agent in accordance with the methods described herein is IMGN853. It is disclosed that, the FRA-targeting agent is vintafolide.

In some embodiments, the antibody according to the present invention that immunospecifically binds folate receptor alpha (FRA) is for use in methods described herein, wherein the FRA-expressing lung cancer is FRA-expressing non-small cell lung cancer (NSCLC). In some embodiments, the NSCLC is adenocarcinoma.

For each of the aforementioned methods, the reference FRA expression level corresponds to the FRA expression level above which a patient population afflicted with the FRA-expressing lung cancer that is administered the antibody according to the present invention that immunospecifically bind folate receptor alpha (FRA) demonstrates a statistically significant improvement in at least one clinical outcome relative to a patient population afflicted with the FRA-expressing lung cancer that is administered placebo. The improved clinical outcome may be, for example, progression free survival and/or overall survival. In some embodiments, the reference FRA expression level corresponds to 42% +1 or greater anti-FRA staining as described herein. In some embodiments, the reference FRA expression level is 21% +2 or greater anti-FRA staining as described herein. In some embodiments, the reference FRA expression level is 14% +3 or greater staining as described herein. In some embodiments, the reference FRA expression level is a FRAMSCOR (or M-score) of 7. In some embodiments, the reference FRA expression level is a HBSCOR of 0.25.

In some embodiments, the patient populations afflicted with the FRA-expressing lung cancer are further administered a chemotherapeutic agent such as, for example, a taxane, a platinum-containing compound (e.g., cisplatin, carboplatin), and antifolate (e.g., pemetrexed), or any combination thereof.

FRA expression level may be measured in accordance with the methods described herein by protein quantification or RNA quantification. Either cytoplasmic or membranous FRA expression may be measured. In a preferred embodiment, the FRA expression level is measured by immunohistochemical analysis. In preferred embodiments, the FRA expression level is determined by immunoassay with at least one of the following antibodies:

(a) an antibody that binds the same epitope as farletuzumab;
(b) an antibody comprising SEQ ID NO: 1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) an antibody comprising a mature light chain variable region comprising the amino acid sequence of SEQ ID NO:7 and a mature heavy chain variable region comprising the amino acid SEQ ID NO: 8;
(d) farletuzumab;
(e) the 548908 antibody;
(f) an antibody that binds the same epitope as the 548908 antibody;
(g) the 6D398 antibody;
(h) an antibody that binds the same epitope as the 6D398 antibody;
(i) the BN3.2 antibody (Leica Biosystems);
(j) an antibody that binds the same epitope as the BN3.2 antibody;
(k) an antibody that binds the same epitope as the 26B3 antibody;
(1) an antibody comprising SEQ ID NO: 14 (GYFMN) as CDRH1, SEQ ID NO: 15 (RIFPYNGDTFYNQKFKG) as CDRH2, SEQ ID NO: 16 (GTHYFDY) as CDRH3, SEQ ID NO: 17 (RTSENIFSYLA) as CDRL1 , SEQ ID NO: 18 (NAKTLAE) as CDRL2 and SEQ ID NO: 19 (QHHYAFPWT) as CDRL3;
(m) the 26B3 antibody;
(n) an antibody that binds the same epitope as the 19D4 antibody;
(o) an antibody comprising SEQ ID NO: 20 (HPYMH) as CDRH1, SEQ ID NO: 21 (RIDPANGNTKYDPKFQG) as CDRH2, SEQ ID NO: 22 (EEVADYTMDY) as CDRH3, SEQ ID NO: 23 (RASESVDTYGNNFIH) as CDRL1, SEQ ID NO: 24 (LASNLES) as CDRL2 and SEQ ID NO: 25 (QQNNGDPWT) as CDRL3;
(p) the 19D4 antibody;
(q) an antibody that binds the same epitope as the 9F3 antibody;
(r) an antibody comprising SEQ ID NO: 26 (SGYYWN) as CDRH1, SEQ ID NO: 27 (YIKSDGSNNYNPSLKN) as CDRH2, SEQ ID NO: 28 (EWKAMDY) as CDRH3, SEQ ID NO: 29 (RASSTVSYSYLH) as CDRL1, SEQ ID NO: 30 (GTSNLAS) as CDRL2 and SEQ ID NO: 31 (QQYSGYPLT) as CDRL3;
(s) the 9F3 antibody;
(t) an antibody that binds the same epitope as the 24F12 antibody;
(u) an antibody comprising SEQ ID NO: 32 (SYAMS) as CDRH1, SEQ ID NO: 33 (EIGSGGSYTYYPDTVTG) as CDRH2, SEQ ID NO: 34 (ETTAGYFDY) as CDRH3, SEQ ID NO: 35 (SASQGINNFLN) as CDRL1, SEQ ID NO: 36 (YTSSLHS) as CDRL2 and SEQ ID NO: 37 (QHFSKLPWT) as CDRL3;
(v) the 24F12 antibody;
(w) an antibody that comprises a variable region light chain selected from the group consisting of:

(i) LK26HuVK;
(ii) LK26HuVKY;
(iii) LK26HuVKPW; and
(iv) LK26HuVKPW,Y;

(x) an antibody that comprises a variable region heavy chain selected from the group consisting of:

(i) LK26HuVH;
(ii) LK26HuVH FAIS,N;
(iii) LK26HuVH SLF;
(iv) LK26HuVH I,I;
(v) LK26KOLHuVH;

(y) an antibody that comprises the heavy chain variable region LK26KOLHuVH (SEQ ID NO: 46) and the light chain variable region LK26HuVKPW,Y (SEQ ID NO: 41);
(z) an antibody that comprises the heavy chain variable region LK26HuVH SLF (SEQ ID NO: 44) and the light chain variable region LK26HuVKPW,Y (SEQ ID NO: 41);
(aa) an antibody that comprises the heavy chain variable region LK26HuVH FAIS,N (SEQ ID NO: 43) and the light chain variable region LK26HuVKPW,Y (SEQ ID NO: 41); and
(bb) the murine monoclonal LK26 antibody.

In the methods described herein, FRA expression level may be assessed by digital imaging technology or manual pathology quantification. In some embodiments, the FRA expression level is assessed by FRAMSCOR or HBSCOR.

The biological sample assayed in accordance with the methods described herein may be urine, blood, serum, plasma, saliva, circulating cells, circulating tumor cells, or tumor tissue (e.g., pleural tissue). In some embodiments, the biological sample comprises pleural cells derived from effusion.

The described methods may further involve administering a chemotherapeutic agent to the patient regardless of whether the patient's FRA expression level equals or exceeds the reference FRA expression level. In a preferred embodiment, the chemotherapeutic agent comprises a platinum-containing compound, such as cisplatin or carboplatin; a taxane (for example, paclitaxel); an antifolate (e.g., pemetrexed); or any combination thereof. For example, in the methods described herein, the patient may be administered carboplatin and paclitaxel; carboplatin and pemetrexed; or cisplatin and pemetrexed.

Additional aspects of the summarized subject matter are provided in greater detail in the detailed description and provided examples and associated figures.

## BRIEF DESCRIPTION OF THE DRAWINGS

The summary, as well as the following detailed description, is further understood when read in conjunction with the appended drawings. For the purpose of illustrating the disclosed methods, there are shown in the drawings exemplary embodiments of the methods; however, the methods are not limited to the specific embodiments disclosed. In the drawings:

Figure 1 shows pleural tissue suitability for FRA quantification via FRAMSCOR and HBSCOR. Normal (left top panel) or malignant non-small cell lung adenocarcinoma (NSCLC) tissue samples (all other panels) were procured from patients. Tissues were formalin-fixed and sectioned onto glass slides and stained for FRA expression using the 26B3 anti-FRA antibody. Stained slides were visualized by microscopy and documented via photography. This figure represents pleural tissue specimen and staining that is suitable for quantified FRA cytoplasmic or membrane staining (top row). Specimens with poor tissue preservation, poor tissue morphology or over staining as well as those consisting of malignant cells in pleural effusions, not pleural tissue, are omitted from the FRA expression clinical outcome correlation study. Examples of the latter are shown on the bottom row. Cells derived from effusions may be used to measure the minimal FRA levels needed for anti-FRA therapeutic response.

Figure 2 shows a representative sample of a reference data set used for scoring +1 (low expression), +2 (moderate levels) and +3 (high levels) of FRA expression in malignant pleural tissue.

Figure 3 shows a representative expression cut-point analysis measuring cytoplasmic levels of FRA to clinical outcome (overall survival) using HBSCOR method. A significant improvement in response is observed in patients whose FRA expression level exceeds a HBSCOR of ~0.29 or greater. At this level, significant clinical responses (Hazard Ratios of < 0.5) are observed as compared to patients whose tumor FRA expression level is less than this value. Positive clinical outcomes (Hazard Ratio < 0.7) are observed in patients treated with farletuzumab with a HBSCOR of 0.25 or greater.

Figures 4A and 4B illustrate clinical responses of patients treated with standard-of-care (SOC) chemotherapy +/- farletuzumab and expressing high levels of membrane localized (FRAMSCOR) FRA. Panel A shows a representative measurement of overall survival (OS) in patients with high levels of FRA using FRAMSCOR method whereby patients treated with farletuzumab + SOC (blue line; triangle) and a FRAMSCOR of greater than 7 showed a clinically meaningful improvement in OS, over placebo + SOC treated patients (red line; circle) 18.3 mo vs 10.0 mo (Hazard Ratio 0.54; p = 0.0266), respectively. Similar clinical benefit was observed when measuring PFS. Panel B shows that patients treated with farletuzumab + SOC (blue line; triangle) with a FRAMSCOR of less than 7 did not have a clinically meaningful improvement in OS over placebo + SOC treated patients (red line; circle) (p = 0.386).

Figures 5A and 5B illustrate clinical responses of patients treated with standard-of-care chemotherapy +/- farletuzumab and expressing high levels of cytoplasmic FRA (Panel B) vs patients expressing low levels of cytoplasmic FRA (Panel A) as determined using the HBSCOR method. Panel A shows a representative measurement of overall survival (OS) in patients with low levels of FRA whereby patients having a suboptimal HBSCOR (HBSCOR < 0.38) had no clinical improvement when treated with farletuzumab (closed circle) as compared to placebo control (open circle) (Hazard Ratio 1.03; p = 0.5389). Panel B shows statistically significant improvement in overall survival in patients having an optimal HBSCOR (HBSCOR > 0.38; Hazard Ratio 0.24; p = 0.0069) which translates into an 8.8 month improvement in overall survival. Similar effects were observed when measuring PFS.

## DETAILED DESCRIPTION OF ILLUSTRATIVE EMBODIMENTS

The disclosed methods may be understood more readily by reference to the following detailed description taken in connection with the accompanying figures, which form a part of this disclosure. It is to be understood that the disclosed methods are not limited to the specific methods described and/or shown herein, and that the terminology used herein is for the purpose of describing particular embodiments by way of example only and is not intended to be limiting of the claimed methods.

Similarly, unless specifically otherwise stated, any description as to a possible mechanism or mode of action or reason for improvement is meant to be illustrative only, and the disclosed methods are not to be constrained by the correctness or incorrectness of any such suggested mechanism or mode of action or reason for improvement.

It is to be appreciated that certain features of the disclosed methods which are, for clarity, described herein in the context of separate embodiments, may also be provided in combination in a single embodiment. Conversely, various features of the disclosed methods that are, for brevity, described in the context of a single embodiment, may also be provided separately or in any subcombination.

As used herein, the singular forms "a," "an," and "the" include the plural.

The term "about" as used herein when referring to a measurable value such as an amount, a temporal duration, and the like, is meant to encompass variations of up to $\pm 10\%$ from the specified value, as such variations are appropriate to perform the disclosed methods. Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

The term "antibody" refers to (a) immunoglobulin polypeptides, i.e., polypeptides of the immunoglobulin family that contain an antigen binding site that specifically binds to a specific antigen (e.g., folate receptor alpha), including all immunoglobulin isotypes (IgG, IgA, IgE, IgM, IgD, and IgY), classes (e.g. IgG1, IgG2, IgG3, IgG4, IgAl, IgA2), subclasses, and various monomeric and polymeric forms of each isotype, unless otherwise specified, and (b) conservatively substituted variants of such immunoglobulin polypeptides that immunospecifically bind to the antigen (e.g., folate receptor alpha). Antibodies are generally described in, for example, Harlow & Lane, Antibodies: A Laboratory Manual (Cold Spring Harbor Laboratory Press, 1988). Unless otherwise apparent from the context, reference to an antibody also includes antibody derivatives as described in more detail below.

"Antibody fragments" comprise a portion of a full length antibody, generally the antigen-binding or variable region thereof, such as Fab, Fab', $F(ab')_2$, and Fv fragments; diabodies; biobodies; linear antibodies; single-chain antibody molecules; and multi specific antibodies formed from antibody fragments. Various techniques have been developed for the production of antibody fragments, including proteolytic digestion of antibodies and recombinant production in host cells; however, other techniques for the production of antibody fragments will be apparent to the skilled practitioner. In some embodiments, the antibody fragment of choice is a single chain Fv fragment (scFv). "Single-chain Fv" or "scFv" antibody fragments comprise the $V_H$ and $V_L$ domains of antibody, wherein these domains are present in a single polypeptide chain. Generally, the Fv polypeptide further comprises a polypeptide linker between the $V_H$ and $V_L$ domains which enables the scFv to form the desired structure for antigen binding. For a review of scFv and other antibody fragments, see James D. Marks, Antibody Engineering, Chapter 2, Oxford University Press (1995) (Carl K. Borrebaeck, Ed.).

An "antibody derivative" means an antibody, as defined above, that is modified by covalent attachment of a heterologous molecule such as, e.g., by attachment of a heterologous polypeptide (e.g., a cytotoxin) or therapeutic agent (e.g., a chemotherapeutic agent), or by glycosylation, deglycosylation, acetylation or phosphorylation not normally associated with the antibody, and the like.

The term "monoclonal antibody" refers to an antibody that is derived from a single cell clone, including any eukaryotic or prokaryotic cell clone, or a phage clone, and not the method by which it is produced. Thus, the term "monoclonal antibody" is not limited to antibodies produced through hybridoma technology.

An "antigen" is an entity to which an antibody specifically binds. For example, folate receptor alpha is the antigen to which an anti-folate receptor-alpha antibody specifically binds.

A FRA targeting agent is a therapeutic agent that targets or exerts its effect through FRA. FRA targeting agents include but are not limited to FRA-binding proteins, such as antibodies that immunospecifically bind FRA and antigen-binding fragments thereof such a farletuzumab; drug conjugates of such antibodies and antigen-binding fragments such as IMGN853 (Immunogen); and small molecules such as vintafolide (EC 145; Endocyte). Vintafolide is a folate-desacetylvinblastine monohydrazide conjugate, which allowings liberation of the drug into the cytoplasm of cancerous cells via the FRA and endocytosis. In preferred embodiments, the FRA targeting agent is farletuzumab.

The terms "cancer" and "tumor" are well known in the art and refer to the presence, e.g., in a subject, of cells possessing characteristics typical of cancer-causing cells, such as uncontrolled proliferation, immortality, metastatic potential, rapid growth and proliferation rate, and certain characteristic morphological features. Cancer cells are often in the form of a tumor, but such cells may exist alone within a subject, or may be non-tumorigenic cancer cells, such as leukemia cells. As used herein, the term "cancer" includes pre-malignant as well as malignant cancers.

As used herein, the term "folate receptor alpha" (also referred to as FRA, FR-alpha, FOLR-1 or FOLR1) refers to the alpha isoform of the high affinity receptor for folate. Membrane bound FRA is attached to the cell surface by a glycosyl phosphatidylinositol (GPI) anchor, recycles between membrane and endocytic compartments and is capable of transporting folate into the cell. FRA is expressed in a variety of epithelial tissues including those of the female reproductive tract, placenta, breast, kidney proximal tubules, choroid plexus, lung and salivary glands. Soluble forms of FRA include but are not limited to those derived by the action of proteases or phospholipase on membrane anchored folate receptors.

The consensus nucleotide and amino acid sequences for human FRA are set forth herein as SEQ ID NOs: 9 and 10, respectively.

```
SEQ ID NO: 9
tcaaggttaa acgacaagga cagacatggc tcagcggatg acaacacagc tgctgctcct   60

tctagtgtgg gtggctgtag taggggaggc tcagacaagg attgcatggg ccaggactga  120

gcttctcaat gtctgcatga acgccaagca ccacaaggaa aagccaggcc ccgaggacaa  180

gttgcatgag cagtgtcgac cctggaggaa gaatgcctgc tgttctacca acaccagcca  240

ggaagcccat aaggatgttt cctacctata tagattcaac tggaaccact gtggagagat  300

ggcacctgcc tgcaaacggc atttcatcca ggacacctgc ctctacgagt gctcccccaa  360

cttggggccc tggatccagc aggtggatca gagctggcgc aaagagcggg tactgaacgt  420

gcccctgtgc aaagaggact gtgagcaatg gtgggaagat tgtcgcacct cctacacctg  480

caagagcaac tggcacaagg gctggaactg gacttcaggg tttaacaagt gcgcagtggg  540

agctgcctgc caacctttcc atttctactt ccccacaccc actgttctgt gcaatgaaat  600

ctggactcac tcctacaagg tcagcaacta cagccgaggg agtggccgct gcatccagat  660

gtggttcgac ccagcccagg gcaaccccaa tgaggaggtg gcgaggttct atgctgcagc  720

catgagtggg gctgggccct gggcagcctg gcctttcctg cttagcctgg ccctaatgct  780

gctgtggctg ctcagctgac ctccttttac cttctgatac ctggaaatcc ctgccctgtt  840

cagccccaca gctcccaact atttggttcc tgctccatgg tcgggcctct gacagccact  900

ttgaataaac cagacaccgc acatgtgtct tgagaattat ttggaaaaaa aaaaaaaaaa  960

aa                                                                 962
```

```
SEQ ID NO: 10
Met Ala Gln Arg Met Thr Thr Gln Leu Leu Leu Leu Leu Val Trp Val

Ala Val Val Gly Glu Ala Gln Thr Arg Ile Ala Trp Ala Arg Thr Glu

Leu Leu Asn Val Cys Met Asn Ala Lys His His Lys Glu Lys Pro Gly

Pro Glu Asp Lys Leu His Glu Gln Cys Arg Pro Trp Arg Lys Asn Ala
```

Cys Cys Ser Thr Asn Thr Ser Gln Glu Ala His Lys Asp Val Ser Tyr
Leu Tyr Arg Phe Asn Trp Asn His Cys Gly Glu Met Ala Pro Ala Cys
Lys Arg His Phe Ile Gln Asp Thr Cys Leu Tyr Glu Cys Ser Pro Asn
Leu Gly Pro Trp Ile Gln Gln Val Asp Gln Ser Trp Arg Lys Glu Arg
Val Leu Asn Val Pro Leu Cys Lys Glu Asp Cys Glu Gln Trp Trp Glu
Asp Cys Arg Thr Ser Tyr Thr Cys Lys Ser Asn Trp His Lys Gly Trp
Asn Trp Thr Ser Gly Phe Asn Lys Cys Ala Val Gly Ala Ala Cys Gln
Pro Phe His Phe Tyr Phe Pro Thr Pro Thr Val Leu Cys Asn Glu Ile
Trp Thr His Ser Tyr Lys Val Ser Asn Tyr Ser Arg Gly Ser Gly Arg
Cys Ile Gln Met Trp Phe Asp Pro Ala Gln Gly Asn Pro Asn Glu Glu
Val Ala Arg Phe Tyr Ala Ala Ala Met Ser Gly Ala Gly Pro Trp Ala
Ala Trp Pro Phe Leu Leu Ser Leu Ala Leu Met Leu Leu Trp Leu Leu
Ser

Variants, for example, naturally occurring allelic variants or sequences containing at least one amino acid substitution, are encompassed by the terms as used herein.

As used herein, the term "not bound to a cell" refers to a protein that is not attached to the cellular membrane of a cell, such as a cancerous cell. In a particular embodiment, the FRA not bound to a cell is unbound to any cell and is freely floating or solubilized in biological fluids, e.g., urine, serum, plasma, or pleural effusion. For example, a protein that is not bound to a cell may be shed, secreted or exported from normal or cancerous cells, for example, from the surface of cancerous cells, into biological fluids.

The "level" or "expression level" of a specified RNA, as used herein, refers to the level of the RNA as determined using any method known in the art for the measurement of RNA levels. Such methods include, but are not limited to, spectrophotometry (e.g., ultraviolet absorbance), fluorometry, hybridization assays, and microcapillary electrophoresis.

The "level" or "expression level" of a specified protein, as used herein, refers to the level of the protein as determined using any method known in the art for the measurement of protein levels. Such methods include, for example, electrophoresis, capillary electrophoresis, high performance liquid chromatography (HPLC), thin layer chromatography (TLC), hyperdiffusion chromatography, fluid or gel precipitation reactions, absorption spectroscopy, colorimetric assays, spectrophotometric assays, flow cytometry, immunodiffusion (single or double), solution phase assay, immunofluorimetry, immunoprecipitation, equilibrium dialysis, immunodiffusion, solution phase assay, immunoelectrophoresis, Western blotting, radioimmunoassay (RIA), enzyme-linked immunosorbent assays (ELISAs), immunofluorescent assays, and electrochemiluminescence immunoassay (exemplified below), and the like. In a preferred embodiment, the level is determined using antibody-based techniques, as described in more detail herein.

Antibodies used in immunoassays to determine the level of expression of a specified protein, such as for example FRA, may be labeled with a detectable label. The term "labeled", with regard to the binding agent or antibody, is intended to encompass direct labeling of the binding agent or antibody by coupling (i.e., physically linking) a detectable substance to the binding agent or antibody, as well as indirect labeling of the binding agent or antibody by reactivity with another reagent that is directly labeled. An example of indirect labeling includes detection of a primary antibody using a fluorescently labeled secondary antibody. In one embodiment, the antibody is labeled, e.g., radio-labeled, chromophore-labeled, fluorophore-labeled, or enzyme-labeled. In another embodiment, the antibody is an antibody derivative (e.g., an antibody conjugated with a substrate or with the protein or ligand of a protein-ligand pair (e.g., biotin-streptavidin), or an antibody fragment (e.g., a single-chain antibody, an isolated antibody hypervariable domain).

Expression levels of a specific marker (e.g., FRA) may be determined by any methods of RNA or protein quantification known in the art. Such methods include, but are not limited to, spectrophotometry (e.g., ultraviolet absorbance), fluorometry, hybridization assays, electrophoresis, capillary electrophoresis, high performance liquid chromatography (HPLC), thin layer chromatography (TLC), hyperdiffusion chromatography, fluid or gel precipitation reactions, absorption spectroscopy, colorimetric assays, spectrophotometric assays, flow cytometry, immunodiffusion (single or double), solution phase assay, immunofluorimetry, immunoprecipitation, equilibrium dialysis, immunodiffusion, solution phase assay, immunoelectrophoresis, Western blotting, radioimmunoassay (RIA), enzyme-linked immunosorbent assays (ELTSAs), immunofluorescent assays, and electrochemiluminescence immunoassay (exemplified below), and the like. In a preferred embodiment, the level is determined using antibody-based techniques, as described in more detail herein. In a preferred embodiment, immunohistochemical analysis (e.g., of tumor tissue) is employed. In one embodiment, proteomic methods, e.g., mass spectrometry, are used. Mass spectrometry is an analytical technique that consists of ionizing chemical compounds to generate charged molecules (or fragments thereof) and measuring their mass-to-charge ratios. In a typical mass spectrometry procedure, a sample is obtained from a subject, loaded onto the mass spectrometry, and its components (e.g., FRA) are ionized by different methods (e.g., by impacting them with an electron beam), resulting in the formation of charged particles (ions). The mass-to-charge ratio of the particles is then calculated from the motion of the ions as they transit through electromagnetic fields.

For example, matrix-associated laser desorption/ionization time-of-flight mass spectrometry (MALDI-TOF MS) or surface-enhanced laser desorption/ionization time-of-flight mass spectrometry (SELDI-TOF MS) which involves the application of a sample, such as urine or serum, to a protein-binding chip (Wright, G.L., Jr., et al. (2002) Expert Rev Mol Diagn 2:549; Li, J., et al. (2002) Clin Chem 48:1296; Laronga, C., et al. (2003) Dis Markers 19:229; Petricoin, E.F., et al. (2002) 359:572; Adam, B.L., et al. (2002) Cancer Res 62:3609; Tolson, J., et al. (2004) Lab Invest 84:845; Xiao, Z., et al. (2001) Cancer Res 61:6029) can be used to determine the level of FRA.

Furthermore, *in vivo* techniques for determination of the level of a marker (e.g., FRA) include introducing into a subject a labeled antibody directed against the marker, which binds to the marker to enable its detection. The presence, level, or location of the detectable marker in a subject may be determined using standard imaging techniques (e.g., PET).

As used herein, a subject who is "afflicted with" or "having lung cancer" is one who is clinically diagnosed with lung cancer at any stage by a qualified clinician, or one who exhibits one or more signs or symptoms of such a cancer and is subsequently clinically diagnosed with such a cancer by a qualified clinician. A non-human subject that serves as an animal model of folate receptor-alpha-expressing lung cancer may also fall within the scope of a subject "afflicted with folate receptor-alpha-expressing lung cancer."

As used herein, a "folate receptor-alpha-expressing lung cancer" includes any type of lung cancer characterized in that the cancer cells express or present on their surface folate receptor alpha. A lung cancer may have been, but is not required to have been, clinically diagnosed as expressing FRA to be encompassed by the term "folate receptor-alpha-expressing lung cancer" as used herein. The phrase "folate receptor-alpha-expressing lung cancer" specifically includes FRA-expressing non-small cell lung cancer (NSCLC) and FRA-expressing non-small cell lung adenocarcinoma.

The term "sample" or "biological sample" as used herein refers to a collection of similar fluids, cells, or tissues isolated from a subject, as well as fluids, cells, or tissues present within a subject. The sample assessed for the expression level of FRA may be derived from urine, blood, serum, plasma, pleural effusion, sputum, bronchial washings, circulating cells, circulating tumor cells, cells that are not tissue associated (i.e., free cells), tissues (e.g., pleural tissue, surgically resected tumor tissue, biopsies, including fine needle aspiration), histological preparations, and the like.

In some embodiments, only a portion of the sample is subjected to an assay for determining the level of a marker, or various portions of the sample are subjected to various assays for determining the level of a marker. Also, in many embodiments, the sample may be pre-treated by physical or chemical means prior to the assay. For example, samples, may be subjected to centrifugation, dilution and/or treatment with a solubilizing substance (e.g., guanidine treatment) prior to assaying the samples for a marker. Such techniques serve to enhance the accuracy, reliability and reproducibility of the assays.

The term "reference expression level" when used to describe the level of expression of a marker (e.g., FRA) refers to an accepted or pre-determined level of a marker which is used to compare with the level of the marker in a sample derived from a subject. In one embodiment, the reference expression level of FRA is predetermined using a population of patients afflicted with the FRA-expressing lung cancer that are treated with a FRA targeting agent (e.g., an antibody that immunospecifically binds FRA) relative to a population of patients afflicted with the FRA-expressing lung cancer that receive placebo in lieu of the FRA targeting agent. The patient populations may have received standard-of-care therapy for such FRA-expressing lung cancer in addition to the antibody or placebo.

As used herein, the term "contacting the sample" with a specific binding agent, e.g., an antibody, includes exposing the sample, or any portion thereof with the agent, such that at least a portion of the sample comes into contact with the agent. The sample or portion thereof may be altered in some way, such as by subjecting it to physical or chemical treatments (e.g., dilution or guanidine treatment), prior to the act of contacting it with the agent.

The term "inhibit" or "inhibition of" means to reduce by a measurable amount, or to prevent entirely.

The term "deplete," in the context of the effect of an anti-FRA therapeutic agent on folate receptor alpha-expressing cells, refers to a reduction in the number of, or elimination of, the folate receptor alpha-expressing cells.

The term "functional," in the context of an antibody to be used in accordance with the methods described herein, indicates that the antibody is (1) capable of binding to antigen and/or (2) depletes or inhibits the proliferation of antigen-expressing cells.

The term "treatment" or "treat" or "positive therapeutic response" refers to slowing, stopping, or reversing the progression of a folate receptor alpha-expressing lung cancer in a patient, as evidenced by a decrease or elimination of a clinical or diagnostic symptom of the disease, by administration of a FRA targeting agent to the subject after the onset of a clinical or diagnostic symptom of the folate receptor alpha-expressing lung cancer at any clinical stage. Treatment can include, for example, a decrease in the severity of a symptom, the number of symptoms, or frequency of relapse, depletion of FRA-expressing lung cancer cells, inhibition of the growth of FRA-expressing lung cancer cells, or a statistically significant and/or clinically relevant improvement in a specific clinical outcome (e.g., progression-free survival, overall survival).

The phrase "responsive to treatment with a FRA targeting agent" is intended to mean that the candidate subject (i.e., an individual with FRA-expressing lung cancer), following administration of the FRA targeting agent, would have a positive therapeutic response with respect to the lung cancer.

The term "pharmaceutically acceptable" refers to those properties and/or substances which are acceptable to the patient from a pharmacological/ toxicological point of view and to the manufacturing pharmaceutical chemist from a physical/chemical point of view regarding composition, formulation, stability, patient acceptance and bioavailability and includes properties and/or substances approved by a regulatory agency of the Federal or a state government or listed in the U.S. Pharmacopeia or other generally recognized pharmacopeia for use in animals, and more particularly in humans. The term "pharmaceutically compatible ingredient" refers to a pharmaceutically acceptable diluent, adjuvant, excipient, or vehicle with which an anti-folate receptor alpha antibody is administered. "Pharmaceutically acceptable carrier" refers to a medium that does not interfere with the effectiveness of the biological activity of the active ingredient(s) and is not toxic to the host to which it is administered.

The terms "effective amount" and "therapeutically effective amount" are used interchangeably herein and, in the context of the administration of a pharmaceutical agent, refer to the amount of the agent (e.g., a FRA targeting agent) that is sufficient to inhibit the occurrence or ameliorate one or more clinical or diagnostic symptoms of a folate receptor alpha-expressing lung cancer in a patient. A therapeutically effective amount of an agent may vary according to factors such as the disease state, age, sex, and weight of the individual, and the ability of the agent to elicit a desired response in the individual. Such results may include, but are not limited to, the treatment of a folate-receptor alpha-expressing lung cancer, as determined by any means suitable in the art. An effective amount of an agent is administered according to the methods described herein in an "effective regimen." The term "effective regimen" refers to a combination of amount of the agent and dosage frequency adequate to accomplish treatment of a folate receptor alpha-expressing lung cancer.

The terms "patient" and "subject" are used interchangeably to refer to humans and other non-human animals, including veterinary subjects, that receive diagnostic, prophylactic or therapeutic treatment. The term "non-human animal" includes all vertebrates, e.g., mammals and non-mammals, such as non-human primates, mice, rabbits, sheep, dog, cat, horse, cow, chickens, amphibians, and reptiles. In a preferred embodiment, the subject is a human.

Therapeutic agents are typically substantially pure from undesired contaminants. This means that an agent is typically at least about 50% w/w (weight/weight) pure as well as substantially free from interfering proteins and contaminants. Sometimes the agents are at least about 80% w/w and, more preferably at least 90 or about 95% w/w pure. However, using conventional protein purification techniques, homogeneous peptides of at least 99% purity w/w can be obtained.

**Methods for predicting a likelihood of responsiveness to treatment with a FRA targeting agent**

Provided herein are methods for predicting a likelihood of responsiveness to treatment with an antibody according to the present invention that immunospecifically binds FRA in a patient having a folate receptor alpha (FRA)-expressing lung cancer. In some embodiments of the methods for predicting a likelihood of responsiveness to treatment with an antibody according to the present invention that immunospecifically binds FRA, the FRA-expressing lung cancer is non-small cell lung cancer (NSCLC). In some embodiments, the NSCLC is adenocarcinoma.

The described methods for predicting a likelihood of responsiveness to treatment with an antibody according to the present invention that immunospecifically binds FRA in a patient having a folate receptor alpha (FRA)-expressing lung cancer involve determining the patient’s FRA expression level in a biological sample of the patient.

Determination of the level of expression of FRA in a biological sample of a patient may be performed upon diagnosis, upon surgical resection, upon initiation of first-line therapy, upon completion of first-line therapy, upon symptomatic progression, serologic progression, and/or radiologic progression of the cancer, upon initiation of second or greater-line therapy, and/or upon completion of such therapy.

In the disclosed methods for predicting a likelihood of responsiveness to treatment with an antibody according to the present invention that immunospecifically binds FRA in a patient having a folate receptor alpha (FRA)-expressing lung cancer, the FRA expression level may be determined by any means known in the art including known methods of RNA or protein quantification. Such methods include, but are not limited to, use of antibody to detect protein expression, nucleic acid hybridization, quantitative RT-PCR, immunoprecipitation, equilibrium dialysis, immunodiffusion, immunohistochemistry, fluorescence-activated cell sorting (FACS), fluorimetry, hybridization assays, electrophoresis, capillary electrophoresis, high performance liquid chromatography (HPLC), thin layer chromatography (TLC), hyperdiffusion chromatography, fluid or gel precipitation reactions, absorption spectroscopy, colorimetric assays, spectrophotometric assays, flow cytometry, immunodiffusion (single or double), solution phase assay, immunoprecipitation, equilibrium dialysis, immunodiffusion, solution phase assay, immunoelectrophoresis, Western blotting, radioimmunoassay (RIA), enzyme-linked immunosorbent assays (ELISAs), immunofluorescent assays, and electrochemiluminescence immunoassay, and the like. In a preferred embodiment, the level is determined using antibody-based techniques, as described in more detail herein. In a preferred embodiment, immunohistochemical analysis (e.g., of pleural tissue) is employed. The step of determining the expression level of FRA may be performed ex vivo or in vivo.

For *ex vivo* assessments, the biological sample used in determining the level of FRA expression may be urine, whole blood, serum, plasma, pleural effusion, sputum, bronchial washings, circulating cells, circulating tumor cells, cells that are not tissue associated (i.e., free cells), tissues (e.g., pleural tissue, surgically resected tumor tissue, biopsies, including fine needle aspiration), histological preparations, and the like. The tissue sample on which the assay is performed can be fixed or frozen to permit histological sectioning. Preferably, the excised tissue samples are fixed in aldehyde fixatives such as formaldehyde, paraformaldehyde, glutaraldehyde; or heavy metal fixatives such as mercuric chloride. More preferably, the excised tissue samples are fixed in formalin and embedded in paraffin wax prior to incubation with the antibody. Optionally, FFPE specimens can be treated with citrate, EDTA, enzymatic digestion or heat to increase accessibility of epitopes. Alternatively, a protein fraction can be isolated from cells from known or suspected lung cancer and analyzed by ELISA, Western blotting, immunoprecipitation or the like. In another variation, cells can be analyzed for expression of folate receptor alpha by FACS analysis. In a further variation, mRNA can be extracted from cells from known or suspected lung cancer. The mRNA or a nucleic acid derived therefrom, such as a cDNA can then be analyzed by hybridization to a nucleic probe binding to DNA encoding folate receptor alpha.

For example, the step of determining FRA expression level may involve determining the level of FRA expression in a biological sample of the lung cancer tissue obtained from the subject. FRA expression levels may be determined by an immunoassay in which a sample containing cells known or suspected to be from a cancer (e.g., lung cancer) is contacted with an anti-FRA antibody or antigen-binding fragment. After contact, the presence or absence of a binding event of the antibody or antigen-binding fragment to the cells in the specimen is determined. The binding is related to the presence or absence of the antigen expressed on cancerous cells in this specimen. Generally, the sample is contacted with a labeled specific binding partner of the anti-FRA antibody or antigen-binding fragment capable of producing a detectable signal. Alternatively, the anti-FRA antibody or fragment itself can be labeled. Examples of types of labels include enzyme labels, radioisotopic labels, nonradioactive labels, fluorescent labels, toxin labels and chemiluminescent labels. Many such labels are readily known to those skilled in the art. For example, suitable labels include, but should not be considered limited to, radiolabels, fluorescent labels (such as DyLight® 649), epitope tags, biotin, chromophore labels, ECL labels, or enzymes. More specifically, the described labels include ruthenium,
$^{111}$In-DOTA, $^{111}$In- diethylenetriaminepentaacetic acid (DTPA), horseradish peroxidase, alkaline phosphatase and beta-galactosidase, poly-histidine (HIS tag), acridine dyes, cyanine dyes, fluorone dyes, oxazin dyes, phenanthridine dyes, rhodamine dyes, Alexafluor® dyes, and the like. Detection of a signal from the label indicates the presence of the antibody or fragment specifically bound to folate receptor alpha in the sample.

As mentioned above, in some embodiments, FRA expression level may be determined by an immunoassay in which a sample containing cells known or suspected to be from a cancer (e.g., lung cancer) is contacted with an anti-FRA antibody or antigen-binding fragment. In some embodiments, the FRA is not bound to a cell in the sample. Methods for determining the expression level of FRA in a sample derived from the subject are disclosed, for example, in U.S. Publ. No. 20130017195. Methods for determining the expression level of FRA which is not bound to a cell in a sample derived from the subject are disclosed, for example, in U.S. Publ. No. 20120207771. The sample employed in the determination of the expression level of FRA may be derived from urine, blood, serum, plasma, pleural effusion, sputum, bronchial washings, circulating cells, circulating tumor cells, cells that are not tissue associated (i.e., free cells), tissues (e.g., pleural tissue, surgically resected tumor tissue, biopsies, including fine needle aspiration), histological preparations, and the like, for example. In preferred embodiments, the sample is tissue, urine, or serum.

In various aspects, the expression level of FRA is determined by contacting the sample with an antibody that binds FRA. For example, the anti-FRA antibody may be selected from the group consisting of:

(a) an antibody that binds the same epitope as the MORAb-003 antibody;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3; );
(c) an antibody comprising a mature light chain variable region comprising the amino acid sequence of SEQ ID NO:7:

```
  1  DIQLTQSPSS LSASVGDRVT ITCSVSSSIS SNNLHWYQQK PGKAPKPWIY
 51  GTSNLASGVP SRFSGSGSGT DYTFTISSLQ PEDIATYYCQ QWSSYPYMYT
101  FGQGTKVEIK RTVAAPSVFI FPPSDEQLKS GTASVVCLLN NFYPREAKVQ
151  WKVDNALQSG NSQESVTEQD SKDSTYSLSS TLTLSKADYE KHKVYACEVT
201  HQGLSSPVTK SFNRGEC
```

and a mature heavy chain variable region comprising the amino acid SEQ ID NO: 8:

```
  1  EVQLVESGGG VVQPGRSLRL SCSASGFTFS GYGLSWVRQA PGKGLEWVAM
 51  ISSGGSYTYY ADSVKGRFAI SRDNAKNTLF LQMDSLRPED TGVYFCARHG
101  DDPAWFAYWG QGTPVTVSSA STKGPSVFPL APSSKSTSGG TAALGCLVKD
151  YFPEPVTVSW NSGALTSGVH TFPAVLQSSG LYSLSSVVTV PSSSLGTQTY
201  ICNVNHKPSN TKVDKKVEPK SCDKTHTCPP CPAPELLGGP SVFLFPPKPK
251  DTLMISRTPE VTCVVVDVSH EDPEVKFNWY VDGVEVHNAK TKPREEQYNS
301  TYRVVSVLTV LHQDWLNGKE YKCKVSNKAL PAPIEKTISK AKGQPREPQV
351  YTLPPSRDEL TKNQVSLTCL VKGFYPSDIA VEWESNGQPE NNYKTTPPVL
401  DSDGSFFLYS KLTVDKSRWQ QGNVFSCSVM HEALHNHYTQ KSLSLSPGK
```

(CDRs underlined);

(d) the MORAb-003 antibody (USAN name: farletuzumab), as described in U.S. Publ. No. 20090274697 and U.S. Patent No. 8,124,083;

(e) the 548908 antibody (Novus; catalog no. MAB5646);

(f) an antibody that binds the same epitope as the 548908 antibody;

(g) the 6D398 antibody (USBiological Life Sciences);

(h) an antibody that binds the same epitope as the 6D398 antibody;

(i) the BN3.2 antibody (Leica Biosystems);

(j) an antibody that binds the same epitope as the BN3.2 antibody;

(k) an antibody that binds the same epitope as the 26B3 antibody;

(1) an antibody comprising SEQ ID NO: 14 (GYFMN) as CDRH1, SEQ ID NO: 15 (RIFPYNGDTFYNQKFKG) as CDRH2, SEQ ID NO: 16 (GTHYFDY) as CDRH3, SEQ ID NO: 17 (RTSENIFSYLA) as CDRL1 , SEQ ID NO: 18 (NAKTLAE) as CDRL2 and SEQ ID NO: 19 (QHHYAFPWT) as CDRL3;

(m) the 26B3 antibody;

(n) an antibody that binds the same epitope as the 19D4 antibody;

(o) an antibody comprising SEQ ID NO: 20 (HPYMH) as CDRH1, SEQ ID NO: 21 (RIDPANGNTKYDPKFQG) as CDRH2, SEQ ID NO: 22 (EEVADYTMDY) as CDRH3, SEQ ID NO: 23 (RASESVDTYGNNFIH) as CDRL1, SEQ ID NO: 24 (LASNLES) as CDRL2 and SEQ ID NO: 25 (QQNNGDPWT) as CDRL3;

(p) the 19D4 antibody (see U.S. Patent 8,475,795);

(q) an antibody that binds the same epitope as the 9F3 antibody;

(r) an antibody comprising SEQ ID NO: 26 (SGYYWN) as CDRH1, SEQ ID NO: 27 (YIKSDGSNNYNPSLKN) as CDRH2, SEQ ID NO: 28 (EWKAMDY) as CDRH3, SEQ ID NO: 29 (RASSTVSYSYLH) as CDRL1, SEQ ID NO: 30 (GTSNLAS) as CDRL2 and SEQ ID NO: 31 (QQYSGYPLT) as CDRL3;

(s) the 9F3 antibody (see U.S. Patent 8,475,795);

(t) an antibody that binds the same epitope as the 24F12 antibody;

(u) an antibody comprising SEQ ID NO: 32 (SYAMS) as CDRH1, SEQ ID NO: 33 (EIGSGGSYTYYPDTVTG) as CDRH2, SEQ ID NO: 34 (ETTAGYFDY) as CDRH3, SEQ ID NO: 35 (SASQGINNFLN) as CDRL1, SEQ ID NO: 36 (YTSSLHS) as CDRL2 and SEQ ID NO: 37 (QHFSKLPWT) as CDRL3;

(v) the 24F12 antibody (see U.S. Patent 8,475,795);

(w) an antibody that comprises a variable region light chain selected from the group consisting of:

(i) LK26HuVK as set forth in SEQ ID NO: 38:

Asp Ile Gln Leu Thr Gln Ser Pro Ser Ser Leu Ser Ala Ser Val Gly
Asp Arg Val Thr Ile Thr Cys Ser Val Ser Ser Ser Ile Ser Ser Asn
Asn Leu His Trp Tyr Gln Gln Lys Pro Gly Lys Ala Pro Lys Leu Leu
Ile Tyr Gly Thr Ser Asn Leu Ala Ser Gly Val Pro Ser Arg Phe Ser
Gly Ser Gly Ser Gly Thr Asp Phe Thr Phe Thr Ile Ser Ser Leu Gln
Pro Glu Asp Ile Ala Thr Tyr Tyr Cys Gln Gln Trp Ser Ser Tyr Pro
Tyr Met Tyr Thr Phe Gly Gln Gly Thr Lys Val Glu Ile Lys,

(ii) LK26HuVKY as set forth in SEQ ID NO: 39:

Asp Ile Gln Leu Thr Gln Ser Pro Ser Ser Leu Ser Ala Ser Val Gly
Asp Arg Val Thr Ile Thr Cys Ser Val Ser Ser Ser Ile Ser Ser Asn
Asn Leu His Trp Tyr Gln Gln Lys Pro Gly Lys Ala Pro Lys Leu Leu
Ile Tyr Gly Thr Ser Asn Leu Ala Ser Gly Val Pro Ser Arg Phe Ser
Gly Ser Gly Ser Gly Thr Asp Tyr Thr Phe Thr Ile Ser Ser Leu Gln
Pro Glu Asp Ile Ala Thr Tyr Tyr Cys Gln Gln Trp Ser Ser Tyr Pro
Tyr Met Tyr Thr Phe Gly Gln Gly Thr Lys Val Glu Ile Lys,

(iii) LK26HuVKPW as set forth in SEQ ID NO: 40:

Asp Ile Gln Leu Thr Gln Ser Pro Ser Ser Leu Ser Ala Ser Val Gly
Asp Arg Val Thr Ile Thr Cys Ser Val Ser Ser Ser Ile Ser Ser Asn
Asn Leu His Trp Tyr Gln Gln Lys Pro Gly Lys Ala Pro Lys Pro Trp
Ile Tyr Gly Thr Ser Asn Leu Ala Ser Gly Val Pro Ser Arg Phe Ser
Gly Ser Gly Ser Gly Thr Asp Phe Thr Phe Thr Ile Ser Ser Leu Gln
Pro Glu Asp Ile Ala Thr Tyr Tyr Cys Gln Gln Trp Ser Ser Tyr Pro
Tyr Met Tyr Thr Phe Gly Gln Gly Thr Lys Val Glu Ile Lys,

and

(iv) LK26HuVKPW,Y as set forth in SEQ ID NO: 41:

Asp Ile Gln Leu Thr Gln Ser Pro Ser Ser Leu Ser Ala Ser Val Gly
Asp Arg Val Thr Ile Thr Cys Ser Val Ser Ser Ser Ile Ser Ser Asn
Asn Leu His Trp Tyr Gln Gln Lys Pro Gly Lys Ala Pro Lys Pro Trp
Ile Tyr Gly Thr Ser Asn Leu Ala Ser Gly Val Pro Ser Arg Phe Ser
Gly Ser Gly Ser Gly Thr Asp Tyr Thr Phe Thr Ile Ser Ser Leu Gln
Pro Glu Asp Ile Ala Thr Tyr Tyr Cys Gln Gln Trp Ser Ser Tyr Pro
Tyr Met Tyr Thr Phe Gly Gln Gly Thr Lys Val Glu Ile Lys;

(x) an antibody that comprises a variable region heavy chain selected from the group consisting of:

(i) LK26HuVH as set forth in SEQ ID NO: 42:

Gln Val Gln Leu Gln Glu Ser Gly Pro Gly Leu Val Arg Pro Ser Gln
Thr Leu Ser Leu Thr Cys Thr Ala Ser Gly Phe Thr Phe Ser Gly Tyr
Gly Leu Ser Trp Val Arg Gln Pro Pro Gly Arg Gly Leu Glu Trp Val
Ala Met Ile Ser Ser Gly Gly Ser Tyr Thr Tyr Tyr Ala Asp Ser Val
Lys Gly Arg Val Thr Met Leu Arg Asp Thr Ser Lys Asn Gln Phe Ser
Leu Arg Leu Ser Ser Val Thr Ala Ala Asp Thr Ala Val Tyr Tyr Cys
Ala Arg His Gly Asp Asp Pro Ala Trp Phe Ala Tyr Trp Gly Gln Gly

Ser Leu Val Thr Val Ser Ser,

(ii) LK26HuVH FAIS,N as set forth in SEQ ID NO: 43:

Gln Val Gln Leu Gln Glu Ser Gly Pro Gly Leu Val Arg Pro Ser Gln
Thr Leu Ser Leu Thr Cys Thr Ala Ser Gly Phe Thr Phe Ser Gly Tyr
Gly Leu Ser Trp Val Arg Gln Pro Pro Gly Arg Gly Leu Glu Trp Val
Ala Met Ile Ser Ser Gly Gly Ser Tyr Thr Tyr Tyr Ala Asp Ser Val
Lys Gly Arg Phe Ala Ile Ser Arg Asp Asn Ser Lys Asn Gln Phe Ser
Leu Arg Leu Ser Ser Val Thr Ala Ala Asp Thr Ala Val Tyr Tyr Cys
Ala Arg His Gly Asp Asp Pro Ala Trp Phe Ala Tyr Trp Gly Gln Gly
Ser Leu Val Thr Val Ser Ser,

(iii) LK26HuVH SLF as set forth in SEQ ID NO: 44:

Gln Val Gln Leu Gln Glu Ser Gly Pro Gly Leu Val Arg Pro Ser Gln
Thr Leu Ser Leu Thr Cys Thr Ala Ser Gly Phe Thr Phe Ser Gly Tyr
Gly Leu Ser Trp Val Arg Gln Pro Pro Gly Arg Gly Leu Glu Trp Val
Ala Met Ile Ser Ser Gly Gly Ser Tyr Thr Tyr Tyr Ala Asp Ser Val
Lys Gly Arg Val Thr Met Leu Arg Asp Thr Ser Lys Asn Ser Leu Phe
Leu Arg Leu Ser Ser Val Thr Ala Ala Asp Thr Ala Val Tyr Tyr Cys
Ala Arg His Gly Asp Asp Pro Ala Trp Phe Ala Tyr Trp Gly Gln Gly
Thr Thr Val Thr Val Ser Ser,

(iv) LK26HuVH I,I as set forth in SEQ ID NO: 45:

Gln Val Gln Leu Gln Glu Ser Gly Pro Gly Leu Val Arg Pro Ser Gln
Thr Leu Ser Leu Thr Cys Thr Ala Ser Gly Phe Thr Phe Ser Gly Tyr
Gly Leu Ser Trp Val Arg Gln Pro Pro Gly Arg Gly Leu Glu Trp Val
Ala Met Ile Ser Ser Gly Gly Ser Tyr Thr Tyr Tyr Ala Asp Ser Val
Lys Gly Arg Val Thr Met Leu Arg Asp Thr Ser Lys Asn Gln Phe Ser
Leu Arg Leu Ser Ser Val Thr Ala Ala Asp Thr Ala Ile Tyr Ile Cys
Ala Arg His Gly Asp Asp Pro Ala Trp Phe Ala Tyr Trp Gly Gln Gly
Ser Leu Val Thr Val Ser Ser,

(v) LK26KOLHuVH as set forth in SEQ ID NO: 46:

Glu Val Gln Leu Val Glu Ser Gly Gly Gly Val Val Gln Pro Gly Arg
Ser Leu Arg Leu Ser Cys Ser Ala Ser Gly Phe Thr Phe Ser Gly Tyr
Gly Leu Ser Trp Val Arg Gln Ala Pro Gly Lys Gly Leu Glu Trp Val

Ala Met Ile Ser Ser Gly Gly Ser Tyr Thr Tyr Tyr Ala Asp Ser Val
Lys Gly Arg Phe Ala Ile Ser Arg Asp Asn Ala Lys Asn Thr Leu Phe
Leu Gln Met Asp Ser Leu Arg Pro Glu Asp Thr Gly Val Tyr Phe Cys
Ala Arg His Gly Asp Asp Pro Ala Trp Phe Ala Tyr Trp Gly Gln Gly
Thr Pro Val Thr Val Ser Ser;

(y) an antibody that comprises the heavy chain variable region LK26KOLHuVH (SEQ ID NO: 46) and the light chain variable region LK26HuVKPW,Y (SEQ ID NO: 41);
(z) an antibody that comprises the heavy chain variable region LK26HuVH SLF (SEQ ID NO: 44) and the light chain variable region LK26HuVKPW,Y (SEQ ID NO: 41);
(aa) an antibody that comprises the heavy chain variable region LK26HuVH FAIS,N (SEQ ID NO: 43) and the light chain variable region LK26HuVKPW,Y (SEQ ID NO: 41); and
(bb) the murine monoclonal LK26 antibody, the heavy and light chains of which are presented herein as SEQ ID NOs: 11 and 12, respectively:

SEQ ID NO: 11

Gln Val Xaa Leu Gln Xaa Ser Gly Gly Asp Leu Val Lys Pro Gly Gly
Ser Leu Lys Leu Ser Cys Ala Ala Ser Gly Phe Thr Phe Ser Gly Tyr
Gly Leu Ser Trp Val Arg Gln Thr Pro Asp Lys Arg Leu Glu Trp Val
Ala Met Ile Ser Ser Gly Gly Ser Tyr Thr Tyr Tyr Ala Asp Ser Val
Lys Gly Arg Phe Ala Ile Ser Arg Asp Asn Ala Lys Asn Ser Leu Phe
Leu Gln Met Ser Ser Leu Lys Ser Asp Asp Thr Ala Ile Tyr Ile Cys
Ala Arg His Gly Asp Asp Pro Ala Trp Phe Ala Tyr Trp Gly Gln Gly
Thr Leu Val Thr Val Ser Ala (wherein Xaa refers to any amino acid)

SEQ ID NO: 12

Asp Ile Glu Leu Thr Gln Ser Pro Ala Leu Met Ala Ala Ser Pro Gly
Glu Lys Val Thr Ile Thr Cys Ser Val Ser Ser Ser Ile Ser Ser Asn
Asn Leu His Trp Tyr Gln Gln Lys Ser Glu Thr Ser Pro Lys Pro Trp
Ile Tyr Gly Thr Ser Asn Leu Ala Ser Gly Val Pro Leu Arg Phe Arg
Gly Phe Gly Ser Gly Thr Ser Tyr Ser Leu Thr Ile Ser Ser Met Glu
Ala Glu Asp Ala Ala Thr Tyr Tyr Cys Gln Gln Trp Ser Ser Tyr Pro

Tyr Met Tyr Thr Phe Gly Gly Gly Thr Lys Leu Glu Ile Lys

as described in European Patent Application No. 86104170.5 (Rettig). In certain embodiments, the anti-FRA antibody includes (i) the heavy chain variable region LK26KOLHuVH (SEQ ID NO: 46) and the light chain variable region LK26HuVKPW,Y (SEQ ID NO: 41); the heavy chain variable region LK26HuVH SLF (SEQ ID NO: 44) and the light chain variable region LK26HuVKPW,Y (SEQ ID NO: 41); or the heavy chain variable region LK26HuVH FAIS,N (SEQ ID NO: 43) and the light chain variable region LK26HuVKPW,Y (SEQ ID NO: 41). Chinese hamster ovary (CHO) cells producing MORAb-003 have been deposited with the ATCC (10801 University Boulevard, Manassas, VA 20110) on April 24, 2006 and assigned accession no. PTA-7552.

Other useful antibodies that immunospecifically bind to folate receptor alpha comprise mature light and heavy chain variable regions having at least 90% and preferably at least 95% or 99% sequence identity to SEQ ID NO: 7 and SEQ ID NO: 8, respectively. Other useful antibodies that immunospecifically bind to folate receptor alpha or derivatives thereof can competitively inhibit binding of farletuzumab to folate receptor alpha, as determined, for example, by immunoassay. Competitive inhibition means that an antibody when present in at least a two-fold and preferably five-fold excess inhibits binding of farletuzumab to folate receptor alpha by at least 50%, more typically at least 60%, yet more typically at least 70%, and most typically at least 75%, at least 80%, at least 85%, at least 90%, or at least 95%.

The antibody that immunospecifically binds to folate receptor alpha may also be a derivative of an anti-folate receptor alpha antibody disclosed above. Typical modifications include, e.g., glycosylation, deglycosylation, acetylation, pegylation, phosphorylation, amidation, derivatization by known protecting/blocking groups, proteolytic cleavage, linkage to a cellular ligand or other protein, and the like. Additionally, the derivative may contain one or more non-classical amino acids.

In certain embodiments, the anti-FRA antibody is selected from the group consisting of a murine antibody, a human antibody, a humanized antibody, a bispecific antibody, a chimeric antibody, a Fab, Fab'2, ScFv, SMIP, affibody, avimer, versabody, nanobody, biobody, and a domain antibody. Alternatively, or in combination, the anti-FRA antibody is labeled, for example, with a label selected from the group consisting of a radio-label, a biotin-label, a chromophore-label, a fluorophore-label, or an enzyme-label.

In a further aspect, the level of folate receptor alpha (FRA) expression in a sample derived from the subject is assessed by a two-antibody sandwich assay. In some embodiments of the sandwich assay, the sample is contacted with (a) 9F3 antibody immobilized to a solid support and labeled 24F12 antibody, (b) 26B3 antibody immobilized to a solid support and labeled 19D4 antibody, and (c) 9F3 antibody immobilized to a solid support and labeled 26B3 antibody. For example, the sample may be urine, whole blood, serum, plasma, pleural effusion, sputum, bronchial washings, circulating cells, circulating tumor cells, cells that are not tissue associated (i.e., free cells), tissues (e.g., pleural tissue, surgically resected tumor tissue, biopsies, including fine needle aspiration), histological preparations, and the like.

In certain embodiments, the sample is treated with guanidine prior to determining the level of FRA expression in the sample. Alternatively or in combination, the sample is diluted prior to determining the level of FRA expression in the sample. Alternatively, or in combination, the sample is centrifuged, vortexed, or both, prior to determining the level of FRA expression in the sample.

In another variation, FRA expression level in known or suspected lung cancer can be detected *in vivo* by administering a labeled anti-FRA antibody or antigen-binding fragment thereof to a patient and detecting the antibody or fragment by *in vivo* imaging. Any of the antibodies described above may likewise be employed in the *in vivo* imaging analysis.

The level of FRA in a lung tissue sample can (but need not) be determined with respect to one or more standards. The standards can be historically or contemporaneously determined. The standard can be, for example, a FRA-expressing lung tissue sample known to be cancerous from a different subject, a FRA-expressing lung tissue sample known not to be cancerous from a different subject, a tissue from either the patient or other subject known not to express FRA, or a FRA-expressing lung cancer cell line.

The presence of detectable signal from binding of an anti-FRA antibody or fragment to FRA relative to a standard (if used) indicates the presence of FRA in the tissue sample, and the level of detectable binding provides an indication of the level of expression of FRA. In assays performed on tissue sections, the level of expression can be expressed as a percentage of the surface area of the sample showing detectable expression of FRA. Alternatively, or additionally, the level (intensity) of expression can be used as a measure of the total expression in the sample or of the cells expressing FRA in the sample. The intensity of expression can determined, for example, via digital imaging or manual microscopic assessment of tissue sections using methods as previously described (Potts, Drug Discov Today, 2009; 14(19-20):935-41; O'Shannessy et al., Oncotarget, 2012; 3(4):414-25; U.S. Patent 8,475,795; manufacturer's instructions, Catalog no. IPI4006K G10 (Biocare Medical; Concord, CA). The intensity of FRA expression may be used to determine a FRAMSCOR or HBSCOR as described herein. In particular, **the FRAMSCOR (M-score)** is calculated as a weighted average, assuming a 0, 1+, 2+, 3+ scoring system as follows (see Figure 2):

$x$ = % tumor stained at 1+
$y$ = % tumor stained at 2+
$\boldsymbol{z}$ = % tumor stained at 3+

Then

$$M = \frac{x + 2y + 3z}{6}$$

For purposes of example, if a patient's tumor has 20% FRA staining at +1, 10% FRA staining at +2 and 20% FRA staining at +3, then the M score = 16.6. **The HBSCOR (H-Score)** reports the mean optical density value for biomarker staining (in this case, staining of FRA) computed from all the cells in a target tissue compartment. It utilizes proprietary tissue recognition features to determine the tissue compartment via a linear score and a continuous extension of the H-score with no cell classification. The H-score is a standard scoring method that is commonly used by pathologists and those skilled in the art to score biomarker expression in tissues, which is basically the sum of the intensity scores at all intensity levels (1+, + 2 x 2+, + 3x 3+). The HBSCOR is derived from the sum of cell measurements (optical density) divided by the total number of cells. HBSCOR in turn reports the value for biomarker staining computed from all the cells in a target tissue compartment. This calculation is quantified using the following formula:

$$\text{HBSCOR} = \frac{\sum^{\text{Cells}} \text{Cell Measurement}}{\text{Number of Cells}}$$

Once the patient's FRA expression level is determined, it is compared to a reference FRA expression level. In a preferred embodiment, the patient's FRA expression level is presented in terms of a FRAMSCOR (*i.e.,* M-score) or HBSCOR (*i.e.,* H-score) for comparison to a reference FRA expression level. In a preferred embodiment, the reference FRA expression level is predetermined. For example, a reference data set may be established using samples from unrelated patients with low, moderate and high FRA expression levels. This data set represents a standard by which relative FRA expression levels are compared among patients and quantified using the manual and digital analysis FRAMSCOR and HBSCOR methods. In some embodiments, the reference FRA expression level is determined by comparison of a patient population afflicted with the FRA-expressing lung cancer that is administered the antibody according to the present invention that immunospecifically binds FRA to a patient population afflicted with the FRA-expressing lung cancer that is administered placebo. The patient populations afflicted with the FRA-expressing lung cancer may also have received standard-of-care chemotherapy. The FRA expression level for each patient in the respective populations afflicted with the FRA-expressing lung cancer is determined in accordance with the methods described above. Clinical outcomes (e.g., progression-free survival or overall survival) for the patient populations are monitored. Clinical outcomes for the patient populations relative to FRA expression levels are then compared as described in the examples provided below. The reference FRA expression level corresponds to the FRA expression level above which the patient population afflicted with the FRA-expressing lung cancer that is administered the antibody according to the present invention that immunospecifically binds FRA demonstrates a statistically significant improvement in at least one clinical outcome relative to the patient population afflicted with the FRA-expressing lung cancer that is administered placebo. A patient FRA expression level that equals or exceeds the reference FRA expression level is indicative that the patient will benefit from treatment with the antibody according to the present invention that immunospecifically binds FRA.

Methods of Treatment

Also provided herein is an antibody according to the present invention that immunospecifically binds FRA for use in methods of treating a patient having a folate receptor alpha (FRA)-expressing lung cancer. In some embodiments of the methods for treating a patient having FRA-expressing lung cancer, the cancer is NSCLC. In some embodiments, the NSCLC is adenocarcinoma. The disclosed methods for treating a FRA-expressing lung cancer in a patient include methods in which an antibody that immunospecifically binds folate receptor alpha (FRA) is administered to a patient whose FRA expression level equals or exceeds a reference FRA expression level.

In accordance with the methods of treating a patient with folate receptor alpha (FRA)-expressing lung cancer described herein, the patient's FRA expression level in a biological sample of the patient is quantified and compared to a reference FRA expression level as described above. If the patient's FRA expression level equals or exceeds the reference FRA expression level, then the patient is administered an antibody according to the present invention that immunospecifically binds FRA.

It is disclosed that in the methods of treatment described herein, the FRA targeting agent is vintafolide. In some embodiments, the FRA targeting agent is an antibody that immunospecifically binds FRA, such as but not limited to an antibody that immunospecifically binds to folate receptor alpha expressed on lung cancer cells; antigen-binding fragments of such an antibody; derivatives; and variants thereof. In a preferred embodiment, the antibody that immunospecifically binds to folate receptor alpha is an antibody selected from the group consisting of:

(a) an antibody comprising SEQ ID NO: 1 as CDRH1, SEQ ID NO:2 as CDRH2, SEQ ID NO:3 as CDRH3, SEQ ID NO:4 as CDRL1, SEQ ID NO:5 as CDRL2 and SEQ ID NO:6 as CDRL3;

(b) an antibody comprising a mature light chain variable region comprising the amino acid sequence of SEQ ID NO:7 and/or a mature heavy chain variable region comprising the amino acid sequence of SEQ ID NO: 8;

(c) farletuzumab;

(d) an antibody that specifically binds to folate receptor alpha comprising a mature light and heavy chain variable regions having at least 90% and preferably at least 95% or 99% sequence identity to SEQ ID NO: 7 and SEQ ID NO: 8, respectively;

(e) an antibody or derivative thereof can competitively inhibit binding of farletuzumab to folate receptor alpha, as determined, for example, by immunoassay.

A derivative of an antibody that immunospecifically binds FRA can also be used in the practice of the present methods. Typical modifications include, e.g., glycosylation, deglycosylation, acetylation, pegylation, phosphorylation, amidation, derivatization by known protecting/blocking groups, proteolytic cleavage, linkage to a cellular ligand or other protein, and the like. Additionally, the derivative may contain one or more non-classical amino acids. In some embodiments, that antibody that immunospecifically binds FRA is conjugated to a toxin, such as but not limited to a microtubule inhibitor, a DNA damaging agent (e.g., a radionuclide), a DNA repair inhibitor, or a signal transduction inhibitor. Linkers for antibody conjugation and methods for conjugating antibodies are known in the art. An exemplary antibody-drug conjugate that can be employed as a FRA-targeting agent in accordance with the methods described herein is IMGN853.

The present methods can be combined with other means of treatment such as surgery (e.g., debulking surgery), radiation, targeted therapy, chemotherapy, immunotherapy, use of growth factor inhibitors, or anti-angiogenesis factors. The antibody according to the present invention that immunospecifically binds FRA can be administered concurrently to a patient undergoing surgery, chemotherapy or radiation therapy treatments. Alternatively, a patient can undergo surgery, chemotherapy or radiation therapy prior or subsequent to administration of the antibody according to the present invention that immunospecifically binds FRA by at least an hour and up to several months, for example at least an hour, five hours, 12 hours, a day, a week, a month, or three months, prior or subsequent to administration of the antibody according to the present invention that immunospecifically binds FRA. For example, in some embodiments the antibody according to the present invention that immunospecifically binds FRA is for use in methods of treatment provided herein, wherein the methods further involve administration of a therapeutically effective amount of a platinum-containing compound, an antifolate, and/or a taxane to the subject in addition to the antibody according to the present invention that immunospecifically binds FRA. Exemplary platinum-containing compounds are cisplatin or carboplatin. Examples of taxanes for use in the methods of treatment include but are not limited to paclitaxel, docetaxel, and semi-synthetic, synthetic, and/or modified versions and formulations thereof, including but not limited to nab-paclitaxel (Abraxane®), cabazitaxel (Jevtana®), DJ-927 (Tesetaxel®), paclitaxel poliglumex (Opaxio®), XRP9881 (Larotaxel®), EndoTAG + paclitaxel (EndoTAG®-1), Polymeric-micellar paclitaxel (Genexol-PM®), DHA-paclitaxel (Taxoprexin®), BMS-184476. An exemplary antifolate is pemetrexed. The platinum-containing compound may be administered to the patient once every week, once every two weeks, once every three weeks, or once every four weeks. The taxane may be administered to the patient once every week, once every two weeks, once every three weeks, or once every four weeks. The antifolate may be administered to the patient once every week, once every two weeks, once every three weeks, or once every four weeks. In embodiments in which both a platinum-containing compound and a taxane or antifolate are administered to the patient as part of the treatment regimen, the taxane or antifolate may be administered before, after, or simultaneously with the platinum-containing compound.

In some embodiments, the antibody according to the present invention that immunospecifically binds FRA is for use in methods of treatment described herein, wherein the patient may have received surgical resection of the lung cancer, prior platinum-based therapy, prior taxane-based therapy, and/or prior platinum and taxane-based therapy for treatment of the cancer prior to quantifying the patient's FRA expression level. In some embodiments of the methods in which the patient received surgical resection of the cancer, prior platinum-based therapy, prior taxane-based therapy, and/or prior platinum and taxane-based therapy for treatment of the cancer prior to determining the patient's FRA expression level, the patient may have exhibited symptomatic progression, serologic progression, and/or radiologic progression of the cancer prior to the step of determining the patient's FRA expression level.

Administration of the therapeutic agents (including the antibody according to the present invention that immunospecifically binds FRA, the taxane, the antifolate, and/or the platinum-containing compound) in accordance with the methods of treatment described herein may be by any means known in the art.

Various delivery systems can be used to administer the therapeutic agents (including the antibody according to the present invention that immunospecifically binds FRA, the taxane, the antifolate, and/or the platinum-containing compound) including intradermal, intramuscular, intraperitoneal, intravenous, subcutaneous, intranasal, epidural, and oral routes. The agents can be administered, for example by infusion or bolus injection, by absorption through epithelial or mucocutaneous linings (e.g., oral mucosa, rectal and intestinal mucosa, and the like). Administration can be systemic or local.

The therapeutic agents can be administered by injection, by means of a catheter, by means of a suppository, or by means of an implant, the implant being of a porous, non-porous, or gelatinous material, including a membrane, such as a sialastic membrane, or a fiber. The therapeutic agents and pharmaceutical compositions thereof for use as described herein may be administered orally in any acceptable dosage form such as capsules, tablets, aqueous suspensions, solutions or the like.

Preferred methods of administration of the therapeutic agents include but are not limited to intravenous injection and intraperitoneal administration.

Alternatively, the therapeutic agents can be delivered in a controlled release system. For example, a pump can be used (see Langer, 1990, Science 249:1527-1533; Sefton, 1989, CRC Crit. Ref Biomed. Eng. 14:201; Buchwald et al., 1980, Surgery 88:507; Saudek et al., 1989, N. Engl. J. Med. 321:574). Alternatively, polymeric materials can be used (see Medical Applications of Controlled Release (Langer & Wise eds., CRC Press, Boca Raton, Fla., 1974); Controlled Drug Bioavailability, Drug Product Design and Performance (Smolen & Ball eds., Wiley, New York, 1984); Ranger & Peppas, 1983, Macromol. Sci. Rev. Macromol. Chem. 23:61. See also Levy et al., 1985, Science 228:190; During et al., 1989, Ann. Neurol. 25:351; Howard et al., 1989, J. Neurosurg. 71:105.) Other controlled release systems are discussed, for example, in Langer, supra.

The therapeutic agents can be administered as pharmaceutical compositions comprising a therapeutically or prophylactically effective amount of the therapeutic agent(s) and one or more pharmaceutically acceptable or compatible ingredients. For example, the pharmaceutical composition typically includes one or more pharmaceutical carriers (e.g., sterile liquids, such as water and oils, including those of petroleum, animal, vegetable or synthetic origin, such as peanut oil, soybean oil, mineral oil, sesame oil and the like). Water is a more typical carrier when the pharmaceutical composition is administered intravenously. Saline solutions (e.g., phosphate buffered saline) and aqueous dextrose and glycerol solutions can also be employed as liquid carriers, particularly for injectable solutions. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The composition, if desired, can also contain minor amounts of wetting or emulsifying agents, pH buffering agents (e.g., amino acids) and/or solubilizing or stabilizing agents (e.g., nonionic surfactants such as tween or sugars such as sucrose, trehalose or the like). The preferred formulation of farletuzumab contains farletuzumab, sodium phosphate, sodium chloride (NaCl), and polysorbate-80, pH 7.2. A preferred final formulation of farletuzumab contains 5 mg/mL farletuzumab, 10 mM sodium phosphate, 150 mM NaCl, and 0.01% polysorbate-80, pH 7.2.

The pharmaceutical compositions provided herein can take the form of solutions, suspensions, emulsion, tablets, pills, capsules, powders, sustained-release formulations and the like. Also included are solid form preparations which are intended to be converted, shortly before use, to liquid preparations. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the nucleic acid or protein, typically in purified form, together with a suitable amount of carrier so as to provide the form for proper administration to the patient. The formulations correspond to the mode of administration.

Typically, compositions for intravenous administration are solutions in sterile isotonic aqueous buffer. When necessary, the pharmaceutical can also include a solubilizing agent and a local anesthetic such as lignocaine to ease pain at the site of the injection. Generally, the ingredients are supplied either separately or mixed together in unit dosage form, for example, as a dry lyophilized powder or a concentrate in a hermetically sealed container such as an ampoule or sachette indicating the quantity of active agent. When the pharmaceutical composition is to be administered by infusion, it can be dispensed with an infusion bottle containing sterile pharmaceutical grade water or saline. When the pharmaceutical composition is administered by injection, an ampoule of sterile water for injection or saline can be provided so that the ingredients can be mixed prior to administration.

The amount of the therapeutic agent that is effective in the treatment of lung cancer can be determined by standard clinical techniques. In addition, *in vitro* assays may optionally be employed to help identify optimal dosage ranges. The precise dose to be employed in the formulation also depends on the route of administration, and the stage of the cancer, and should be decided according to the judgment of the practitioner and each patient's circumstances. Effective doses may be extrapolated from dose-response curves derived from *in vitro* or animal model test systems. A dose can be formulated in animal models to achieve a circulating plasma concentration range that includes the $IC_{50}$ (i.e., the concentration of the test compound that achieves a half-maximal inhibition of symptoms) as determined in cell culture.

For example, toxicity and therapeutic efficacy of the agents can be determined in cell cultures or experimental animals by standard pharmaceutical procedures for determining the $LD_{50}$ (the dose lethal to 50% of the population) and the $ED_{50}$ (the dose therapeutically effective in 50% of the population). The dose ratio between toxic and therapeutic effects is the therapeutic index and it can be expressed as the ratio $LD_{50}/ED_{50}$. Agents that exhibit large therapeutic indices are preferred. When an agent exhibits toxic side effects, a delivery system that targets the agent to the site of affected tissue can be used to minimize potential damage to non-folate receptor alpha-expressing cells and, thereby, reduce side effects.

In some embodiments, the subject can be administered a therapeutic agent described herein in a daily dose range of about 0.01 $\mu$g to about 500 mg per kg of the weight of the subject. Typically, the dosage of the therapeutic agent (e.g., the antibody according to the present invention that immunospecifically binds FRA, preferably farletuzumab) administered to a patient with a folate receptor alpha-expressing lung cancer is about 0.1 mg/kg to about 100 mg/kg of the subject's body weight. More typically, the dosage administered to a subject is about 1.25 mg/kg to about 12.5 mg/kg of the subject's body weight, or even more typically about 2.5 mg/kg to about 10.0 mg/kg of the subject's body weight. In some embodiments, the dosage of the antibody according to the present invention that immunospecifically binds FRA (preferably farletuzumab) administered to a subject having folate receptor alpha-expressing lung cancer is about 5.0 mg/kg to about 7.5 mg/kg of the subject's body weight. In some embodiments, the antibody according to the present invention that immunospecifically binds FRA is for use in methods of treatment described herein, wherein a loading dose of the antibody according to the present invention that immunospecifically binds FRA of about 7.5 mg/kg to about 12.5 mg/kg, preferably about 10 mg/kg, is administered to the subject. In some embodiments, the antibody according to the present invention that immunospecifically binds FRA is for use in methods of treatment described herein, wherein two loading doses of the antibody according to the present invention that immunospecifically binds FRA of about 7.5 mg/kg to about 12.5 mg/kg weekly, preferably about 10 mg/kg, is administered to the subject in the first two weeks of treatment. In some embodiments, the dosage of the taxane administered to a subject having folate receptor alpha-expressing lung cancer is about 50 $mg/m^2$ to about 250 $mg/m^2$ of the subject's body weight, preferably about 75 $mg/m^2$ to about 200 $mg/m^2$. In some embodiments, the dosage of carboplatin administered to a subject having folate receptor alpha-expressing lung cancer is about AUC 3, preferably about AUC 4, more preferably about AUC 5-6, and in some preferred embodiments, about AUC 6. In some embodiments, the dosage of cisplatin administered to a subject having folate receptor alpha-expressing lung cancer is about 50 $mg/m^2$ to about 250 $mg/m^2$ of the subject's body weight, preferably about 75 $mg/m^2$ to about 200 $mg/m^2$. In some embodiments, the dosage of antifolate administered to a subject having folate receptor alpha-expressing lung cancer is about 400 to about 600 $mg/m^2$. In a preferred embodiment, at least four to six cycles of chemotherapy in combination with administration of the antibody according to the present invention that immunospecifically binds FRA is administered to the patient.

For effective treatment, one skilled in the art may recommend a dosage schedule and dosage amount of the therapeutic agent(s) adequate for the subject being treated. It may be preferred that dosing occur one to four or more times daily, once per week, once per every two weeks, once per every three weeks, or once per every four weeks for as long as needed. Typically, the antibody according to the present invention that immunospecifically binds FRA is administered to the subject weekly.

The dosing may occur less frequently if the compositions are formulated in sustained delivery vehicles. The dosage schedule may also vary depending on the active drug concentration, which may depend on the needs of the subject.

Kits

Further disclosed herein are kits for predicting a likelihood of responsiveness to treatment with a FRA targeting agent in a patient having FRA-expressing lung cancer. In some embodiments, the kits contain an anti-FRA antibody, a vessel for containing the antibody when not in use, and instructions for using the anti-FRA antibody for determining the level of FRA expression of a subject. One or more additional containers may enclose elements, such as reagents or buffers, to be used in the marker assay(s). Such kits can also, or alternatively, contain a detection reagent that contains a reporter group suitable for direct or indirect detection of antibody binding.

Also disclosed herein are kits for treating a FRA-expressing lung cancer in a patient comprising a FRA targeting agent (e.g., vintafolide, an antibody that immunospecifically binds FRA such a farletuzumab, or an antibody-drug conjugate such as IMGN853), a vessel for containing the FRA targeting agent when not in use, and instructions for use of the FRA targeting agent. Farletuzumab is the preferred antibody that immunospecifically binds FRA in the kits. In some embodiments, the kits for treating a subject having FRA-expressing lung cancer also contain an anti-FRA antibody for use in quantifying the level of FRA expression in a biological sample of the patient. This latter anti-FRA antibody may be the same or different than the antibody that immunospecifically binds FRA that is administered therapeutically. In some embodiments, the kits also contain a vessel for containing the anti-FRA antibody when not in use and instructions for using the anti-FRA antibody for determining the expression level of FRA of a subject.

The kits for treating a subject having FRA-expressing lung cancer also may contain additional therapeutic agents (e.g., a platinum-containing compound, a taxane, and/or an antifolate) as described herein. Examples of platinum-containing compounds for inclusion in the kits include, but are not limited to, cisplatin and carboplatin. Examples of taxanes for inclusion in the kits include, but are not limited to, paclitaxel, docetaxel, and semi-synthetic, synthetic, and/or modified versions and formulations thereof, including but not limited to nab-paclitaxel (Abraxane®), cabazitaxel (Jevtana®), DJ-927 (Tesetaxel®), paclitaxel poliglumex (Opaxio®), XRP9881 (Larotaxel®), EndoTAG + paclitaxel (EndoTAG®-1), Polymeric-micellar paclitaxel (Genexol-PM®), DHA-paclitaxel (Taxoprexin®), BMS-184476. An example of an antifolate for inclusion in the kits is pemetrexed. The therapeutic agents can be in any of a variety of forms suitable for distribution in a kit. Forms of the therapeutic agents suitable for distribution in the kits can include a liquid, powder, tablet, suspension and the like formulation for providing the therapeutic agent. The kits can also include a pharmaceutically acceptable diluent (e.g., sterile water) for injection, reconstitution or dilution of the therapeutic agent(s). One or more additional containers may enclose elements, such as reagents or buffers, to be used in the marker assay(s). Such kits can also, or alternatively, contain a detection reagent that contains a reporter group suitable for direct or indirect detection of antibody binding.

Kits also typically contain a label or instructions for use in the methods described herein. The label or instruction refers to any written or recorded material that is attached to, or otherwise accompanies a kit at any time during its manufacture, transport, sale or use. It can be a notice in the form prescribed by a governmental agency regulating the manufacture, use or sale of pharmaceuticals or biological products, which notice reflects approval by the agency of manufacture, use or sale for human administration. The label or instruction can also encompass advertising leaflets and brochures, packaging materials, instructions, audio or videocassettes, computer discs, as well as writing imprinted directly on the pharmaceutical kits.

The following example is provided to further describe some of the embodiments disclosed herein. The example is intended to illustrate, not to limit, the disclosed embodiments.

EXAMPLE 1

Patients

Eligible subjects must have had newly diagnosed, unresectable, histologically or cytologically confirmed adenocarcinoma of the lung, with FRA expression (defined by 1+ or greater membranous staining) in at least 5% of tumor cells by immunohistochemistry, classified as Stage IV with at least one unidimensionally measurable lesion according to Response Evaluation Criteria in Solid Tumors (RECIST) version 1.1 using computed tomography (CT) or magnetic resonance imaging (MRI) (ClinicalTrials.gov identifier NCT01218516). As no data exists regarding the degree and frequency of FRA positivity that theoretically may be needed for a maximal anti-FRA antibody pharmacologic effect(s), patients whose tumors were at least 5% positive at a +1 intensity membrane FRA expression (using methods typically employed by those skilled in the art) were designated as having FRA-positive tumors and eligible for study inclusion. Subjects had received no prior chemotherapy, radiation therapy, or surgery with curative intent for their lung cancer.

Treatment

Subjects were randomized in a 1:1 ratio to receive the selected platinum doublet (carboplatin area under the curve (AUC) pharmacokinetic exposure level of 6 and paclitaxel 200 mg/m$^2$; carboplatin AUC 5-6 and pemetrexed 500 mg/m$^2$; or cisplatin 75 mg/m$^2$ and pemetrexed 500 mg/m$^2$) with either farletuzumab or placebo. Randomization was stratified by Eastern Cooperative Oncology Group (ECOG) performance status (0 or 1) and type of chemotherapy regimen chosen. All randomized subjects were treated with farletuzumab 7.5 mg/kg or placebo in combination with one of the 3 acceptable chemotherapy regimens, administered intravenously (IV) on Day 1 of a 21-day cycle except for the first cycle when farletuzumab or placebo monotherapy was administered on Day 8 as a loading dose. Beginning at Cycle 2, farletuzumab or placebo was administered on Day 1 of all additional cycles in combination with chemotherapy.

All protocol-acceptable platinum doublets were administered once every 3 weeks for at least 4, but no more than 6, cycles. Chemotherapy regimens were not changed once initiated. Chemotherapy doses could be reduced or delayed due to toxicity according to the country-specific, approved package inserts and based on the degree of toxicity experienced by the subject. In the event that chemotherapy was discontinued prior to the 4th cycle or was held for more than 6 weeks, the subject was discontinued from protocol treatment.

Those subjects who experienced clinical benefit from combination therapy could go on to receive farletuzumab or placebo on Day 1 of every 21-day cycle until documented radiographic progression or other protocol-approved measures of disease progression. Once disease progression had occurred, each subject was followed for survival status and use of additional systemic therapy for Stage IV adenocarcinoma of the lung. During this follow-up period, subjects were contacted monthly for the first 9 months, and every 2 months thereafter until death.

Assessments

Radiographic disease evaluations occurred every 2 cycles during combination therapy and every 3 cycles during monotherapy, read locally per independent review. CT scans (or MRIs) were also evaluated independently by central review using RECIST v.1.1. Subjects who discontinued study treatment prior to radiographic progression for any reason were followed radiographically every 9 weeks until documented radiographic progression or other protocol-approved measures of disease progression.

Safety evaluations were conducted throughout the study and included review of adverse events (AEs), physical examinations, laboratory evaluations, anti-drug antibodies (ADA), and electrocardiography (ECG).

PFS was defined as the time (in months) from the date of randomization to the date of the first observation of progression based on radiologic assessment by RECIST or definitive clinical disease progression as assessed by the investigators (e.g., new occurrence of positive fluid cytology), or the date of death, whatever the cause, in the absence of progressive disease.

To demonstrate that relative FRA expression levels in the tumor tissue or a threshold level of expression are important for improved farletuzumab-mediated clinical improvements in patients with non-small cell lung adenocarcinoma (NSCLC) in combination with standard-of-care chemotherapies (carboplatin + paclitaxel; carboplatin + pemetrexed; or cisplatin + pemetrexed), 5$\mu$m tissue section slides prepared from tumor lesions from chemo-naive, stage IV patients with NSCLC adenocarcinoma were analyzed for cytoplasmic or membrane FRA expression levels.

FRA protein levels were determined by immunohistochemical (IHC) analysis for this study using the 26B3 anti-FRA antibody (O'Shannessy et al., Oncotarget, 2012; 3(4):414-25; also contained within Catalog no. IPI4006K G10 (Biocare Medical; Concord, CA); see also U.S. Patent 8,475,795) following standard protocol instructions. To quantify FRA expression, levels were determined by one of two procedures and analyzed by trained pathologists in a blinded fashion. Either cytoplasmic or membrane FRA expression was determined via digital imaging or manual microscopic assessment of 26B3-stained tissue sections using methods as previously described (Potts, Drug Discov Today, 2009; 14(19-20):935-41; O'Shannessy et al., Oncotarget, 2012; 3(4):414-25; U.S. Patent 8,475,795; manufacturer's instructions, Catalog no. 1P14006K G10 (Biocare Medical; Concord, CA).

Digital analysis of FRA in 26B3-stained sectioned tissue slides was conducted using Image Analysis via the Cell Map and/or Stain Map algorithms according to Flagship Biosciences (Westminster, CO). Manual analysis of FRA expression was conducted by microscopic evaluation by two independent labs with trained pathologists skilled in the art.

To quantify membrane or cytoplasmic FRA expression levels derived by digital imaging or manual analysis, an algorithm was developed to quantify FRA staining as a percentage of tumor positivity and signal intensity. These values identified for use in the manual pathology scoring method are represented as FRA M Score (FRAMSCOR) for membrane staining. The values identified for use in the digital imaging analysis are represented as HBS Score (HBSCOR) for cytoplasmic staining. Previous methods used to accurately quantify biomarker expression levels determined that, in order to achieve highly accurate scoring, tumor sections must be well defined and IHC staining needs to be uniform. To assess the quality of tissue sections and staining obtained for patient inclusion into this study, a blinded pathologist skilled in the art independently evaluated the integrity of the FRA 26B3- stained NSCLC tumor tissue sections to compare FRA expression in pleural tissue using pleural tissue derived standards. After full assessment, the pathologist deemed 85 of the 130 tissue slides suitable for FRAMSCOR and HBSCOR pleural tissue analysis. While suitable for detecting the "presence" of FRA expression, detailed expression analysis of the remaining 45 slides was not possible due to: 1.) poor tissue preservation that precluded the ability to accurately analyze membrane expression; 2.) inability to accurately determine FRA expression in pleural tissue (a subset of slides only contained malignant cells within a pleural effusion background); and/or 3.) gross over staining that prohibited precise staining analysis in a linear range. Figure 1 shows the suitability of tissue integrity/morphology and staining versus examples of those not suitable for FRA expression quantification by FRAMSCOR or HBSCOR that were part of the original clinical set of the intent-to-treat population (ITT).

To determine FRA staining intensity in the 85 suitable pleural tumor sections, a pleural tissue reference data set was first established using samples from unrelated patients with low, moderate and high FRA expression levels. This data set represents a standard by which relative FRA expression levels are compared among patients and quantified using the manual and digital analysis FRAMSCOR and HBSCOR methods. Figure 2 provides an example of the reference data set used for scoring +1 (low expression), +2 (moderate expression) and +3 (high expression) as known by those skilled in the art of FRA in NSCLC adenocarcinoma samples. In addition, the percentage of tumor positivity is also factored into the algorithm for each method (1 to 100% positivity of the tumor surface area) (not shown) using the formulas below.

**The FRAMSCOR (M-score)** is calculated as a weighted average, assuming a 0, 1+, 2+, 3+ scoring system as follows (see Figure 2):

$x$ = % tumor stained at 1+
$y$ = % tumor stained at 2+

*z* = % tumor stained at 3+

Then

$$M = \frac{x+2y+3z}{6}$$

For purposes of example, if a patient's tumor has 20% 26B3-FRA staining at +1, 10% 26B3-FRA staining at +2 and 20% 26B3-FRA staining at +3, then the M score = 16.6

**The HBSCOR** reports the mean optical density value for biomarker staining (in this case 26B3-staining of FRA) computed from all the cells in a target tissue compartment. It utilizes proprietary tissue recognition features to determine the tissue compartment via a linear score and a continuous extension of the H-score with no cell classification. The H-score is a standard scoring method that is commonly used by pathologists and those skilled in the art to score biomarker expression in tissues, which is basically the sum of the intensity scores at all intensity levels (1+, + 2 x 2+, + 3x 3+). The HBSCOR is derived from the sum of cell measurements (optical density) divided by the total number of cells. HBSCOR in turn reports the value for biomarker staining computed from all the cells in a target tissue compartment. This calculation is quantified using the following formula:

$$\text{HBSCOR} = \frac{\sum^{\text{Cells}} \text{Cell Measurement}}{\text{Number of Cells}}$$

Evaluable slides containing patient-derived tissue stained for FRA using 26B3 antibody were analyzed and quantified using the FRAMSCOR and HBSCOR methods described above under blinded analysis. The data was first analyzed using an expression cut-point analysis. Folate Receptor Alpha (FRA) expression correlation to overall survival (OS) was conducted to determine if high or low FRA expression correlated with therapeutic response to farletuzumab treatment over placebo control treated patients. Figure 3 shows an example of this analysis whereby patient specimens were quantified for FRA expression via the HBSCOR method. As shown, a significant response in patient OS is observed, as clinically positive Hazard Ratios occur in farletuzumab-treated patients that express higher levels of FRA versus those with low expression. To determine the overall impact of FRA expression and farletuzumab treatment to patient clinical response as a function of progression free survival (PFS) or OS, Kaplan Meier analysis was conducted. As shown in Figure 4, patients selected for high FRA membrane expression using FRAMSCOR showed a statistically significant clinical improvement in OS (8.4 month improvement, HR 0.54; p = 0.0266) when treated with farletuzumab + SOC as compared to those treated with placebo + SOC only (p = 0.386). Improved PFS responses were also found in patients exhibiting high FRA membrane staining vs. low membrane staining (not shown). Similar effects were observed when patients with optimal FRA cytoplasmic expression using the HBSCOR method was used whereby patients treated with farletuzumab had a statistically significant clinical benefit in PFS and OS (Figure 5, Panel B) as compared to those treated with farletuzumab expressing less than optimal FRA levels (Figure 5, Panel A). These findings teach the use of identifying FRA expression in biopsies of lung cancer patients to determine those exhibiting a minimal threshold level using the FRAMSCOR or HBSCOR method to improve therapeutic benefit than just recruiting patients with tumor exhibiting any FRA expression as was done for the ITT population which failed to demonstrate statistical clinical benefit in patients treated with farletuzumab + SOC over those treated with placebo + SOC (PFS HR 0.91, p = 0.7045 and OS HR 0.91, p = 0.6525).

Multivariate analysis was conducted using standard factors that may affect NSCLC lung cancer patient clinical responses to SOC treatment such as smoking, age or ECOG status. No effects were seen in the multivariate analysis that impacts the statistically positive effect of farletuzumab on clinical response in patients with high levels of FRA.

These findings teach the use of employing good quality tissue biopsies containing pleural malignant tissue and stained in a manner than enables quantifiable analysis of FRA expression either localized to the membrane or in the cytoplasm for use in identifying patients who may respond to anti-folate receptor alpha antibody therapy. Subsequent analysis has found that patient samples derived from pleural effusions and fine needle aspirates can also be quantified to identify those exhibiting suitable FRA expression for anti-FRA therapeutic effects. In these cases a non-pleural based reference standard may be developed for comparison. Moreover, these results demonstrate that patients with tumors expressing higher levels of FRA in either membrane and/or cytoplasm as determined via FRAMSCOR or HBSCOR methods have a significant improvement in clinical outcomes including PFS and OS. These teachings should position the use of anti-FRA antibody therapies in NSCLC patients most likely to respond to anti-FRA antibody therapy(s). Levels of FRA are important for defining patients who will respond to anti-FRA antibody therapy in combination with SOC. Future trials and clinical utility should incorporate the treatment of patients exhibiting necessary threshold levels of FRA above the minimal cutpoint for efficacy. Those levels defined here include those with FRAMSCORs greater than or equal to 7 and HBSCORs greater than or equal to 0.25 (see Figure 3). On average this translates to NSCLC tumors with greater than 42% expression of FRA at a +1 intensity, 21% expression of FRA at a +2 intensity and/or tumors with greater than 14% expression of FRA at a +3 intensity.

## Claims

1. An antibody that immunospecifically binds folate receptor alpha (FRA) for use in

   (a) a method for treating a FRA-expressing lung cancer in a patient, said method comprising:

      determining the patient's FRA expression level in a biological sample of the patient;
      comparing the patient's FRA expression level to a reference FRA expression level; and
      administering a therapeutically effective amount of said antibody to said patient if said patient's FRA expression level equals or exceeds said reference FRA expression level; or

   (b) a method for treating a FRA-expressing lung cancer in a patient, wherein the patient's FRA expression level equals or exceeds a reference FRA expression level;

   wherein said reference FRA expression level corresponds to the FRA expression level above which a patient population afflicted with said FRA-expressing lung cancer that is administered said antibody that immunospecifically binds FRA demonstrated a statistically significant improvement in at least one clinical outcome relative to a patient population afflicted with said FRA-expressing lung cancer that is administered placebo;
   wherein said reference FRA expression level is:

   (i) 42% +1 or greater anti-FRA staining;
   (ii) 21% +2 or greater anti-FRA staining;
   (iii) 14% +3 or greater staining;
   (iv) a FRAMSCOR of 7; or
   (v) a HBSCOR of 0.25;

and
wherein the antibody that immunospecifically binds FRA is:

an antibody comprising SEQ ID NO:1 as CDRH1, SEQ ID NO:2 as CDRH2, SEQ ID NO:3 as CDRH3, SEQ ID NO:4 as CDRL1, SEQ ID NO:5 as CDRL2 and SEQ ID NO:6 as CDRL3;
an antibody comprising a mature light chain variable region comprising the amino acid sequence of SEQ ID NO:7 and/or a mature heavy chain variable region comprising the amino acid sequence of SEQ ID NO: 8;
farletuzumab;
an antibody that specifically binds to FRA comprising a mature light and heavy chain variable regions having at least 90% and preferably at least 95% or 99% sequence identity to SEQ ID NO: 7 and SEQ ID NO: 8, respectively; or

an antibody or derivative thereof can competitively inhibit binding of farletuzumab to FRA,
wherein the FRAMSCOR (M-score) is calculated as a weighted average, assuming a 0, 1+, 2+, 3+ scoring system as follows:

*x* = % tumor stained at 1+
*y* = % tumor stained at 2+
*z* = % tumor stained at 3+, then

$$M = \frac{x + 2y + 3z}{6}$$ ;

and wherein the HBSCOR is derived from the sum of cell measurements (optical density) divided by the total number of cells.

**2.** A method for predicting a likelihood of responsiveness to treatment with an antibody that immunospecifically binds folate receptor alpha (FRA) in a patient having a FRA-expressing lung cancer, said method comprising:

determining the patient's FRA expression level in a biological sample of the patient; and
comparing said patient's FRA expression level to a reference FRA expression level,
wherein said reference FRA expression level corresponds to the FRA expression level above which a patient population afflicted with said FRA-expressing lung cancer that is administered said antibody that immunospecifically binds FRA demonstrated a statistically significant improvement in at least one clinical outcome relative to a patient population afflicted with said FRA-expressing lung cancer that is administered placebo;
wherein said reference FRA expression level is:

(i) 42% +1 or greater anti-FRA staining;
(ii) 21% +2 or greater anti-FRA staining;
(iii) 14% +3 or greater staining;
(iv) a FRAMSCOR of 7; or
(v) a HBSCOR of 0.25;

wherein said patient is likely to respond to treatment with said antibody that immunospecifically binds FRA if said patient's FRA expression level equals or exceeds said reference FRA expression level, and
wherein the antibody that immunospecifically binds FRA is:

an antibody comprising SEQ ID NO:1 as CDRH1, SEQ ID NO:2 as CDRH2, SEQ ID NO:3 as CDRH3, SEQ ID NO:4 as CDRL1, SEQ ID NO:5 as CDRL2 and SEQ ID NO:6 as CDRL3;
an antibody comprising a mature light chain variable region comprising the amino acid sequence of SEQ ID NO:7 and/or a mature heavy chain variable region comprising the amino acid sequence of SEQ ID NO: 8;
farletuzumab;
an antibody that specifically binds to FRA comprising a mature light and heavy chain variable regions having at least 90% and preferably at least 95% or 99% sequence identity to SEQ ID NO: 7 and SEQ ID NO: 8, respectively; or
an antibody or derivative thereof can competitively inhibit binding of farletuzumab to FRA,
wherein the FRAMSCOR (M-score) is calculated as a weighted average, assuming a 0, 1+, 2+, 3+ scoring system as follows:

$x$ = % tumor stained at 1+
$y$ = % tumor stained at 2+
$z$ = % tumor stained at 3+, then

$$M = \frac{x + 2y + 3z}{6};$$

and wherein the HBSCOR is derived from the sum of cell measurements (optical density) divided by the total number of cells.

**3.** The antibody for use according to claim 1, or the method of claim 2 wherein said patient populations were further administered a chemotherapeutic agent; optionally, wherein said chemotherapeutic agent comprises a taxane, cisplatin, carboplatin, and/or pemetrexed.

**4.** The antibody for use according to claim 1 or claim 3, or the method of claim 2 or claim 3 wherein said FRA expression level is

(a) measured by protein quantification or RNA quantification;
(b) measured by immunohistochemical analysis; and/or
(c)

(i) cytoplasmic FRA expression; or
(ii) membranous FRA expression.

**5.** The antibody for use according to any one of claims 1, 3, and 4, wherein the method of treating further comprises administering a therapeutically effective amount of

(a) a chemotherapeutic agent to said patient; optionally, wherein said chemotherapeutic agent comprises a platinum-containing compound; for example, wherein said platinum-containing compound comprises cisplatin or carboplatin; and/or
(b) a taxane to said patient; for example, wherein said taxane is paclitaxel;

optionally, wherein (a) or (b) further comprise administering a therapeutically effective amount of pemetrexed to said patient.

**6.** The method of any one of claims 2-4, wherein the treatment further comprises a therapeutically effective amount of

(a) a chemotherapeutic agent; optionally, wherein said chemotherapeutic agent comprises a platinum-containing compound; for example, wherein said platinum-containing compound comprises cisplatin or carboplatin; and/or
(b) a taxane; for example, wherein said taxane is paclitaxel;

optionally, wherein (a) or (b) further comprise a therapeutically effective amount of pemetrexed.

**7.** The antibody for use according to any one of claims 1, 3, and 4, wherein the method for treating further comprises

(a) administering carboplatin and paclitaxel to said patient; optionally, wherein:

(i) carboplatin is administered to said patient to achieve an area under the curve (AUC) of about 6 or less; and/or
(ii) paclitaxel is administered to said patient at a dose of about 50 mg/m$^2$ to about 250 mg/m$^2$;

(b) administering carboplatin and pemetrexed to said patient; optionally, wherein:

(i) said carboplatin is administered to said patient to achieve an area under the curve of about 5-6 or less; and/or

(ii) said pemetrexed is administered to said patient at a dose of about 400 to about 600 $mg/m^2$; or

(c) administering cisplatin and pemetrexed to said patient optionally, wherein:

(i) cisplatin is administered to said patient at a dose of about 50 $mg/m^2$ to about 250 $mg/m^2$; and/or
(ii) pemetrexed is administered at a dose of about 400 $mg/m^2$ to about 600 $mg/m^2$.

**8.** The method of any one of claims 2-4, wherein the treatment further comprises

(a) a therapeutically effective amount of carboplatin and paclitaxel; optionally, wherein:

(i) the therapeutically effective amount of carboplatin achieves an area under the curve (AUC) of about 6 or less; and/or
(ii) the therapeutically effective amount of paclitaxel is a dose of about 50 $mg/m^2$ to about 250 $mg/m^2$;

(b) a therapeutically effective amount of carboplatin and pemetrexed; optionally, wherein:

(i) the therapeutically effective amount of carboplatin achieves an area under the curve of about 5-6 or less; and/or
(ii) the therapeutically effective amount of pemetrexed is a dose of about 400 to about 600 $mg/m^2$; or

(c) a therapeutically effective amount of cisplatin and pemetrexed optionally, wherein:

(i) the therapeutically effective amount of cisplatin is a dose of about 50 $mg/m^2$ to about 250 $mg/m^2$; and/or
(ii) the therapeutically effective amount of pemetrexed is a dose of about 400 $mg/m^2$ to about 600 $mg/m^2$.

**9.** The antibody for use according to any one of claims 1, 3-5, or 7, or the method of any one of claims 2-4, 6, or 8, wherein said FRA expression level is determined by immunoassay with at least one of the following antibodies:

(a) an antibody that binds the same epitope as farletuzumab;
(b) an antibody comprising SEQ ID NO:1 (GFTFSGYGLS) as CDRH1, SEQ ID NO:2 (MISSGGSYTYYADSVKG) as CDRH2, SEQ ID NO:3 (HGDDPAWFAY) as CDRH3, SEQ ID NO:4 (SVSSSISSNNLH) as CDRL1, SEQ ID NO:5 (GTSNLAS) as CDRL2 and SEQ ID NO:6 (QQWSSYPYMYT) as CDRL3;
(c) an antibody comprising a mature light chain variable region comprising the amino acid sequence of SEQ ID NO:7 and a mature heavy chain variable region comprising the amino acid SEQ ID NO: 8;
(d) farletuzumab;
(e) the 548908 antibody;
(f) an antibody that binds the same epitope as the 548908 antibody;
(g) the 6D398 antibody;
(h) an antibody that binds the same epitope as the 6D398 antibody;
(i) the BN3.2 antibody;
(j) an antibody that binds the same epitope as the BN3.2 antibody;
(k) an antibody that binds the same epitope as the 26B3 antibody;
(1) an antibody comprising SEQ ID NO: 14 (GYFMN) as CDRH1, SEQ ID NO: 15 (RIFPYNGDTFYNQKFKG) as CDRH2, SEQ ID NO: 16 (GTHYFDY) as CDRH3, SEQ ID NO: 17 (RTSENIFSYLA) as CDRL1, SEQ ID NO: 18 (NAKTLAE) as CDRL2 and SEQ ID NO: 19 (QHHYAFPWT) as CDRL3;
(m) the 26B3 antibody;
(n) an antibody that binds the same epitope as the 19D4 antibody;
(o) an antibody comprising SEQ ID NO: 20 (HPYMH) as CDRH1, SEQ ID NO: 21 (RIDPANGNTKYDPKFQG) as CDRH2, SEQ ID NO: 22 (EEVADYTMDY) as CDRH3, SEQ ID NO: 23 (RASESVDTYGNNFIH) as CDRL1, SEQ ID NO: 24 (LASNLES) as CDRL2 and SEQ ID NO: 25 (QQNNGDPWT) as CDRL3;
(p) the 19D4 antibody;
(q) an antibody that binds the same epitope as the 9F3 antibody;
(r) an antibody comprising SEQ ID NO: 26 (SGYYWN) as CDRH1, SEQ ID NO: 27 (YIKSDGSNNYNPSLKN) as CDRH2, SEQ ID NO: 28 (EWKAMDY) as CDRH3, SEQ ID NO: 29 (RASSTVSYSYLH) as CDRL1, SEQ ID NO: 30 (GTSNLAS) as CDRL2 and SEQ ID NO: 31 (QQYSGYPLT) as CDRL3;
(s) the 9F3 antibody;
(t) an antibody that binds the same epitope as the 24F12 antibody;

(u) an antibody comprising SEQ ID NO: 32 (SYAMS) as CDRH1, SEQ ID NO: 33 (EIGSGGSYTYYPDTVTG) as CDRH2, SEQ ID NO: 34 (ETTAGYFDY) as CDRH3, SEQ ID NO: 35 (SASQGINNFLN) as CDRL1, SEQ ID NO: 36 (YTSSLHS) as CDRL2 and SEQ ID NO: 37 (QHFSKLPWT) as CDRL3;
(v) the 24F12 antibody;
(w) an antibody that comprises a variable region light chain selected from the group consisting of:

(i) LK26HuVK;
(ii) LK26HuVKY;
(iii) LK26HuVKPW; and
(iv) LK26HuVKPW,Y;

(x) an antibody that comprises a variable region heavy chain selected from the group consisting of:

(i) LK26HuVH;
(ii) LK26HuVH FAIS,N;
(iii) LK26HuVH SLF;
(iv) LK26HuVH I,I;
(v) LK26KOLHuVH;

(y) an antibody that comprises the heavy chain variable region LK26KOLHuVH (SEQ ID NO: 46) and the light chain variable region LK26HuVKPW,Y (SEQ ID NO: 41);
(z) an antibody that comprises the heavy chain variable region LK26HuVH SLF (SEQ ID NO: 44) and the light chain variable region LK26HuVKPW,Y (SEQ ID NO: 41);
(aa) an antibody that comprises the heavy chain variable region LK26HuVH FAIS,N (SEQ ID NO: 43) and the light chain variable region LK26HuVKPW,Y (SEQ ID NO: 41); and
(bb) the murine monoclonal LK26 antibody.

**10.** The antibody for use according to any one of claims 1, 3-5, 7, or 9, or the method of any one of claims 2-4, 6, 8, or 9, wherein said patient's FRA expression level is assessed by digital imaging technology or manual pathology quantification.

**11.** The antibody for use according to claim 4, or the method of claim 4 wherein said patient's FRA expression level is assessed by FRAMSCOR or HBSCOR.

**12.** The antibody for use according to any one of claims 1, 3-5, 7, or 9-11, or the method of any one of claims 2-4, 6, or 8-11, wherein said biological sample

(a) is whole blood, serum, plasma, circulating cells, circulating tumor cells, free cells, tissue, pleural effusion, urine, saliva, sputum, or bronchial washing;
(b) comprises pleural tissue; and/or
(c) comprises pleural cells derived from effusion.

**13.** The antibody for use according to claim 1, or the method of claim 2 wherein said at least one clinical outcome is progression free survival and/or overall survival.

**14.** The antibody for use according to any one of claims 1, 3-5, 7, or 9-13, or the method of any one of claims 2-4, 6, or 8-13, wherein said FRA-expressing lung cancer is FRA-expressing non-small cell lung cancer (NSCLC); optionally, wherein said NSCLC is adenocarcinoma.

## Patentansprüche

**1.** Antikörper, der den Folatrezeptor Alpha (FRA) immunspezifisch bindet, zur Verwendung bei

(a) einem Verfahren zur Behandlung eines FRA-exprimierenden Lungenkrebses bei einem Patienten, wobei das Verfahren umfasst:

Bestimmen des FRA-Expressionsniveaus des Patienten in einer biologischen Probe des Patienten;

Vergleichen des FRA-Expressionsniveaus des Patienten mit einem Referenz-FRA-Expressionsniveau; und Verabreichen einer therapeutisch wirksamen Menge des Antikörpers an den Patienten, wenn das FRA-Expressionsniveau des Patienten dem Referenz-FRA-Expressionsniveau entspricht oder dieses überschreitet; oder

(b) einem Verfahren zur Behandlung eines FRA-exprimierenden Lungenkrebses bei einem Patienten, wobei das FRA-Expressionsniveau einem Referenz-FRA-Expressionsniveau entspricht oder dieses überschreitet;

wobei das Referenz-FRA-Expressionsniveau dem FRA-Expressionsniveau entspricht, oberhalb dessen eine an dem FRA-exprimierenden Lungenkrebs leidende Patientenpopulation, der der Antikörper verabreicht wurde, der FRA immunspezifisch bindet, in mindestens einem klinischen Ergebnis eine statistisch signifikante Verbesserung in Bezug auf eine an dem FRA-exprimierenden Lungenkrebs leidende Patientenpopulation, der Placebo verabreicht wurde, zeigte;
wobei das Referenz-FRA-Expressionsniveau ist:

(i) 42% +1 oder mehr Anti-FRA-Färbung;
(ii) 21% +2 oder mehr Anti-FRA-Färbung;
(iii) 14% +3 oder mehr Färbung;
(iv) ein FRAMSCOR von 7; oder
(v) ein HBSCOR von 0,25; und

wobei der Antikörper, der FRA immunspezifisch bindet, ist:

ein Antikörper, umfassend SEQ ID NO: 1 als CDRH1, SEQ ID NO: 2 als CDRH2, SEQ ID NO: 3 als CDRH3, SEQ ID NO: 4 als CDRL1, SEQ ID NO: 5 als CDRL2 und SEQ ID NO: 6 als CDRL3;
ein Antikörper, umfassend eine reife variable Leichtekettenregion, die die Aminosäuresequenz von SEQ ID NO: 7 umfasst und/oder eine reife variable Schwerekettenregion, die die Aminosäuresequenz von SEQ ID NO: 8 umfasst;
Farletuzumab;
ein Antikörper, der spezifisch an den FRA bindet, umfassend eine reife variable Leichtekettenregion und eine reife variable Schwerekettenregion mit mindestens 90% und vorzugsweise mindestens 95% oder 99% Sequenzidentität zu SEQ ID NO: 7 bzw. SEQ ID NO: 8; oder
ein Antikörper oder Derivat davon kann die Bindung von Farletuzumab an FRA kompetitiv hemmen,
wobei der FRAMSCOR (M-Score) als gewichteter Mittelwert berechnet wird, wobei ein Bewertungssystem von 0, 1+, 2+, 3+ wie folgt angenommen wird:

x = % Tumor, gefärbt bei 1+
y = % Tumor, gefärbt bei 2+
z = % Tumor, gefärbt bei 3+,
dann gilt

$$M = \frac{x + 2y + 3z}{6};$$

und wobei der HBSCOR von der Summe der Zellmessungen (optische Dichte), dividiert durch die Gesamtzahl an Zellen, abgeleitet ist.

**2.** Verfahren zur Vorhersage der Wahrscheinlichkeit eines Ansprechens auf die Behandlung mit einem Antikörper, der Folatrezeptor Alpha (FRA) immunspezifisch bindet, bei einem Patienten mit einem FRA-exprimierenden Lungenkrebs, wobei das Verfahren umfasst:

Bestimmen des FRA-Expressionsniveaus des Patienten in einer biologischen Probe des Patienten; und
Vergleichen des FRA-Expressionsniveaus des Patienten mit einem Referenz-FRA-Expressionsniveau,
wobei das Referenz-FRA-Expressionsniveau dem FRA-Expressionsniveau entspricht, oberhalb dessen eine an dem FRA-exprimierenden Lungenkrebs leidende Patientenpopulation, der der Antikörper verabreicht wurde, der FRA immunspezifisch bindet, in mindestens einem klinischen Ergebnis eine statistisch signifikante Verbesserung in Bezug auf eine an dem FRA-exprimierenden Lungenkrebs leidende Patientenpopulation, der Placebo verabreicht wurde, zeigte;
wobei das Referenz-FRA-Expressionsniveau ist:

(i) 42% +1 oder mehr Anti-FRA-Färbung;
(ii) 21% +2 oder mehr Anti-FRA-Färbung;
(iii) 14% +3 oder mehr Färbung;
(iv) ein FRAMSCOR von 7; oder
(v) ein HBSCOR von 0,25;

wobei der Patient wahrscheinlich auf die Behandlung mit dem Antikörper anspricht, der FRA immunspezifisch bindet, wenn das FRA-Expressionsniveau des Patienten dem Referenz-FRA-Expressionsniveau entspricht oder dieses überschreitet, und
wobei der Antikörper, der FRA immunspezifisch bindet, ist:

ein Antikörper, umfassend SEQ ID NO: 1 als CDRH1, SEQ ID NO: 2 als CDRH2, SEQ ID NO: 3 als CDRH3, SEQ ID NO: 4 als CDRL1, SEQ ID NO: 5 als CDRL2 und SEQ ID NO: 6 als CDRL3;
ein Antikörper, umfassend eine reife variable Leichtekettenregion, die die Aminosäuresequenz von SEQ ID NO: 7 umfasst und/oder eine reife variable Schwerekettenregion, die die Aminosäuresequenz von SEQ ID NO: 8 umfasst;
Farletuzumab;
ein Antikörper, der spezifisch an FRA bindet, umfassend eine reife variable Leichtekettenregion und eine reife variable Schwerekettenregion mit mindestens 90% und vorzugsweise mindestens 95% oder 99% Sequenzidentität zu SEQ ID NO: 7 bzw. SEQ ID NO: 8; oder
ein Antikörper oder Derivat davon kann die Bindung von Farletuzumab an FRA kompetitiv hemmen,
wobei der FRAMSCOR (M-Score) als gewichteter Mittelwert berechnet wird, wobei ein Bewertungssystem von 0, 1+, 2+, 3+ wie folgt angenommen wird:

x = % Tumor, gefärbt bei 1+
y = % Tumor, gefärbt bei 2+
z = % Tumor, gefärbt bei 3+,
dann gilt

$$M = \frac{x+2y+3z}{6};$$

und wobei der HBSCOR von der Summe der Zellmessungen (optische Dichte), dividiert durch die Gesamtzahl an Zellen, abgeleitet ist.

**3.** Antikörper zur Verwendung nach Anspruch 1 oder Verfahren nach Anspruch 2, wobei den Patientenpopulationen zudem ein Chemotherapeutikum verabreicht wurde, wobei das Chemotherapeutikum gegebenenfalls ein Taxan, Cisplatin, Carboplatin und/oder Pemetrexed umfasst.

**4.** Antikörper zur Verwendung nach Anspruch 1 oder Anspruch 3 oder Verfahren nach Anspruch 2 oder Anspruch 3, wobei das FRA-Expressionsniveau

(a) durch Proteinquantifizierung oder RNA-Quantifizierung gemessen wird;
(b) durch immunhistochemische Analyse gemessen wird; und/oder
(c)

(i) cytoplasmatische FRA-Expression ist; oder
(ii) membranöse FRA-Expression ist.

**5.** Antikörper zur Verwendung nach einem der Ansprüche 1, 3 und 4, wobei das Behandlungsverfahren zudem das Verabreichen einer therapeutisch wirksamen Menge

(a) eines Chemotherapeutikums an den Patienten, wobei das Chemotherapeutikum gegebenenfalls eine platinhaltige Verbindung umfasst, wobei beispielsweise die platinhaltige Verbindung Cisplatin oder Carboplatin umfasst; und/oder
(b) eines Taxans an den Patienten, wobei beispielsweise das Taxan Paclitaxel ist;

umfasst,
wobei gegebenenfalls (a) oder (b) zudem das Verabreichen einer therapeutisch wirksamen Menge von Pemetrexed an den Patienten umfassen.

**6.** Verfahren nach einem der Ansprüche 2 bis 4, wobei die Behandlung zudem eine therapeutisch wirksame Menge

(a) eines Chemotherapeutikums, wobei das Chemotherapeutikum gegebenenfalls eine platinhaltige Verbindung umfasst, wobei beispielsweise die platinhaltige Verbindung Cisplatin oder Carboplatin umfasst; und/oder
(b) eines Taxans, wobei beispielsweise das Taxan Paclitaxel ist;

umfasst,
wobei gegebenenfalls (a) oder (b) zudem eine therapeutisch wirksame Menge von Pemetrexed umfassen.

**7.** Antikörper zur Verwendung nach einem der Ansprüche 1, 3 und 4, wobei das Behandlungsverfahren zudem umfasst

(a) die Verabreichung von Carboplatin und Paclitaxel an den Patienten; wobei gegebenenfalls

(i) dem Patienten Carboplatin verabreicht wird, so dass eine Fläche unter der Kurve (AUC) von etwa 6 oder weniger erreicht wird; und/oder
(ii) dem Patienten Paclitaxel in einer Dosis von etwa 50 $mg/m^2$ bis etwa 250 $mg/m^2$ verabreicht wird:

(b) die Verabreichung von Carboplatin und Pemetrexed an den Patienten; wobei gegebenenfalls:

(i) dem Patienten Carboplatin verabreicht wird, so dass eine Fläche unter der Kurve von etwa 5 bis 6 oder weniger erreicht wird; und/oder
(ii) dem Patienten Pemetrexed in einer Dosis von etwa 400 bis etwa 600 $mg/m^2$ verabreicht wird; oder

(c) die Verabreichung von Cisplatin und Pemetrexed an den Patienten, wobei gegebenenfalls:

(i) dem Patienten Cisplatin in einer Dosis von etwa 50 $mg/m^2$ bis etwa 250 $mg/m^2$ verabreicht wird; und/oder
(ii) Pemetrexed in einer Dosis von etwa 400 $mg/m^2$ bis etwa 600 $mg/m^2$ verabreicht wird.

**8.** Verfahren nach einem der Ansprüche 2 bis 4, wobei die Behandlung zudem umfasst

(a) eine therapeutisch wirksame Menge von Carboplatin und Paclitaxel; wobei gegebenenfalls:

(i) die therapeutisch wirksame Menge von Carboplatin eine Fläche unter der Kurve (AUC) von etwa 6 oder weniger erreicht; und/oder
(ii) die therapeutisch wirksame Menge von Paclitaxel eine Dosis von etwa 50 $mg/m^2$ bis etwa 250 $mg/m^2$ ist;

(b) eine therapeutisch wirksame Menge von Carboplatin und Pemetrexed; wobei gegebenenfalls:

(i) die therapeutisch wirksame Menge von Carboplatin eine Fläche unter der Kurve von etwa 5 bis 6 oder weniger erreicht; und/oder
(ii) die therapeutisch wirksame Menge von Pemetrexed eine Dosis von etwa 400 bis etwa 600 $mg/m^2$ ist; oder

(c) eine therapeutisch wirksame Menge von Cisplatin und Pemetrexed, wobei gegebenenfalls:

(i) die therapeutisch wirksame Menge von Cisplatin eine Dosis von etwa 50 $mg/m^2$ bis etwa 250 $mg/m^2$ ist; und/oder
(ii) die therapeutisch wirksame Menge von Pemetrexed eine Dosis von etwa 400 $mg/m^2$ bis etwa 600 $mg/m^2$ ist.

**9.** Antikörper zur Verwendung nach einem der Ansprüche 1, 3 bis 5 oder 7 oder Verfahren nach einem der Ansprüche 2 bis 4, 6 oder 8, wobei das FRA-Expressionsniveau bestimmt wird durch Immunassay mit mindestens einem der folgenden Antikörper:

(a) einem Antikörper, der das gleiche Epitop wie Farletuzumab bindet;
(b) einem Antikörper, umfassend SEQ ID NO: 1 (GFTFSGYGLS) als CDRH1, SEQ ID NO: 2 (MISSGGSYTYYADSVKG) als CDRH2, SEQ ID NO: 3 (HGDDPAWFAY) als CDRH3, SEQ ID NO: 4 (SVSSSISSNNLH) als CDRL1, SEQ ID NO: 5 (GTSNLAS) als CDRL2 und SEQ ID NO: 6 (QQWSSYPYMYT) als CDRL3;
(c) einem Antikörper, umfassend eine reife variable Leichtekettenregion, die die Aminosäuresequenz von SEQ ID NO: 7 umfasst, und eine reife variable Schwerekettenregion, die die Aminosäure SEQ ID NO: 8 umfasst;
(d) Farletuzumab;
(e) dem 548908-Antikörper;
(f) einem Antikörper, der das gleiche Epitop wie der 548908-Antikörper bindet;
(g) dem 6D398-Antikörper;
(h) einem Antikörper, der das gleiche Epitop wie der 6D398-Antikörper bindet;
(i) dem BN3.2-Antikörper;
(j) einem Antikörper, der das gleiche Epitop wie der BN3.2-Antikörper bindet;
(k) einem Antikörper, der das gleiche Epitop wie der 26B3-Antikörper bindet;
(l) einem Antikörper, umfassend SEQ ID NO: 14 (GYFMN) als CDRH1, SEQ ID NO: 15 (RIFPYNGDTFYNQKFKG) als CDRH2, SEQ ID NO: 16 (GTHYFDY) als CDRH3, SEQ ID NO: 17 (RTSENIFSYLA) als CDRL1, SEQ ID NO: 18 (NAKTLAE) als CDRL2 und SEQ ID NO: 19 (QHHYAFPWT) als CDRL3;
(m) dem 26B3-Antikörper;
(n) einem Antikörper, der das gleiche Epitop wie der 19D4-Antikörper bindet;
(o) einem Antikörper, umfassend SEQ ID NO: 20 (HPYMH) als CDRH1, SEQ ID NO: 21 (RIDPANGNTKYDPKFQG) als CDRH2, SEQ ID NO: 22 (EEVADYTMDY) als CDRH3, SEQ ID NO: 23 (RASESVDTYGNNFIH) als CDRL1, SEQ ID NO: 24 (LASNLES) als CDRL2 und SEQ ID NO: 25 (QQNNGDPWT) als CDRL3;
(p) dem 19D4-Antikörper;
(q) einem Antikörper, der das gleiche Epitop wie der 9F3-Antikörper bindet;
(r) einem Antikörper, umfassend SEQ ID NO: 26 (SGYYWN) als CDRH1, SEQ ID NO: 27 (YIKSDGSNNYNPSLKN) als CDRH2, SEQ ID NO: 28 (EWKAMDY) als CDRH3, SEQ ID NO: 29 (RASSTVSYSYLH) als CDRL1, SEQ ID NO: 30 (GTSNLAS) als CDRL2 und SEQ ID NO: 31 (QQYSGYPLT) als CDRL3;
(s) dem 9F3-Antikörper;
(t) einem Antikörper, der das gleiche Epitop wie der 24F12-Antikörper bindet;
(u) einem Antikörper, umfassend SEQ ID NO: 32 (SYAMS) als CDRH1, SEQ ID NO: 33 (EIGSGGSYTYYPDTVTG) als CDRH2, SEQ ID NO: 34 (ETTAGYFDY) als CDRH3, SEQ ID NO: 35 (SASQGINNFLN) als CDRL1, SEQ ID NO: 36 (YTSSLHS) als CDRL2 und SEQ ID NO: 37 (QHFSKLPWT) als CDRL3;
(v) dem 24F12-Antikörper;
(w) einem Antikörper, der eine variable Leichtekettenregion umfasst, ausgewählt aus der Gruppe bestehend aus:

(i) LK26HuVK;
(ii) LK26HuVKY;
(iii) LK26HuVKPW; und
(iv) LK26HuVKPW,Y;

(x) einem Antikörper, der eine variable Schwerekettenregion umfasst, ausgewählt aus der Gruppe bestehend aus:

(i) LK26HuVH;
(ii) LK26HuVH FAIS,N;
(iii) LK26HuVH SLF;
(iv) LK26HuVH I,I;
(v) LK26KOLHuVH;

(y) einem Antikörper, der die variable Schwerekettenregion LK26KOLHuVH (SEQ ID NO: 46) und die variable Leichtekettenregion LK26HuVKPW,Y (SEQ ID NO: 41) umfasst;
(z) einem Antikörper, der die variable Schwerekettenregion LK26HuVH SLF (SEQ ID NO: 44) und die variable Leichtekettenregion LK26HuVKPW,Y (SEQ ID NO: 41) umfasst;
(aa) einem Antikörper, der die variable Schwerekettenregion LK26HuVH FAIS,N (SEQ ID NO: 43) und die variable Leichtekettenregion LK26HuVKPW,Y (SEQ ID NO: 41) umfasst; und
(bb) dem monoklonalen Maus-LK26-Antikörper.

**10.** Antikörper zur Verwendung nach einem der Ansprüche 1, 3 bis 5, 7 oder 9 oder Verfahren nach einem der Ansprüche 2 bis 4, 6, 8 oder 9, wobei das FRA-Expressionsniveau des Patienten durch digitale Bildgebungstechnologie oder manuelle Pathologie-Quantifizierung bewertet wird.

**11.** Antikörper zur Verwendung nach Anspruch 4 oder Verfahren nach Anspruch 4, wobei das FRA-Expressionsniveau des Patienten durch FRAMSCOR oder HBSCOR bewertet wird.

**12.** Antikörper zur Verwendung nach einem der Ansprüche 1, 3 bis 5, 7 oder 9 bis 11 oder Verfahren nach einem der Ansprüche 2 bis 4, 6, oder 8 bis 11, wobei die biologische Probe

(a) Vollblut, Serum, Plasma, zirkulierende Zellen, zirkulierende Tumorzellen, freie Zellen, Gewebe, Pleuraerguss, Urin, Speichel, Sputum oder Bronchialwäsche ist;
(b) Pleuragewebe umfasst; und/oder
(c) Pleurazellen umfasst, die aus einem Erguss stammen.

**13.** Antikörper zur Verwendung nach Anspruch 1 oder Verfahren nach Anspruch 2, wobei das mindestens eine klinische Ergebnis progressionsfreies Überleben und/oder Gesamtüberleben ist.

**14.** Antikörper zur Verwendung nach einem der Ansprüche 1, 3 bis 5, 7 oder 9 bis 13 oder Verfahren nach einem der Ansprüche 2 bis 4, 6 oder 8 bis 13, wobei der FRAexprimierende Lungenkrebs FRA-exprimierender nichtkleinzelliger Lungenkrebs (NSCLC) ist, wobei der NSCLC gegebenenfalls ein Adenokarzinom ist.

## Revendications

**1.** Anticorps qui se lie de façon immunospécifique au récepteur de folate alpha (FRA) pour utilisation dans

(a) un procédé de traitement d'un cancer du poumon exprimant FRA chez un patient, ledit procédé comprenant :

la détermination du taux d'expression de FRA du patient dans un échantillon biologique du patient ;
la comparaison du taux d'expression de FRA du patient à un taux d'expression de FRA de référence ;
et
l'administration d'une quantité thérapeutiquement efficace dudit anticorps audit patient si ledit taux d'expression de FRA du patient est égal ou supérieur audit taux d'expression de FRA de référence ; ou

(b) un procédé de traitement d'un cancer du poumon exprimant FRA chez un patient, dans lequel le taux d'expression de FRA du patient est égal ou supérieur à un taux d'expression de FRA de référence ;

où ledit taux d'expression de FRA de référence correspond au taux d'expression de FRA au-dessus duquel une population de patients atteints dudit cancer du poumon exprimant FRA auxquels est administré ledit anticorps qui se lie de façon immunospécifique à FRA présente une amélioration statistiquement significative d'au moins un résultat clinique par rapport à une population de patients atteints dudit cancer du poumon exprimant FRA auxquels est administré un placebo ;
où ledit taux d'expression de FRA de référence est :

(i) 42 % +1 ou plus de coloration anti-FRA ;
(ii) 21 % +2 ou plus de coloration anti-FRA ;
(iii) 14 % +3 ou plus de coloration ;
(iv) un FRAMSCOR de 7 ; ou
(v) un HBSCOR de 0,25 ;

et
où l'anticorps qui se lie de façon immunospécifique à FRA est :

un anticorps comprenant SEQ ID NO : 1 en tant que CDRH1, SEQ ID NO : 2 en tant que CDRH2, SEQ ID NO : 3 en tant que CDRH3, SEQ ID NO : 4 en tant que CDRL1, SEQ ID NO : 5 en tant que CDRL2 et SEQ ID NO : 6 en tant que CDRL3 ;
un anticorps comprenant une région variable de chaîne légère mature comprenant la séquence d'acides aminés de SEQ ID NO : 7 et/ou une région variable de chaîne lourde mature comprenant la séquence d'acides aminés de SEQ ID NO : 8 ;
le farlétuzumab ;
un anticorps qui se lie spécifiquement à FRA comprenant des régions variables de chaîne lourde et légère matures ayant au moins 90 % et, de préférence, au moins 95 % ou 99 % d'identité de séquence avec SEQ ID NO : 7 et SEQ ID NO : 8, respectivement ; ou
un anticorps ou un dérivé de celui-ci qui peut inhiber de façon compétitive la liaison du farlétuzumab à FRA,
où le FRAMSCOR (score M) est calculé sous la forme d'une moyenne pondérée, en supposant un système de notation 0, 1+, 2+, 3+ comme suit :

x = % de tumeur colorée à 1+
y = % de tumeur colorée à 2+
z = % de tumeur colorée à 3+, alors

$$M = \frac{x + 2y + 3z}{6}$$

et où le HBSCOR est dérivé de la somme des mesures de cellule (densité optique) divisée par le nombre total de cellules.

**2.** Procédé de prédiction d'une probabilité de réactivité au traitement avec un anticorps qui se lie de façon immunospécifique au récepteur de folate alpha (FRA) chez un patient ayant un cancer du poumon exprimant FRA, ledit procédé comprenant :

la détermination du taux d'expression de FRA du patient dans un échantillon biologique du patient ; et
la comparaison dudit taux d'expression de FRA du patient à un taux d'expression de FRA de référence,
où ledit taux d'expression de FRA de référence correspond au taux d'expression de FRA au-dessus duquel une population de patients atteints dudit cancer du poumon exprimant FRA auxquels est administré ledit anticorps qui se lie de façon immunospécifique à FRA présente une amélioration statistiquement significative d'au moins un résultat clinique par rapport à une population de patients atteints dudit cancer du poumon exprimant FRA auxquels est administré un placebo ;
où ledit taux d'expression de FRA de référence est :

(i) 42 % +1 ou plus de coloration anti-FRA ;
(ii) 21 % +2 ou plus de coloration anti-FRA ;
(iii) 14 % +3 ou plus de coloration ;
(iv) un FRAMSCOR de 7 ; ou
(v) un HBSCOR de 0,25 ;

où ledit patient est susceptible de répondre au traitement avec ledit anticorps qui se lie de façon immunospécifique à FRA si ledit taux d'expression de FRA du patient est égal ou supérieur audit taux d'expression de FRA de référence, et
où l'anticorps qui se lie de façon immunospécifique à FRA est :

un anticorps comprenant SEQ ID NO : 1 en tant que CDRH1, SEQ ID NO : 2 en tant que CDRH2, SEQ ID NO : 3 en tant que CDRH3, SEQ ID NO : 4 en tant que CDRL1, SEQ ID NO : 5 en tant que CDRL2 et SEQ ID NO : 6 en tant que CDRL3 ;
un anticorps comprenant une région variable de chaîne légère mature comprenant la séquence d'acides aminés de SEQ ID NO : 7 et/ou une région variable de chaîne lourde mature comprenant la séquence d'acides aminés de SEQ ID NO : 8 ;
le farlétuzumab ;
un anticorps qui se lie spécifiquement à FRA comprenant des régions variables de chaîne lourde et légère matures ayant au moins 90 % et, de préférence, au moins 95 % ou 99 % d'identité de séquence avec SEQ ID NO : 7 et SEQ ID NO : 8, respectivement ; ou
un anticorps ou un dérivé de celui-ci qui peut inhiber de façon compétitive la liaison du farlétuzumab à FRA,
où le FRAMSCOR (score M) est calculé sous la forme d'une moyenne pondérée, en supposant un système de notation 0, 1+, 2+, 3+ comme suit :

x = % de tumeur colorée à 1+
y = % de tumeur colorée à 2+
z = % de tumeur colorée à 3+, alors

$$M = \frac{x + 2y + 3z}{6}$$

et où le HBSCOR est dérivé de la somme des mesures de cellule (densité optique) divisée par le nombre total de cellules.

**3.** Anticorps pour utilisation selon la revendication 1, ou procédé selon la revendication 2 où lesdites populations de patients reçoivent en outre un agent chimiothérapeutique ; facultativement, où ledit agent chimiothérapeutique comprend un taxane, le cisplatine, le carboplatine, et/ou le pémétrexed.

**4.** Anticorps pour utilisation selon la revendication 1 ou la revendication 3, ou procédé selon la revendication 2 ou la revendication 3 où ledit taux d'expression de FRA est

(a) mesuré par quantification de protéine ou quantification d'ARN ;
(b) mesuré par analyse immunohistochimique ; et/ou
(c)

(i) l'expression cytoplasmique de FRA ; ou
(ii) l'expression membranaire de FRA.

**5.** Anticorps pour utilisation selon l'une quelconque des revendications 1, 3 et 4, où le procédé de traitement comprend en outre l'administration d'une quantité thérapeutiquement efficace de

(a) un agent chimiothérapeutique audit patient ; facultativement, où ledit agent chimiothérapeutique comprend un composé contenant du platine ; par exemple, où ledit composé contenant du platine comprend le cisplatine ou le carboplatine ; et/ou
(b) un taxane audit patient ; par exemple, où ledit taxane est le paclitaxel ;

facultativement, où (a) ou (b) comprennent en outre l'administration d'une quantité thérapeutiquement efficace de pémétrexed audit patient.

**6.** Procédé selon l'une quelconque des revendications 2 à 4, dans lequel le traitement comprend en outre une quantité thérapeutiquement efficace de

(a) un agent chimiothérapeutique ; facultativement, dans lequel ledit agent chimiothérapeutique comprend un composé contenant du platine ; par exemple, dans lequel ledit composé contenant du platine comprend le cisplatine ou le carboplatine ; et/ou
(b) un taxane ; par exemple, dans lequel ledit taxane est le paclitaxel ;

facultativement, dans lequel (a) ou (b) comprennent en outre une quantité thérapeutiquement efficace de pémétrexed.

**7.** Anticorps pour utilisation selon l'une quelconque des revendications 1, 3 et 4, où le procédé de traitement comprend en outre

(a) l'administration de carboplatine et de paclitaxel audit patient ; facultativement, où :

(i) le carboplatine est administré audit patient pour obtenir une aire sous la courbe (ASC) d'environ 6 ou moins ; et/ou
(ii) le paclitaxel est administré audit patient à une dose d'environ 50 $mg/m^2$ à environ 250 $mg/m^2$ ;

(b) l'administration de carboplatine et de pémétrexed audit patient ; facultativement, où :

(i) ledit carboplatine est administré audit patient pour obtenir une aire sous la courbe d'environ 5 à 6 ou moins ; et/ou
(ii) ledit pémétrexed est administré audit patient à une dose d'environ 400 à environ 600 mg/$m^2$ ; ou

(c) l'administration de cisplatine et de pémétrexed audit patient facultativement, où :

(i) le cisplatine est administré audit patient à une dose d'environ 50 mg/$m^2$ à environ 250 mg/$m^2$ ; et/ou
(ii) le pémétrexed est administré à une dose d'environ 400 mg/$m^2$ à environ 600 mg/$m^2$.

**8.** Procédé selon l'une quelconque des revendications 2 à 4, dans lequel le traitement comprend en outre

(a) une quantité thérapeutiquement efficace de carboplatine et de paclitaxel ; facultativement, dans lequel :

(i) la quantité thérapeutiquement efficace de carboplatine produit une aire sous la courbe (ASC) d'environ 6 ou moins ; et/ou
(ii) la quantité thérapeutiquement efficace de paclitaxel est une dose d'environ 50 mg/$m^2$ à environ 250 mg/$m^2$ ;

(b) une quantité thérapeutiquement efficace de carboplatine et de pémétrexed ; facultativement, dans lequel :

(i) la quantité thérapeutiquement efficace de carboplatine produit une aire sous la courbe d'environ 5 à 6 ou moins ; et/ou
(ii) la quantité thérapeutiquement efficace de pémétrexed est une dose d'environ 400 à environ 600 mg/$m^2$ ; ou

(c) une quantité thérapeutiquement efficace de cisplatine et de pémétrexed facultativement, dans lequel :

(i) la quantité thérapeutiquement efficace de cisplatine est une dose d'environ 50 mg/$m^2$ à environ 250 mg/$m^2$ ; et/ou
(ii) la quantité thérapeutiquement efficace de pémétrexed est une dose d'environ 400 mg/$m^2$ à environ 600 mg/$m^2$.

**9.** Anticorps pour utilisation selon l'une quelconque des revendications 1, 3 à 5, ou 7, ou procédé selon l'une quelconque des revendications 2 à 4, 6, ou 8, où ledit taux d'expression de FRA est déterminé par immunoessai avec au moins un des anticorps suivants :

(a) un anticorps qui se lie au même épitope que le farlétuzumab ;
(b) un anticorps comprenant SEQ ID NO : 1 (GFTFSGYGLS) en tant que CDRH1, SEQ ID NO : 2 (MISSGGSYTYYADSVKG) en tant que CDRH2, SEQ ID NO : 3 (HGDDPAWFAY) en tant que CDRH3, SEQ ID NO : 4 (SVSSSISSNNLH) en tant que CDRL1, SEQ ID NO : 5 (GTSNLAS) en tant que CDRL2 et SEQ ID NO : 6 (QQWSSYPYMYT) en tant que CDRL3 ;
(c) un anticorps comprenant une région variable de chaîne légère mature comprenant la séquence d'acides aminés de SEQ ID NO : 7 et une région variable de chaîne lourde mature comprenant la séquence d'acides aminés SEQ ID NO : 8 ;
(d) le farlétuzumab ;
(e) l'anticorps 548908 ;
(f) un anticorps qui se lie au même épitope que l'anticorps 548908 ;
(g) l'anticorps 6D398 ;
(h) un anticorps qui se lie au même épitope que l'anticorps 6D398 ;
(i) l'anticorps BN3.2 ;
(j) un anticorps qui se lie au même épitope que l'anticorps BN3.2 ;
(k) un anticorps qui se lie au même épitope que l'anticorps 26B3 ;
(l) un anticorps comprenant SEQ ID NO : 14 (GYFMN) en tant que CDRH1, SEQ ID NO : 15 (RIFPYNGDTFYNQKFKG) en tant que CDRH2, SEQ ID NO : 16 (GTHYFDY) en tant que CDRH3, SEQ ID NO : 17 (RTSENIFSYLA) en tant que CDRL1 , SEQ ID NO : 18 (NAKTLAE) en tant que CDRL2 et SEQ ID NO : 19 (QHHYAFPWT) en tant que CDRL3 ;
(m) l'anticorps 26B3 ;
(n) un anticorps qui se lie au même épitope que l'anticorps 19D4 ;

(o) un anticorps comprenant SEQ ID NO : 20 (HPYMH) en tant que CDRH1, SEQ ID NO : 21 (RIDPANGNTKYDPKFQG) en tant que CDRH2, SEQ ID NO : 22 (EEVADYTMDY) en tant que CDRH3, SEQ ID NO : 23 (RASESVDTYGNNFIH) en tant que CDRL1, SEQ ID NO : 24 (LASNLES) en tant que CDRL2 et SEQ ID NO : 25 (QQNNGDPWT) en tant que CDRL3 ;
(p) l'anticorps 19D4 ;
(q) un anticorps qui se lie au même épitope que l'anticorps 9F3 ;
(r) un anticorps comprenant SEQ ID NO : 26 (SGYYWN) en tant que CDRH1, SEQ ID NO : 27 (YIKSDGSNNYNPSLKN) en tant que CDRH2, SEQ ID NO : 28 (EWKAMDY) en tant que CDRH3, SEQ ID NO : 29 (RASSTVSYSYLH) en tant que CDRL1, SEQ ID NO : 30 (GTSNLAS) en tant que CDRL2 et SEQ ID NO : 31 (QQYSGYPLT) en tant que CDRL3 ;
(s) l'anticorps 9F3 ;
(t) un anticorps qui se lie au même épitope que l'anticorps 24F12 ;
(u) un anticorps comprenant SEQ ID NO : 32 (SYAMS) en tant que CDRH1, SEQ ID NO : 33 (EIGSGGSYTYYPDTVTG) en tant que CDRH2, SEQ ID NO : 34 (ETTAGYFDY) en tant que CDRH3, SEQ ID NO : 35 (SASQGINNFLN) en tant que CDRL1, SEQ ID NO : 36 (YTSSLHS) en tant que CDRL2 et SEQ ID NO : 37 (QHFSKLPWT) en tant que CDRL3 ;
(v) l'anticorps 24F12 ;
(w) un anticorps qui comprend une chaîne légère de région variable choisie dans le groupe constitué de :

(i) LK26HuVK ;
(ii) LK26HuVKY ;
(iii) LK26HuVKPW ; et
(iv) LK26HuVKPW,Y ;

(x) un anticorps qui comprend une chaîne lourde de région variable choisie dans le groupe constitué de :

(i) LK26HuVH ;
(ii) LK26HuVH FAIS,N ;
(iii) LK26HuVH SLF ;
(iv) LK26HuVH 1,1 ;
(v) LK26KOLHuVH ;

(y) un anticorps qui comprend la région variable de chaîne lourde LK26KOLHuVH (SEQ ID NO : 46) et la région variable de chaîne légère LK26HuVKPW,Y (SEQ ID NO : 41) ;
(z) un anticorps qui comprend la région variable de chaîne lourde LK26HuVH SLF (SEQ ID NO : 44) et la région variable de chaîne légère LK26HuVKPW,Y (SEQ ID NO : 41) ;
(aa) un anticorps qui comprend la région variable de chaîne lourde LK26HuVH FAIS,N (SEQ ID NO : 43) et la région variable de chaîne légère LK26HuVKPW,Y (SEQ ID NO : 41) ; et
(bb) l'anticorps monoclonal murin LK26.

**10.** Anticorps pour utilisation selon l'une quelconque des revendications 1, 3 à 5, 7, ou 9, ou procédé selon l'une quelconque des revendications 2 à 4, 6, 8, ou 9, où ledit taux d'expression de FRA du patient est évalué par technologie d'imagerie numérique ou quantification de pathologie manuelle.

**11.** Anticorps pour utilisation selon la revendication 4, ou procédé selon la revendication 4 où ledit taux d'expression de FRA du patient est évalué par FRAMSCOR ou HBSCOR.

**12.** Anticorps pour utilisation selon l'une quelconque des revendications 1, 3 à 5, 7, ou 9 à 11, ou procédé selon l'une quelconque des revendications 2 à 4, 6, ou 8 à 11, où ledit échantillon biologique

(a) est du sang total, du sérum, du plasma, des cellules circulantes, des cellules tumorales circulantes, des cellules libres, un tissu, un épanchement pleural, de l'urine, de la salive, des expectorations ou un lavage bronchique ;
(b) comprend du tissu pleural ; et/ou
(c) comprend des cellules pleurales dérivées d'un épanchement.

**13.** Anticorps pour utilisation selon la revendication 1, ou procédé selon la revendication 2 où ledit au moins un résultat clinique est la survie sans progression et/ou la survie globale.

14. Anticorps pour utilisation selon l'une quelconque des revendications 1, 3 à 5, 7, ou 9 à 13, ou procédé selon l'une quelconque des revendications 2 à 4, 6, ou 8 à 13, où ledit cancer du poumon exprimant FRA est un cancer du poumon non à petites cellules (CPNPC) exprimant FRA ; facultativement, où ledit CPNPC est un adénocarcinome.

Figure 1

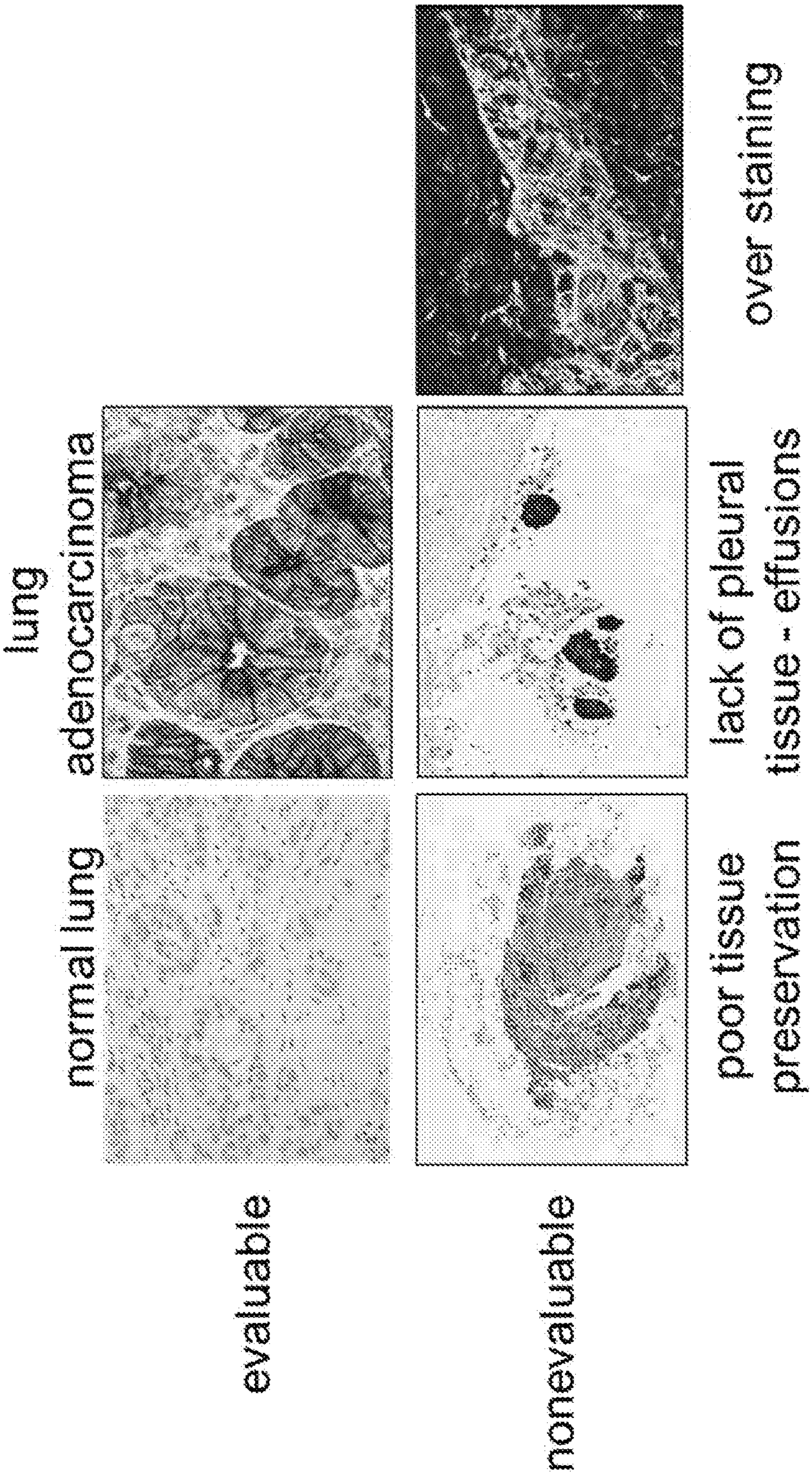
evaluable
nonevaluable
normal lung
lung adenocarcinoma
poor tissue preservation
lack of pleural tissue - effusions
over staining

Figure 2

**Non-Small Cell Lung Adenocarcinoma staining gradation:**

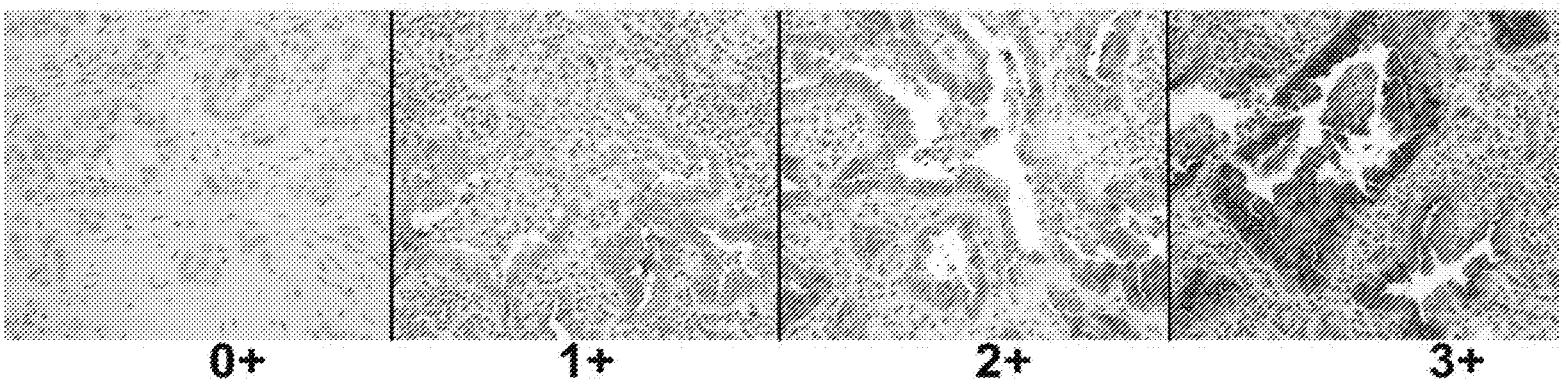

0+ 1+ 2+ 3+

Figure 3

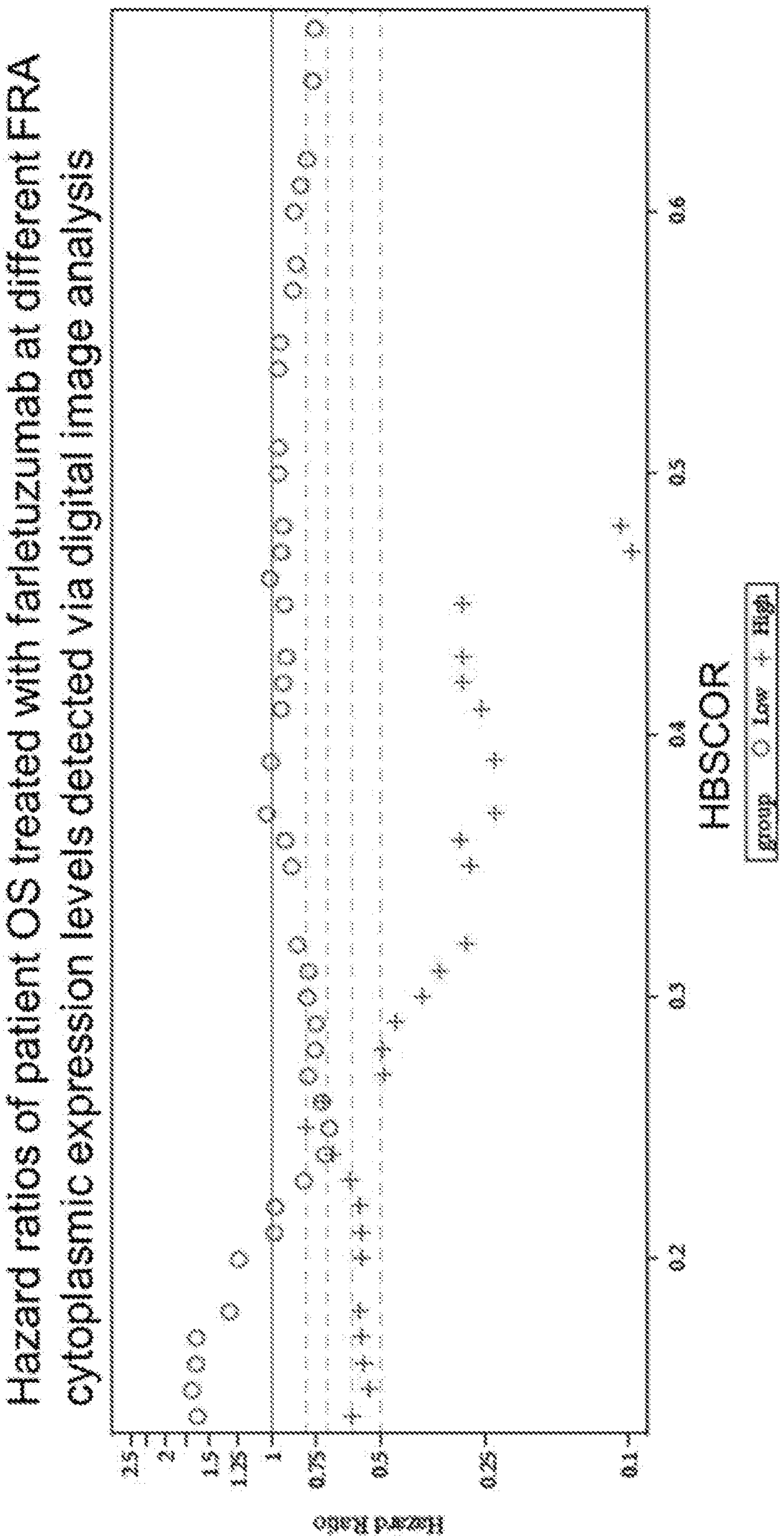
Hazard ratios of patient OS treated with farletuzumab at different FRA cytoplasmic expression levels detected via digital image analysis
Hazard Ratio
2.5
2
1.5
1.25
1
0.75
0.5
0.25
0.1
0.2
0.3
0.4
0.5
0.6
HBSCOR
group
Low
High

Figure 4

A

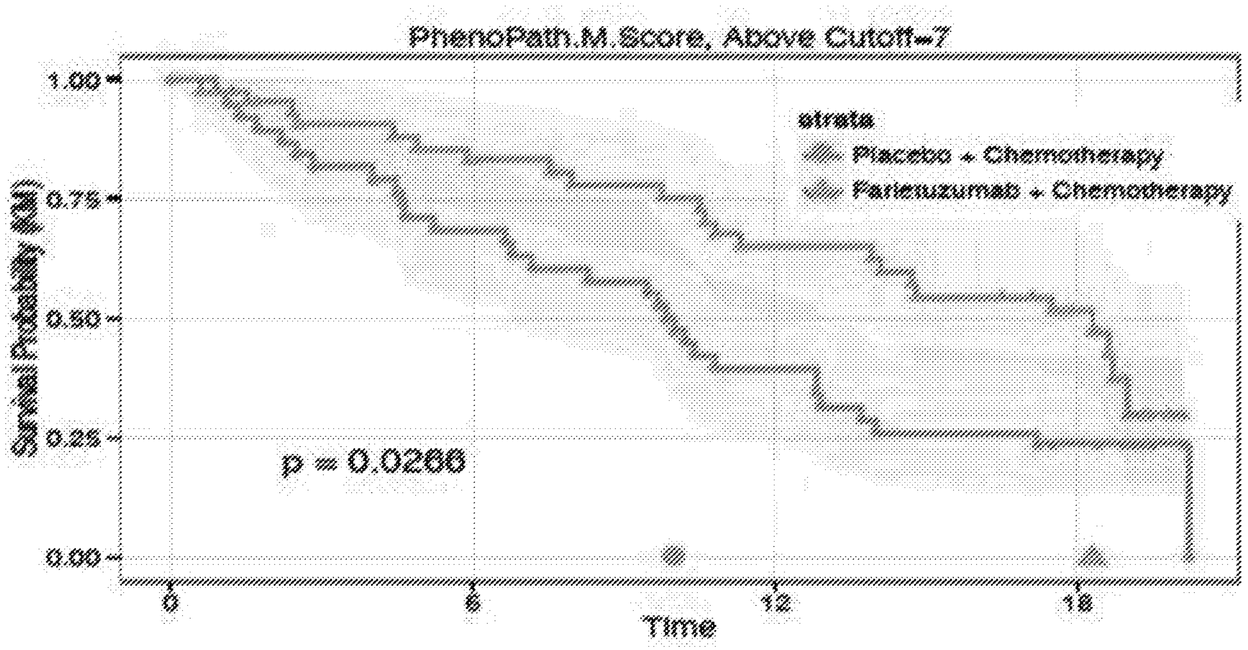
PhenoPath.M.Score, Above Cutoff=7
strata
Placebo + Chemotherapy
Farletuzumab + Chemotherapy
p = 0.0266
1.00
0.75
0.50
0.25
0.00
0
6
12
18
Time

Figure 4

B

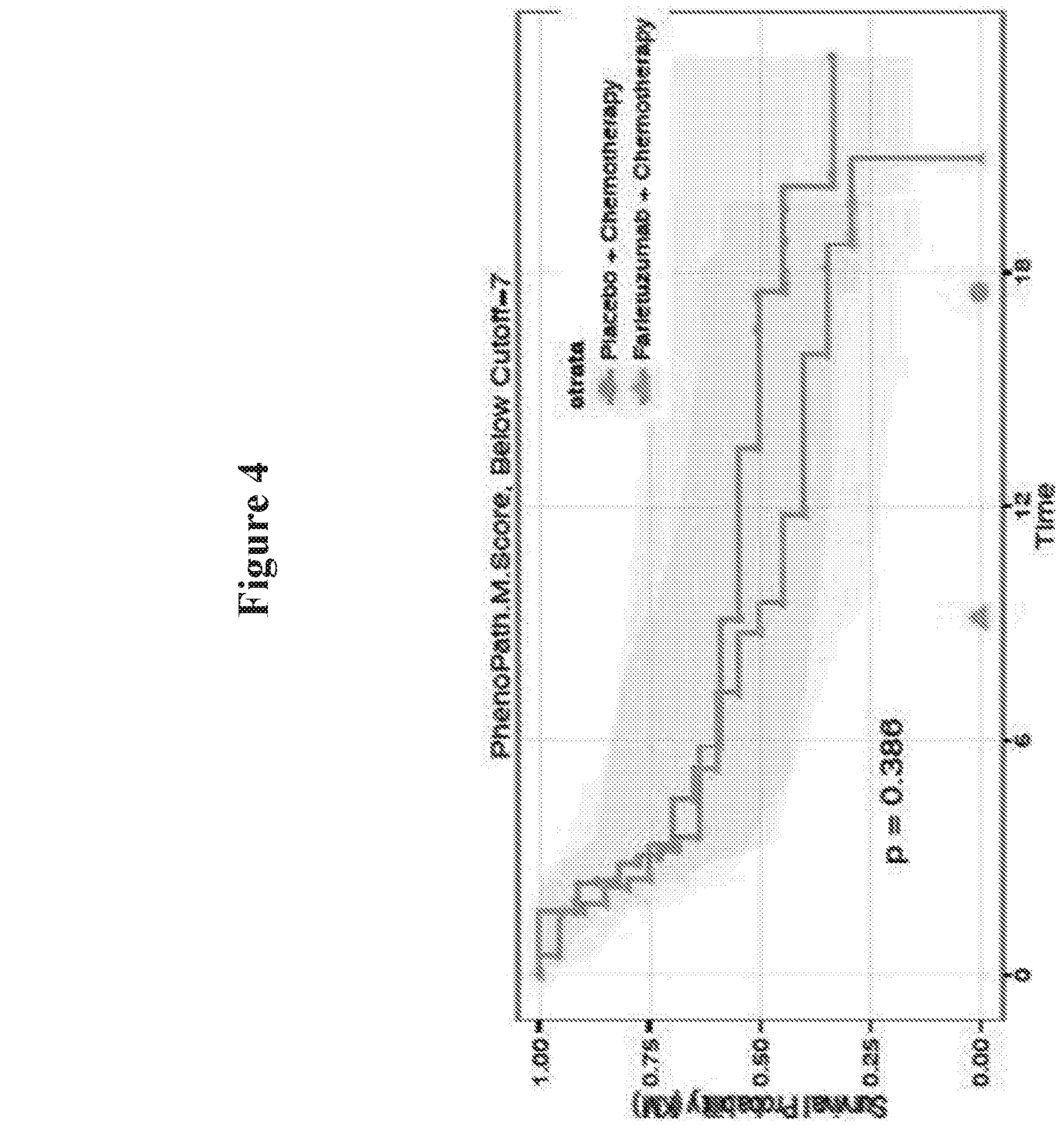
PhenoPath M.Score, Below Cutoff=7
strata
Placebo + Chemotherapy
Pertuzumab + Chemotherapy
p = 0.380
Survival Probability (KM)
1.00
0.75
0.50
0.25
0.00
0
6
12
18
Time

# Figure 5

A

Low FRA cytoplasm
10.8 vs 12.8 months

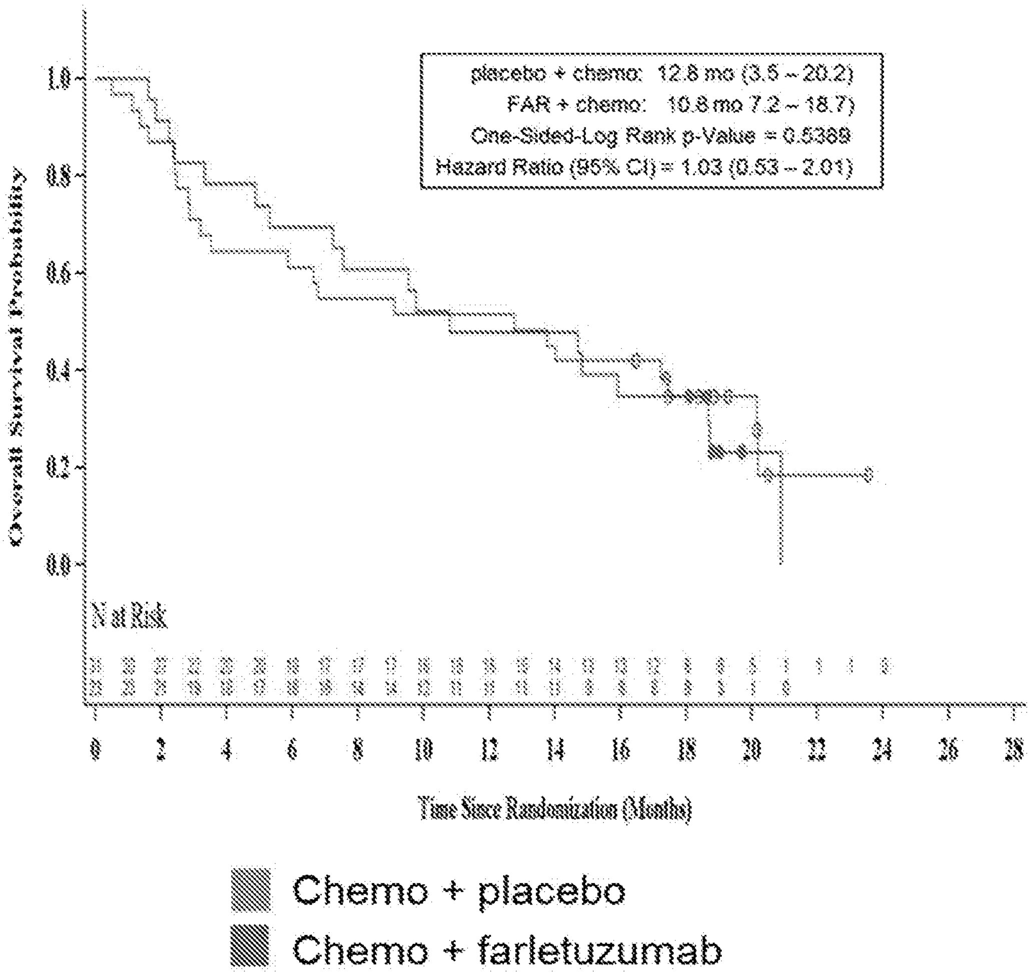

Figure 5

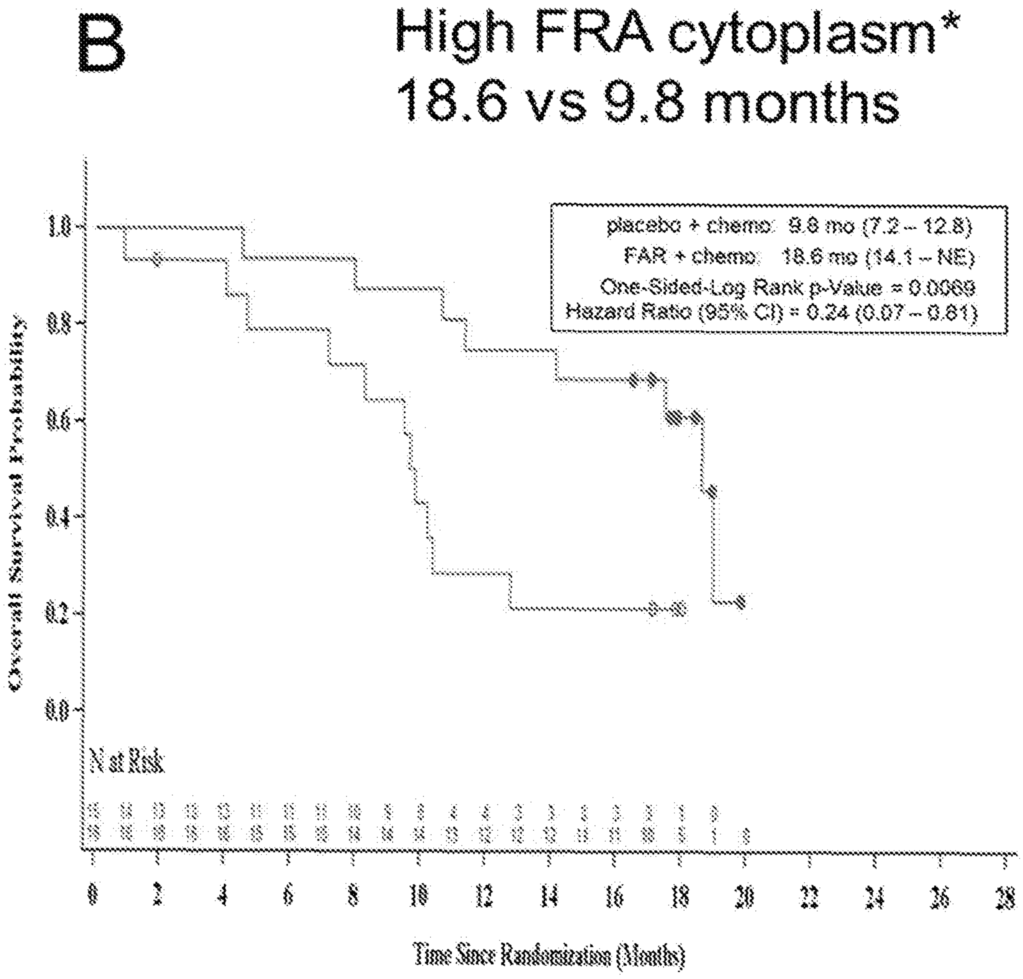
B
High FRA cytoplasm*
18.6 vs 9.8 months
placebo + chemo: 9.8 mo (7.2 – 12.8)
FAR + chemo: 18.6 mo (14.1 – NE)
One-Sided-Log Rank p-Value = 0.0069
Hazard Ratio (95% CI) = 0.24 (0.07 – 0.81)
Overall Survival Probability
1.0
0.8
0.6
0.4
0.2
0.0
N at Risk
0
2
4
6
8
10
12
14
16
18
20
22
24
26
28
Time Since Randomization (Months)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130017195 A
- US 20120207771 A
- US 20090274697 A
- US 8124083 B
- US 8475795 B
- EP 86104170
- WO 62149184 A


### Non-patent literature cited in the description


- **LI et al.** *J Clin Oncol,* 2013, vol. 31, 1039-1049
- **SCAGLIOTTI et al.** *J Clin Oncol,* 2008, vol. 26, 3543-3551
- **KRIS et al.** *JAMA,* 2014, vol. 311, 1998-2006
- **ARMSTRONG et al.** *Gynecol. Oncol.,* June 2013, vol. 129 (3), 452-8
- **THOMAS et al.** *Lung Cancer.,* 2013, vol. 80 (1), 15-8
- **VERGOTE et al.** *Cancer Metastasis Rev.,* 07 January 2015
- **PAIK.** *N Engl J Med,* 2008, vol. 358, 1409-1411
- **HARLOW ; LANE.** Antibodies: A Laboratory Manual. Cold Spring Harbor Laboratory Press, 1988
- **JAMES D. MARKS.** Antibody Engineering. Oxford University Press, 1995
- **WRIGHT, G.L., JR. et al.** *Expert Rev Mol Diagn,* 2002, vol. 2, 549
- **LI, J. et al.** *Clin Chem,* 2002, vol. 48, 1296
- **LARONGA, C. et al.** *Dis Markers,* 2003, vol. 19, 229
- **ADAM, B.L. et al.** *Cancer Res,* 2002, vol. 62, 3609
- **TOLSON, J. et al.** *Lab Invest,* 2004, vol. 84, 845
- **XIAO, Z. et al.** *Cancer Res,* 2001, vol. 61, 6029
- **POTTS.** *Drug Discov Today,* 2009, vol. 14 (19-20), 935-41
- **O'SHANNESSY et al.** *Oncotarget,* 2012, vol. 3 (4), 414-25
- **LANGER.** *Science,* 1990, vol. 249, 1527-1533
- **SEFTON.** *CRC Crit. Ref Biomed. Eng.,* 1989, vol. 14, 201
- **BUCHWALD et al.** *Surgery,* 1980, vol. 88, 507
- **SAUDEK et al.** *N. Engl. J. Med.,* 1989, vol. 321, 574
- Medical Applications of Controlled Release. CRC Press, 1974
- Controlled Drug Bioavailability, Drug Product Design and Performance. Wiley, 1984
- **RANGER ; PEPPAS.** *Macromol. Sci. Rev. Macromol. Chem.,* 1983, vol. 23, 61
- **LEVY et al.** *Science,* 1985, vol. 228, 190
- **DURING et al.** *Ann. Neurol.,* 1989, vol. 25, 351
- **HOWARD et al.** *J. Neurosurg.,* 1989, vol. 71, 105